(19) Europäisches Patentamt
European Patent Office
Office européen des brevets

(11) Publication number: 0 094 790
A2

(12) # EUROPEAN PATENT APPLICATION

(21) Application number: 83302682.6

(22) Date of filing: 11.05.83

(51) Int. Cl.³: C 07 D 239/42
C 07 D 239/52, C 07 D 239/46
C 07 D 251/46, C 07 D 251/16
C 07 D 251/22, C 07 D 405/12
A 01 N 47/36

(30) Priority: 12.05.82 US 377370
11.03.83 US 472249
21.03.83 US 474873

(43) Date of publication of application:
23.11.83 Bulletin 83/47

(84) Designated Contracting States:
AT BE CH DE FR GB IT LI LU NL SE

(71) Applicant: E.I. DU PONT DE NEMOURS AND COMPANY
Legal Department 1007 Market Street
Wilmington Delaware 19898(US)

(72) Inventor: Freerksen, Robert Wayne
2711 Ebright Road
Wilmington Delaware 19810(US)

(72) Inventor: Levitt, George
3218 Romilly Road
Wilmington Delaware 19810(US)

(72) Inventor: Schow, Gail Shelly
1015 Parkside Drive
Wilmington Delaware 19803(US)

(74) Representative: Hildyard, Edward Martin et al,
Frank B. Dehn & Co. European Patent Attorneys Imperial House 15-19 Kingsway
London WC2B 6UZ(GB)

(54) **Herbicidal O-alkoxybenzenesulfonamides.**

(57) Compounds of the formula

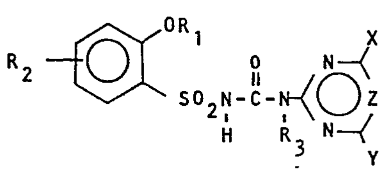

wherein $R_1$ is selected from various aliphatic and aromatic groups;
$R_2$ is H, F, Cl, Br, $CH_3$, $OCH_3$ or $CF_3$;
$R_3$ is H or $CH_3$
X is $CH_3$, $OCH_3$, Cl, $OCF_2H$ or $SCF_2H$;
Y is amino or various organic groups;
Z is CH or N;

and their agriculturally suitable salts; exhibit herbicidal activity. They may be formulated for use in conventional manner.

The novel compounds may be made by a variety of methods, e.g. by reacting an appropriately substituted phenylsulfonyl isocyanate with an appropriate 2-amino or 2-methylamino-pyrimidine or 1,3,5-triazine.

BA-8474-A/8522

. Title Cognate

# HERBICIDAL O-ALKOXYBENZENESULFONAMIDES

## Background of the Invention

This invention relates to O-alkoxybenzenesulfonamides, to benzenesulfonamide compounds and, more particularly, to ortho-(hydroxyethoxy)benzenesulfonamides which are useful as general or selective pre-emergent or post-emergent herbicides.

U.S. Patent 4,169,719 discloses and claims N-(heterocyclicaminocarbonyl)arylsulfonamides of the formula:

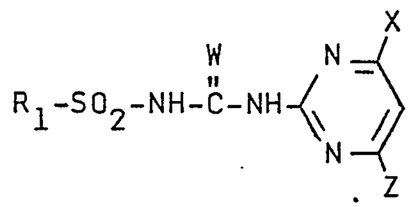

wherein

$R_1$ can be 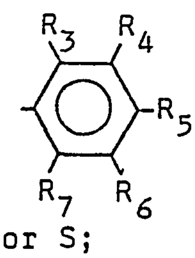 ;

W can be O or S;

$R_3$, $R_4$, $R_5$, $R_6$ and $R_7$ can be H, alkyl, halo or alkoxy, as more specifically defined therein; and

X and Z can be various substituents including H, methyl and methoxy.

These compounds are useful as plant growth regulants and as herbicides.

The following references disclose other herbicidal sulfonamides of similar, though patentably distinct, structures.

U.S. Patent 4,127,405 discloses and claims N-(1,3,5-triazin-2-ylaminocarbonyl)arylsulfonamides, which are also plant growth regulants and herbicides.

U.S. Patent 4,120,691 discloses and claims related N-(1,2,4-triazin-3-ylaminocarbonyl)arylsulfonamides, such as N-[(5,6-dimethyl-1,2,4-triazin-3-yl)aminocarbonyl]benzenesulfonamide, which are useful as plant growth regulants.

U.S. Patents 4,190,432 and 4,231,784 disclose compounds such as N-[(4-methoxy-6-methylamino-1,3,5-triazin-2-yl)aminocarbonyl]benzenesulfonamide which are useful as plant growth regulants and as herbicides, particularly for controlling volunteer corn in soybeans.

U.S. Patent 4,214,890 discloses and claims N-(heterocyclicaminocarbonyl)arylsulfonamides and thienylsulfonamides in which the heterocyclic radical is substituted in one position by a methyl or methoxy radical and in another by a substituted alkoxy or alkylthio radical. These compounds are useful for regulating plant growth and controlling undesired vegetation.

U.S. Patent 4,257,802 discloses and claims N-(heterocyclicaminocarbonyl)arylsulfonamides in which at least one of the acyclic nitrogens thereof is substituted by lower alkyl, or sometimes in the case of aminocarbonyl nitrogens, by methoxy. These compounds are useful for regulating plant growth and as general and selective herbicides.

South African Patent Application No. 82/5042 to Ciba-Geigy discloses N-phenylsulfonyl-N'-pyrimidinyl- and triazinylureas of the general formula

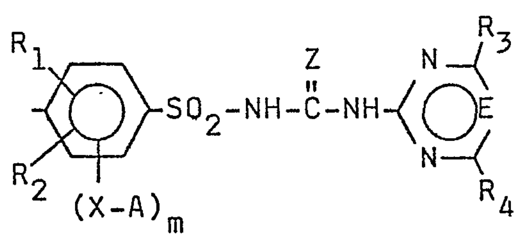

wherein

A is a $C_3$-$C_6$ alkynyl group;
E is CH or N;
X is O, S or a sulfinyl or sulfonyl bridge;
Z is O or S;
m is 1 or 2;
$R_1$ is H, halogen, $C_1$-$C_5$ alkyl or $C_2$-$C_5$ alkenyl or $-Y-R_5$;
$R_2$ is, among other values, H, halogen, $C_1$-$C_5$ alkyl or $C_2$-$C_5$ alkenyl, or a group $-Y-R_5$, $COOR_6$ or $-CO-NR_7R_8$;
$R_3$ and $R_4$ are independently H, $C_1$-$C_4$ alkyl, $C_1$-$C_4$ alkoxy, $C_1$-$C_4$ alkylthio, $C_1$-$C_4$ haloalkyl, $C_1$-$C_4$ haloalkoxy, halogen or alkoxyalkyl having up to 4 carbon atoms;
$R_5$ and $R_6$ are each $C_1$-$C_5$ alkyl, $C_2$-$C_5$ alkenyl or $C_2$-$C_6$ alkynyl;
$R_7$ and $R_8$ are independently H, $C_1$-$C_5$ alkyl, $C_2$-$C_5$ alkenyl or $C_2$-$C_5$ alkynyl; and
Y is O, S, or a sulfinyl or sulfonyl bridge.

It is stated that these compounds are useful as herbicides and as plant growth regulants.

Japanese Patent Application No. 8977/1981 which was laid open for public inspection on July 31, 1982 (Publication No. 123168/1982) discloses and claims substituted phenylsulfonylurea derivatives of the formula

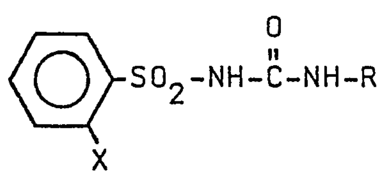

wherein

X represents a phenyl or phenoxy group;
R represents a group of the formula

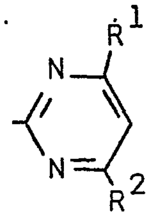 or 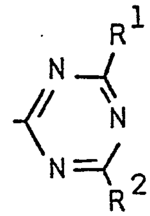 ; and

$R^1$ and $R^2$ represent a methyl or methoxy group.

The compounds are described as broad spectrum herbicides.

## Summary of the Invention

This invention relates to compounds of Formula I, agriculturally suitable compositions containing them and their method-of-use as general or selective pre-emergent or post-emergent herbicides.

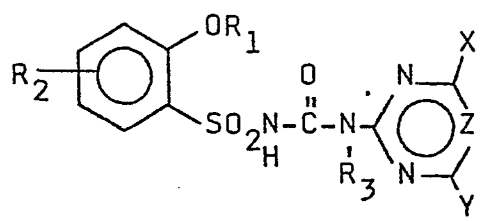

I

wherein

$R_1$ is 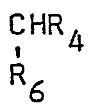, $CH_2CH_2R_5$, cyclopentyl, $C_5$-alkyl, $CH_2C{\equiv}CH$,

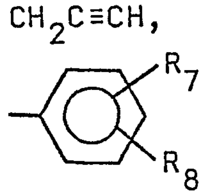, 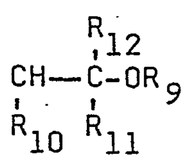 or 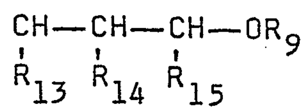;

$R_2$ is H, F, Cl, Br, $CH_3$, $OCH_3$ or $CF_3$;

$R_3$ is H or $CH_3$;

$R_4$ is CN, $CO_2CH_3$, $CO_2C_2H_5$, $SO_2N(CH_3)_2$, cyclopropyl,

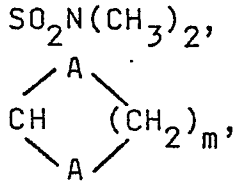, $CH(ACH_3)_2$ or

$CH(ACH_2CH_3)_2$;

$R_5$ is $N(CH_3)_2$, CN, $CO_2CH_3$, $CO_2C_2H_5$ or $SO_2N(CH_3)_2$;

$R_6$ is H, $CH_3$ or $OCH_3$;

$R_7$ is F, Cl, $CO_2CH_3$, $CH_3$, $OCH_3$, $NO_2$, $CF_3$ or $SO_2CH_3$;

$R_8$ is H, Cl, $CH_3$ or $OCH_3$;

$R_9$ is H, $COCH_3$, $COC_2H_5$, $COCH_2CH_2CH_3$, $COCH(CH_3)_2$, $COC_6H_5$, $CONHCH_3$, $CONHC_2H_5$, $CON(CH_3)_2$, 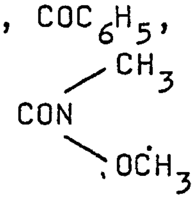 , $CONHC_6H_5$, $SO_2CH_3$, $SO_2C_2H_5$, $SO_2CF_3$, 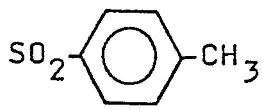 or 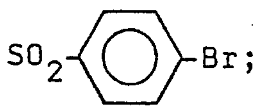 ;

$R_{10}$ is H or $CH_3$;

$R_{11}$ is H or $CH_3$;

$R_{12}$ is H or $CH_3$;

$R_{13}$ is H or $CH_3$;

$R_{14}$ is H or $CH_3$;

$R_{15}$ is H or $CH_3$;

m is 2 or 3;

A is O or S;

Z is CH or N;

X is $CH_3$, $OCH_3$, Cl, $OCF_2H$ or $SCF_2H$; and

Y is $CH_3$, $C_2H_5$, $OCH_3$, $OC_2H_5$, $CH_2OCH_3$, $OCF_3$, $CH(OCH_3)_2$, 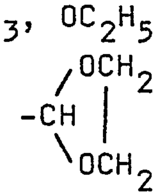 , $NH_2$, $NHCH_3$, $N(CH_3)_2$ or $GCF_2T$ where G is O or S and T is H, CHClF, CHBrF, $CF_2H$ or $CHFCF_3$;

provided that

1) when $R_6$ is $OCH_3$, then $R_4$ is $CO_2CH_3$, $CO_2C_2H_5$ or CN;

2) when $R_{11}$ and $R_{12}$ are both $CH_3$, then $R_9$ is H;

3) when either $R_{13}$, $R_{14}$ or $R_{15}$ is $CH_3$, then the others must be H;

4) when X is Cl, then Y is $OCH_3$, $CH_3$, $OC_2H_5$, $NH_2$, $NHCH_3$ or $N(CH_3)_2$ and Z is CH; and

5) when $R_6$ is H and $R_4$ is $CO_2CH_3$, then X and Y are not simultaneously $CH_3$;

and their agriculturally suitable salts.

Preferred for their higher herbicidal activity and/or more favorable ease of synthesis are:

1) Compounds of Formula I where

$R_1$ is 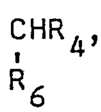, 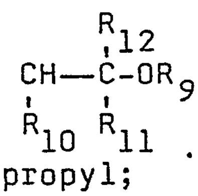 or 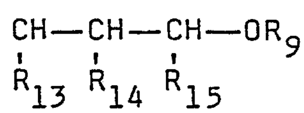;

$R_4$ is cyclopropyl;

$R_6$ is H or $CH_3$;

$R_{12}$ is H;

$R_{13}$ is H;

$R_{14}$ is H; and

$R_{15}$ is H.

2) Compounds of Preferred 1 where

$R_3$ is H.

3) Compounds of Preferred 2 where

$R_2$ is H; and

$R_9$ is H, $COCH_3$, $CONHCH_3$, $SO_2CH_3$ or $SO_2CF_3$.

4) Compounds of Preferred 3 where

$R_1$ is 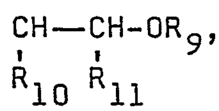, $CH_2CH_2CH_2OR$ or

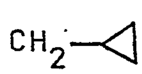 ;

$R_9$ is H;

$R_{10}$ is H or $CH_3$; and

$R_{11}$ is H.

5) Compounds of Preferred 4 where

X and Y are independently $CH_3$ or $OCH_3$.

Specifically preferred for their highest herbicidal activity and/or most favorable ease of synthesis are:

- N-[(4-methoxy-6-methyl-1,3,5-triazin-2-yl)aminocarbonyl]-2-(2-hydroxyethoxy)benzenesulfonamide;
- N-[(4,6-dimethoxy-1,3,5-triazin-2-yl)aminocarbonyl]-2-(2-hydroxyethoxy)benzenesulfonamide;
- N-[(4,6-dimethoxypyrimidin-2-yl)aminocarbonyl]-2-(2-hydroxyethoxy)benzenesulfonamide;
- N-[4-methoxy-6-methylpyrimidin-2-yl)aminocarbonyl]-2-(2-hydroxyethoxy)benzenesulfonamide;
- N-[4,6-dimethylpyrimidin-2-yl)aminocarbonyl]-2-(2-hydroxyethoxy)benzenesulfonamide;
- N-[4-methoxy-6-methylpyrimidin-2-yl)aminocarbonyl]-2-(2-methylsulfonyloxyethoxy)benzenesulfonamide;
- N-[(4,6-dimethoxypyrimidin-2-yl)aminocarbonyl]-2-(cyclopropylmethyloxy)benzenesulfonamide;
- N-[(4-methoxy-6-methyl-1,3,5-triazin-2-yl)aminocarbonyl]-2-(cyclopropylmethyloxy)benzenesulfonamide;
- N-[(4,6-dimethoxy-1,3,5-triazin-2-yl)aminocarbonyl]-2-(cyclopropylmethyloxy)benzenesulfonamide; and
- N-[(4,6-dimethoxy-1,3,5-triazin-2-yl)aminocarbonyl]-2-(2-methylphenoxy)benzenesulfonamide.

Compounds of general formula (I) as disclosed and claimed in our U.S. Patent Applications Serial Nos. 377,370, 472,249 and 474,873 also form preferred aspects of our invention.

## Detailed Description of the Invention

### Synthesis

The compounds of Formula (1) can be prepared by one or more of the methods described below in Equations 1 to 6. Where specific reaction conditions are stated, these are currently preferred but may in general be varied in a manner obvious to those skilled in the art. As shown in Equation 1 below, compounds of Formula (1), wherein $R_9$ is not H can be prepared by reacting an appropriately substituted sulfonyl isocyanate of Formula (2) with an appropriate 2-aminopyrimidine, 2-alkylaminopyrimidine, 2-amino-1,3,5-triazine or 2-alkylamino-1,3,5-triazine of Formula (3), $R_1$, $R_2$, $R_3$, X, Y and Z being as previously defined.

Equation 1

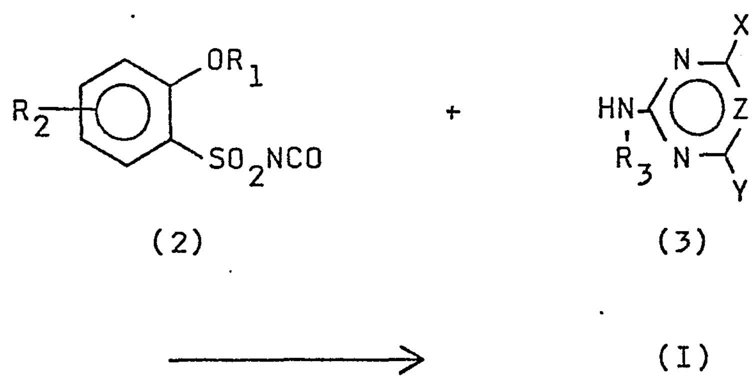

(2) + (3)

(I)

The reaction is best carried out in inert aprotic organic solvents such as methylene chloride, tetrahydrofuran or acetonitrile, at ambient pressure and temperature. The mode of addition is not critical; however, it is often convenient to add the sulfonyl isocyanate to a stirred suspension of amine (3). Since such isocyanates are usually liquids, their addition can be easily controlled.

The reaction is generally exothermic. In some cases, the desired product is insoluble in the warm reaction medium and crystallizes from it in pure form. Products soluble in the reaction medium are isolated by evaporation of the solvent, trituration of the solid residue with solvents such as 1-chlorobutane

or ethyl ether, and filtration. The compounds of Formula I may be purified by recrystallization or chromatography.

Some of the compounds of Formula (1) may best be prepared by the method shown below in Equation 2.

Equation 2

(a)

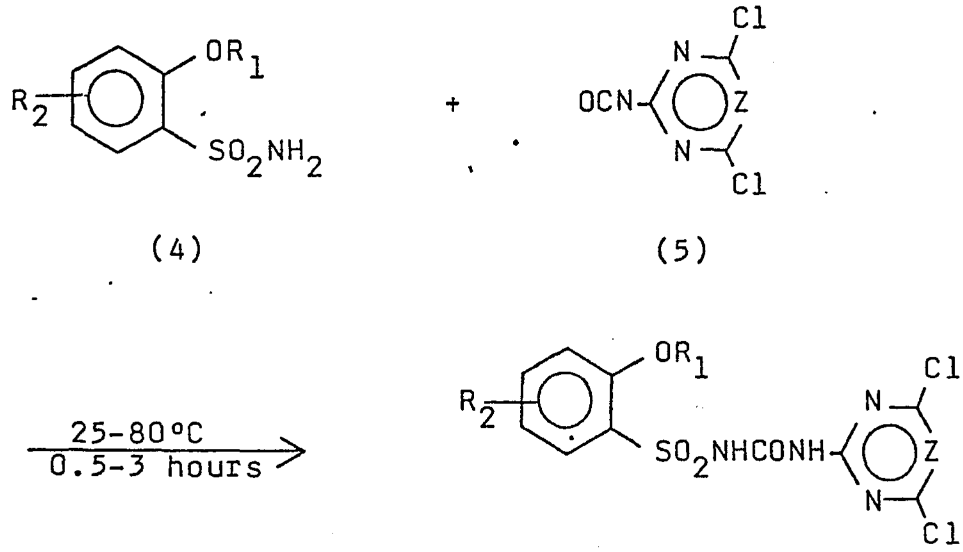


(4) (5)

(6)

(b)

(6)

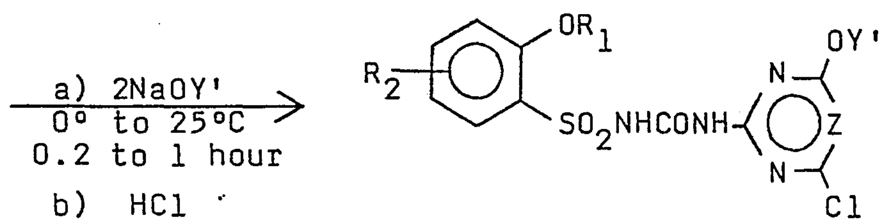


(1a)

(c)

(1a)

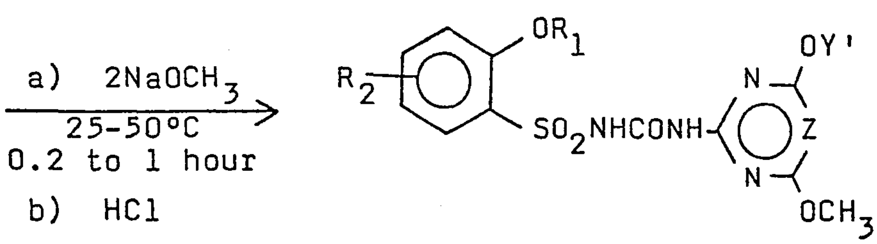


(1b)

(d)

(6) 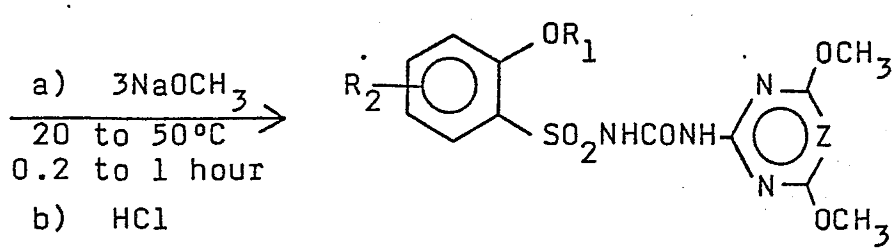


(1c)

wherein Y' is $CH_3$ or $CH_2CH_3$ and $R_1$, $R_2$ and Z are as previously defined, and $R_9$ is not H.

The reactions of Equation 2 are carried out according to similar procedures taught in our EP-A- 30,140. Thus, in reaction 2a, a sulfonamide of Formula (4) is reacted with a dichloropyrimidinyl isocyanate or dichlorotriazinyl isocyanate of Formula (5) in an inert solvent such as acetonitrile at reflux for 0.5 to 3 hours to form a sulfonylurea of Formula (6). The product is isolated by filtration. In reaction 2b, (6) is reacted with two mole equivalents of sodium methoxide or sodium ethoxide in tetrahydrofuran at 0° to 25°C for about one hour, followed by acidification with hydrochloric acid to a pH of about 1, to form a sulfonylurea of Formula (1a). The product is isolated by filtration. In reaction 2c, (1a) is reacted with two mole equivalents of sodium methoxide in methanol at 25° to about 50°C for about 1 hour, followed by acidification with hydrochloric acid to a pH of about 1, to form a sulfonylurea of Formula (1b). Alternatively, as shown in reaction 2d, (6) can be reacted with at least three mole equivalents of sodium methoxide at 20° to 50°C for about 1 hour, followed by acidification with hydrochloric acid to a pH of about 1, to provide (1c) directly, where Y' of (1b) is $OCH_3$. The products of reactions 2c and 2d are isolated by addition of water and filtration.

The heterocyclic isocyanates of Formula (5) in Equation 2 above can be prepared by methods described in Swiss 579,062, U.S. 3,919,228, U.S. 3,732,223 and Angew Chem. Int. Ed., 10, 402 (1976), to which the reader is referred for further information.

Some of the compounds of Formula (1) (where $R_9$ is not H) can be prepared as shown in Equation 3 below. Reaction of a sulfonamide of Formula (4) with an appropriate methyl pyrimidinyl carbamate or methyl triazinyl carbamate of Formula (7) in the presence of an equimolar amount of trimethylaluminum, leads to compounds of Formula (1) wherein $R_1$, $R_2$, $R_3$, X, Y and Z are as previously defined.

Equation 3

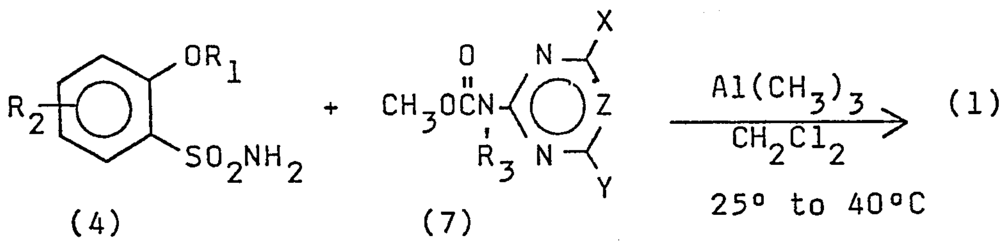


(4) (7)

The reaction of Equation 3 is best run in methylene chloride at about 25° to 40°C for 10 to 96 hours under a nitrogen atmosphere. The product can be isolated by addition of an aqueous acetic acid solution followed by extraction of the product into methylene chloride, or by filtration of a product of low solubility. The product can be purified by trituration with solvents such as 1-chlorobutane, ethyl acetate or ethyl ether or by column chromatography on silica gel.

Many of the compounds of Formula (1) (where $R_9$ is not H) can be prepared by the procedure described by Equation 4 below. Deprotonation of a sulfonamide of Formula (4) and subsequent reaction with an equimolar amount of diphenylcarbonate provides a phenylcarbamate of Formula (10), after neutralization of the reaction mixture. Reaction of a mixture of the phenylcarbamate of Formula (10) with an appropriate 2-aminopyrimidine or 2-amino-1,3,5-triazine of Formula (3) produces the desired compounds of Formula (1).

Equation 4

(a)

(4) 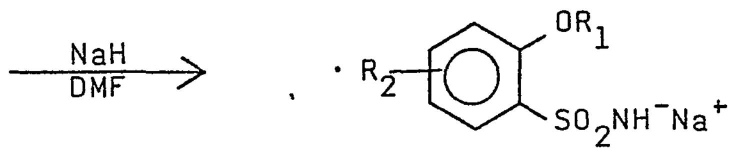


(8)

(b)

(8) + 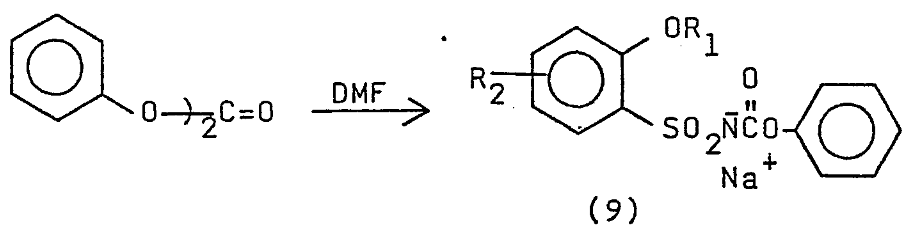


(9)

(c)

(9) + $H_3O^+$ 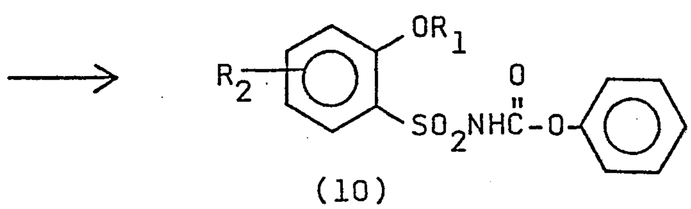

(10)

(d)

(10) + (3)
dioxane
(1)

The deprotonation in Equation 4a and displacement in Equation 4b are run under an inert atmosphere in the absence of water. Bases such as n-butyl lithium or lithium diisopropylamide and solvents such as tetrahydrofuran or 1,4-dioxane may be substituted for sodium hydride and dimethylformamide in reaction 4a in certain cases. Since the deprotonation described by Equation 4a is generally exothermic, external cooling and/or gradual contacting of the reactants is generally preferred to prevent decomposition. The displacement described by reaction 4b is best conducted in a dipolar aprotic solvent such as dimethylformamide or hexamethylphosphoric triamide at a temperature of from 25° to 40° for one-half to twenty four hours. The phenylcarbamate of Formula (10) can be isolated by the addition of an aqueous acid and extraction with a solvent such as ethyl acetate. The desired compounds of Formula (1) are prepared by contacting the phenylcarbamate of Formula (10) with an equimolar amount of an appropriate heterocyclic amine of Formula (3) in an inert solvent such as 1,4-dioxane or tetrahydrofuran at a temperature of from 20° to the reflux temperature of the solvent for from one-half to twenty-four hours. The product can be isolated by evaporation of the reaction solvent and can be purified by trituration with a solvent such as 1-chlorobutane or ethyl ether or by column chromatography.

Many of the compounds of Formula (1) can be prepared by the process shown in Equation 5 below, wherein $R_1$, $R_2$ and $R_3$ are as previously defined and Q is a leaving group such as chloro, bromo, iodo or an alkyl sulfonate ester.

Equation 5

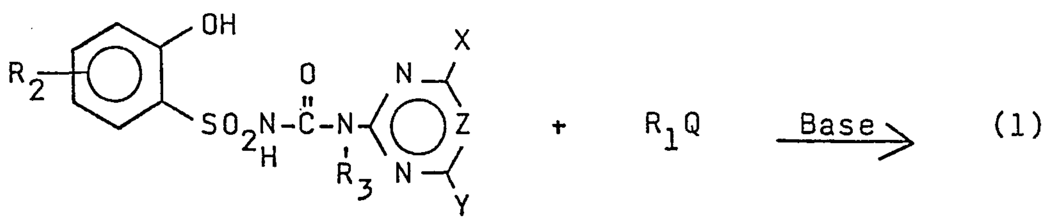


(11)

The reaction can be carried out with dipolar aprotic solvents such as dimethylformamide or solvents such as acetone, methyl ethyl ketone or tetrahydrofuran. Bases which may be employed include potassium carbonate, potassium bicarbonate or triethylamine. The reaction can be conducted at temperatures ranging from 20° to the reflux temperature of the solvent. The reaction is continued, usually for a period of from one-half hour to several days, until the reaction is judged to be complete by analytical methods such as chromatography. The product is isolated by neutralization of the reaction mixture with dilute aqueous acid and extraction with a solvent such as ethyl acetate. The crude product can be purified by trituration with solvents such as 1-chlorobutane or ethyl ether or by column chromatography. The 2-hydroxybenzenesulfonylureas of Formula (11) are themselves prepared from the corresponding 2-benzyloxybenzenesulfonylureas by treatment with trifluoroacetic acid according to the teaching of J.P. Marsh and L. Goodman, J. Org. Chem., 30, 2491 (1965).

Compounds of Formula (1) wherein $R_9$ is not H can also be prepared from compounds of Formula (1) where $R_9$ is H by treatment with an appropriate acyl halide, sulfonyl halide or carbamoyl halide in the presence of an acid acceptor or, in the case wherein $R_9$ is methylamino carbonyl, ethylamino carbonyl or phenylamino carbonyl, by treatment with the appropriate isocyanate. The reaction is best conducted in an inert solvent such as tetrahydrofuran in the presence of a base such as triethylamine at a temperature of from 0° to 40° for from one hour to several hours. The reaction of acid halides may be accelerated by the addition of a small amount of a catalyst such as 4-dimethylaminopyridine.

Compounds of Formula (1) wherein $R_9$ is H can be prepared from compounds of Formula (1) wherein $R_9$ is an alkyl or aryl sulfonyl group by contacting the sulfonate ester with dilute aqueous base. In some cases compounds of Formula (1) wherein $R_9$ is H can be isolated directly from the reaction described by Equation 1 wherein $R_9$ is $SO_2CH_3$.

Some of the compounds of Formula (1) wherein $R_9$ is H can be prepared by one or both of the processes described by Equation 5 or Equation 6, wherein $R_1$, $R_2$, $R_3$, $R_{10}$, $R_{11}$, $R_{12}$, X, Y and Z are as previously defined and Q is a leaving group such as chloro, bromo, iodo or a sulfonate ester.

Equation 6

(11) +

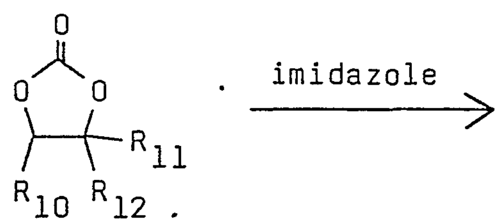


(1)

(12)

The reaction described by Equation 6 is best conducted by mixing the sulfonylurea of Formula (11) with an equimolar amount or an excess of a carbonate ester of Formula (12) in the presence of a catalyst such as imidazole. The reactants are mixed vigorously and heated to a temperature sufficient to cause the liberation of carbon dioxide, with 130° to 160° being preferred. Compounds of Formula (1) are isolated by trituration with a solvent such as diethyl ether, by recrystallization or by chromatography.

Many of the intermediate sulfonyl isocyanates of Formula (2) in Equation 1 above can be prepared from sulfonamides (4) by methods described in U.S. 4,238,621. The method requires reacting sulfonamides

with phosgene, in the presence of n-butyl isocyanate and a tertiary amine catalyst, at reflux in a solvent such as xylene. A preferred catalyst is 1,4-diazabicyclo[2.2.2]octane (DABCO).

Alternatively, many of the sulfonyl isocyanates (2) can be prepared from sulfonamides (4) by a two-step procedure. This consists of first reacting the sulfonamide with n-butyl isocyanate and a base such as potassium carbonate at reflux in an inert solvent such as 2-butanone to form a n-butyl sulfonylurea; and secondly reacting this product with phosgene and DABCO catalyst at reflux in xylene solvent. This method is similar to a procedure taught by Ulrich and Sayigh, Newer Methods of Preparative Organic Chemistry, Vol. VI, p. 223-241, Academic Press, New York and London, W. Foerst Ed.

Alternatively, many of the sulfonyl isocyanates (2) can be prepared from sulfonamides (4) by the method of Ulrich, et al., J. Org. Chem., 34, 3200 (1969) as shown in Equation 7 below.

Equation 7

$$(4) \xrightarrow[\text{2) } COCl_2 \text{, pyridine (cat), solvent}]{\text{1) } SOCl_2} (2)$$

The sulfonamide (4) is boiled under reflux with an excess of thionyl chloride which functions as a reactant and a solvent. The reaction is continued until the sulfonamide protons are undetectable in the proton resonance spectrum. An overnight reaction period (about 16 hours) is generally sufficient. The thionyl chloride is removed under vacuum and the residue dissolved in an inert solvent, such as toluene, benzene, xylenes, etc., treated with a catalytic

amount of pyridine and then at least one equivalent of phosgene. The mixture is heated to between 60° and 140°C, with 80° to 100°C preferred. Conversion to the sulfonyl isocyanate is substantially complete within about 1 1/2 to 3 hours. The mixture containing the sulfonyl isocyanate can be used directly for the next reaction step (formation of compounds of Formula 1) or the sulfonyl isocyanate can be isolated in purified form by filtration and evaporation of the filtrate, optionally followed by vacuum distillation.

Equation 8 shown below illustrates the preparation of the required methyl pyrimidinyl carbamates and methyl triazinyl carbamates of Formula (7) in Equation 3 above, wherein $R_3$, X, Y and Z are as previously defined.

Equation 8

$$(3) \quad + \quad (CH_3O)_2\overset{\overset{O}{\|}}{C} \quad \xrightarrow[\text{25° to 70°C} \atop \text{1 to 24 hours}]{\text{NaH}} \quad (7)$$

According to Equation 8, a heterocyclic amine of Formula (3) is reacted with two equivalents of sodium hydride and excess dimethylcarbonate to form (7). The reaction is run in an inert solvent such as tetrahydrofuran at 25°C to reflux for 1 to 24 hours. The product is isolated by (a) adding about two equivalents of concentrated HCl and water saturated with NaCl, and (b) separating out the organic phase and concentrating to dryness *in vacuo*.

The synthesis of heterocylic amines such as (3) above has been reviewed in "The Chemistry of Heterocyclic Compounds", a series published by Interscience Publ., New York and London. 2-Aminopyrimidines are described by D. J. Brown in "The Pyrimidines", Vol. XVI of this series. 2-Amino-1,3,5-triazines can be

prepared according to methods described by E. M. Smolin and L. Rapaport in "s-Triazines and Derivatives", Vol. XIII of the same series. The synthesis of triazines is also described by F. C. Schaefer, U.S. 3,154,547 and by K. R. Huffman and F. C. Schaefer; J. Org. Chem., 28, 1816 (1963).

The preparation of sulfonamides of Formula (4) may be accomplished by one or more of the methods described below in Equations 9 and 10. Choice of the appropriate method of preparation may be influenced by the substituents $R_1$ and $R_2$ and would be obvious to one skilled in the art.

As shown in Equation 9 below, intermediate sulfonamides of Formula (4) may be prepared by amination of the corresponding sulfonyl chlorides (13) wherein $R_1$ and $R_2$ are as previously defined.

Equation 9

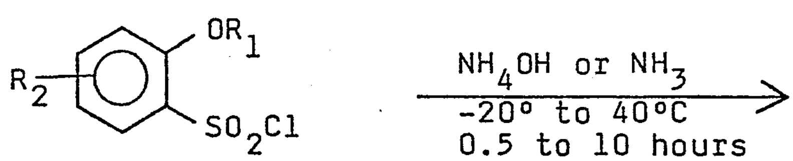


(4)

(13)

Conversion of sulfonyl chlorides to sulfonamides is well known (e.g., L. F. Fieser and M. Fieser, "Advanced Organic Chemistry," p. 699, Reinhold, New York, 1961). The amination described in Equation 9 is conveniently carried out by treating a solution of the sulfonyl chloride (13) with at least two mole equivalents of anhydrous ammonia in a solvent such as ethyl ether or methylene chloride at -20° to 30°C. If the sulfonamide product (4) is insoluble, it may be isolated by filtration followed by washing the salts out with water. If product (4) is soluble in the reaction solution, it may be isolated by filtering off the precipitated ammonium chloride and evaporation of the solvent. Alternatively, many sulfonamides (4) can be prepared by reaction of corresponding sulfonyl chlorides (13) with excess aqueous ammonium hydroxide in tetrahydrofuran at 0° to about 40°C for 0.5 to 10 hours. The sulfonamide product (4) is isolated by evaporation of the tetrahydrofuran solvent, addition of water to the residue and filtration.

Alternatively, sulfonamides of Formula (4) may be prepared by alkylation of the corresponding 2-hydroxybenzenesulfonamide (14) as shown below in Equation 10; wherein $R_2$ and Q are as previously defined and when $R_1$ is a substituted or unsubstituted alkyl residue, or when $R_1$ is an aryl residue and $R_8$ is nitro, trifluoromethyl or methylsulfonyl, and Q is fluoro or chloro.

Equation 10

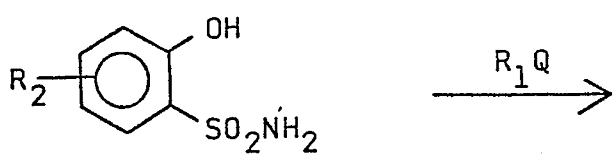


(4)

(14)

The alkylation described by Equation 10 can be carried out in a solvent such as acetone, methyl ethyl ketone or dimethylformamide in the presence of a base such as potassium carbonate. When $R_1$ is an aryl residue, the reaction may be facilitated by the addition of copper salts.

The 2-hydroxybenzenesulfonamides (14) can be prepared using one or more of the methods described below in Equations 11, 12, 13 and 14.

Equation 11

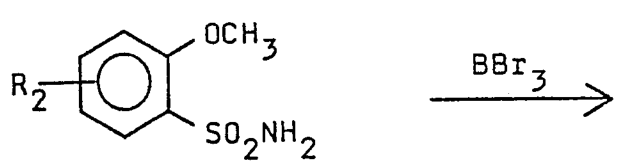


(14)

(15)

Equation 12

(15) 1) $NaSCH_2CH_3$ / DMF, 2) $H^+$ → (14)

Equation 13

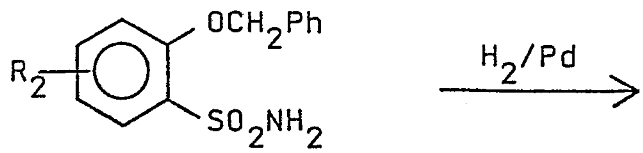

(14)

(16)

Equation 14

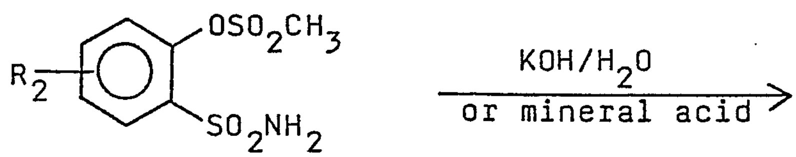

(14)

(17)

The methyl aryl ethers of Formula (15) can be cleaved by methods well known in the art. Cleavage with Lewis acids, such as boron tribromide, is shown in Equation 11 and such reactions are described in J. Am. Chem. Soc., 64, 1128 (1942). Equation 12 illustrates cleavage by sulfur nucleophiles and such reactions are described in Tetrahedron Letters 1327 (1970). Hydrogenolysis of the benzyloxybenzenesulfonamides of Formula (16) may also be employed for the preparation of the 2-hydroxybenzenesulfonamides (14). Reactions of this type are described in J. Chem. Soc., 2903 (1958). Methanesulfonate esters of Formula (17) are cleaved using aqueous base or mineral acids. The cleavage of methanesulfonate esters and the preparation of 2-sulfamylphenyl methanesulfonates (17) is described in Research Disclosure, pg. 52 (1978).

The preparation of sulfonamides of Formula (4) wherein $R_9$ is H can be accomplished by one or more of the methods described below in Equations 15 to 18, wherein $R_1$, $R_2$, $R_{10}$, $R_{11}$, $R_{12}$, $R_{13}$, $R_{14}$, $R_{15}$ and Q are as previously defined.

Equation 15

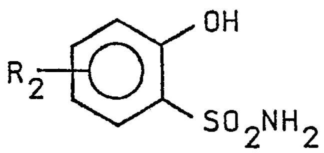

$+ \quad R_1Q \quad \xrightarrow{\text{base}} \quad (4)$

(14)

The reaction described by Equation 15 can be conducted in solvents such as dimethylformamide, dimethyl sulfoxide, acetone or 2-butanone at a temperature of from 25° to the reflux temperature of the solvent for a period of from a few hours to several days. Bases which can be employed include potassium carbonate, potassium bicarbonate and sodium hydride.

Equation 16

(14) +

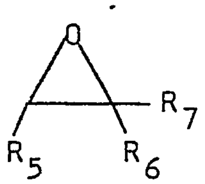

$\xrightarrow{\text{base}} \quad (4)$

(18)

The reaction described by Equation 16 can be conducted in a solvent such as dimethyl sulfoxide or in the absence of a solvent. Bases which may be employed include potassium carbonate, potassium bicarbonate and sodium hydride.

Equation 17

$$(14) \quad + \quad (12) \quad \xrightarrow{\text{imidazole}} \quad (4)$$

Equation 18

(14) +

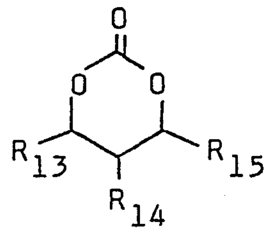

(19)

imidazole

(4)

The reaction described by Equation 17 and Equation 18 are conducted by heating a mixture of one part of the 2-hydroxybenzenesulfonamide of Formula (14), one molar equivalent to several equivalents of an appropriately substituted carbonate ester of Formula (12) or Formula (19) and from one one-hundreth part to one-tenth part of a catalyst such as imidazole. The combined reactants are mixed vigorously in the absence of a solvent and are heated to a temperature at least sufficient to liquefy the mixture and to cause the liberation of carbon dioxide, with a temperature of from 130° to 160° being preferred. Heating is continued until the evolution of carbon dioxide has ceased and the reaction is deemed to be complete by analytical methods such as chromatography. The sulfonamides of Formula (4) are isolated by trituration with a solvent such as diethyl ether and can be purified by recrystallization or by chromatography. Reactions wherein the carbonate esters of Formula (12) or Formula (19) are asymmetrically substituted may result in the formation regioisomeric product mixtures which may require further separation by methods such as chromatography. The carbonate esters of Formula (12) and Formula (19) are themselves prepared from the corresponding diols and phosgene by methods well known in the art.

The sulfonamides of Formula (4) wherein $R_9$ is not H can be prepared from the appropriate sulfonamide of Formula (4) wherein $R_9$ is H by treatment of the alcohol with the appropriate acyl halide, sulfonyl halide, carbamoyl halide or isocyanate. The reaction is best conducted in an inert solvent such as tetrahydrofuran in the presence of a base such as triethylamine at a temperature of from 0° to 40° for from one hour to several hours. The reaction of acid halides may be accelerated by the addition of a catalyst such as 4-dimethylaminopyridine.

Many intermediate sulfonyl chlorides of Formula (13) can be prepared from amines of Formula (20) by diazotization of the amine in dilute sulfuric acid and coupling the diazonium salt with sulfur dioxide in the presence of copper salts, as shown below in Equation 19; wherein $R_1$ and $R_2$ are as previously defined.

<u>Equation 19</u>

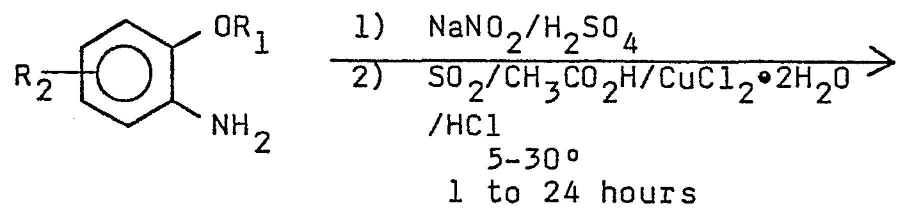


(13)

(20)

The reaction of Equation 19 is accomplished by treating a solution of the amine (20) in dilute sulfuric acid with a solution of sodium nitrite in water at 0° to 5°C. After stirring for 10 to 30 minutes at about 0°C to insure complete diazotization, the solution is added to a mixture of an excess of sulfur dioxide and cupric chloride dihydrate in glacial acetic acid and hydrochloric acid at about 10° to 35°C. The mixture is stirred at 10° to 35°C for 2 to 24 hours. A cosolvent such as ether or <u>n</u>-butyl chloride may be added. The solution is poured into a large excess of

ice-water. The sulfonyl chloride (13) can be isolated by filtration or by extraction into a solvent such as ethyl ether, methylene chloride or, preferably, 1-chlorobutane followed by drying and evaporation of the solvent.

Aromatic amines such as those of Formula (20) may either be purchased or prepared by the reduction of the corresponding aromatic nitro compounds of Formula (21), according to Equation 20, shown below; wherein $R_1$ and $R_2$ are as previously described.

Reduction of aromatic nitro compounds to anilines is well known in the art (e.g., H. O. House, "Modern Synthetic Reactions", Second Edition, Chapters 1-4, W. A. Benjamin, Inc., U.S.A., 1972).

Equation 20

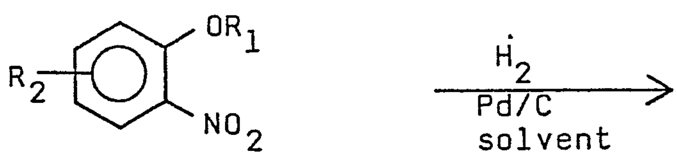


(20)

(21)

In those cases in which $R_1$ is a substituted or unsubstituted alkyl residue the aromatic nitro compounds of Formula (21) can be prepared by the reaction described in Equation 21, wherein $R_1$ is an appropriate alkyl residue and Q is a leaving group such as chloro, bromo, iodo or alkyl sulfonate ester.

Equation 21

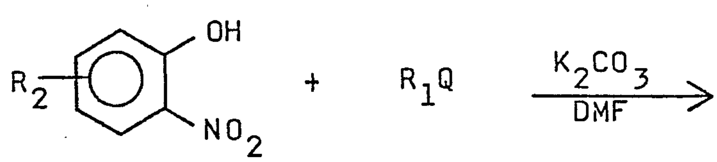


(21)

Aromatic nitro compounds of Formula (21) wherein $R_1$ is an aryl residue can be prepared by the procedure given in Equation 22 below, wherein $R_2$, $R_7$

and $R_8$ are as previously defined and Q is a suitable leaving group such as fluoro, chloro, bromo or iodo.

<u>Equation 22</u>

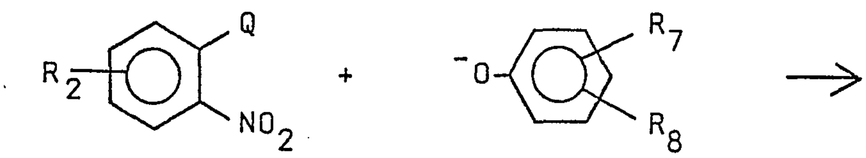

(21)

The reaction shown in Equation 22 may be facilitated by the addition of copper salts.

Compounds of Formula (24) or Formula (25) wherein $R_2$ and $R_8$ are not methyl may also be prepared by the procedure given in Equation 23 below.

<u>Equation 23</u>

a)

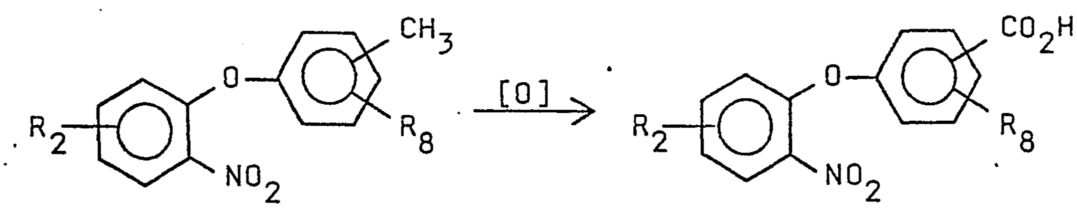


(22) (23)

b)

(23)

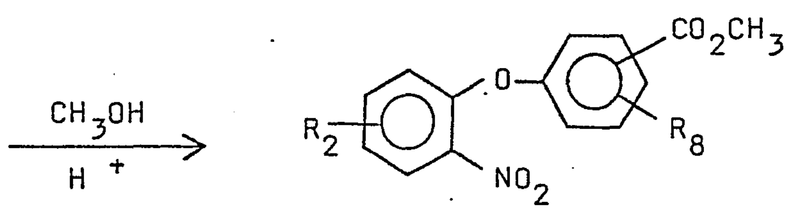


(24)

c)

(24)

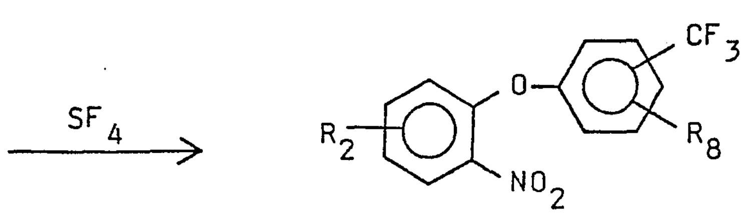


(25)

The methyl group of compounds of Formula (22) may be oxidized with reagents such as potassium permanganate or potassium dichromate. The resulting carboxylic acids of Formula (23) may then be transformed into the corresponding methyl ester with a variety of reagents such as methanol and an acid catalyst or diazomethane to provide compounds of Formula (24) as shown in reaction 23b. Compounds of Formula (25) can be prepared from compounds of Formula (23) as shown in reaction 23c by treatment with a fluorinating agent such as sulfur tetrafluoride in the presence of hydrogen fluoride. The compounds of Formula (22) can themselves be prepared by the reaction described by Equation 22.

Intermediate sulfonyl chlorides of Formula (13) in which $R_1$ is an alkyl or dimethylaminoalkyl group and $R_2$ is not a chloro or bromo substituent may also be prepared by one of the methods described by Equations 23 and 24, below.

Equation 23

a)

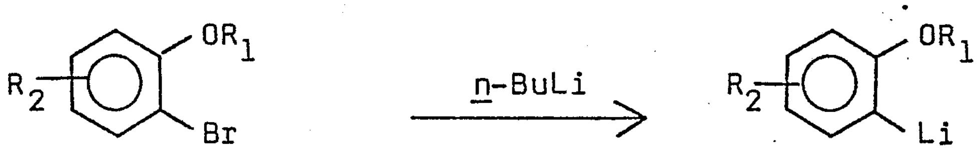


(26) (27)

b)

(27)

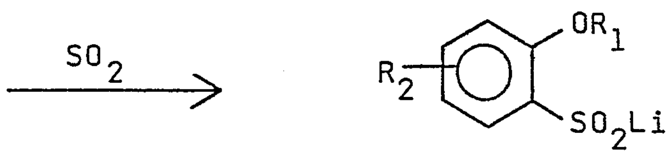


(28)

c)

(28) chlorinating agent (13)

Equation 24

a)

$$(26) \xrightarrow{\underline{n}\text{-BuLi}} (27)$$

b)

$$SO_2Cl_2 \xrightarrow{(27)} (13)$$

Bromoaryl ethers of Formula (26) are dissolved in a solvent or a solvent mixture such as ether or ether and hexane and are cooled to a temperature of between -78° and 0°C under a dry, inert atmosphere. Addition of a solution of n-butyl lithium in hexane effects metal-halogen exchange to provide the organolithium intermediate (27). In some cases, metallation is facilitated by warming the reaction mixture to room temperature for periods of up to one-half hour.

As described in Equation 23, the organolithium intermediate (27) is treated at a temperature of from -50° to -20°C with sulfur dioxide to form the sulfinate salt (28). The salt (28) is then converted into the desired sulfonyl chloride (13) by treatment with a chlorinating agent such as chlorine or N-chlorosuccinimide in solvent mixtures such as aqueous acetic acid or acetone and acetic acid at temperatures of from -20° to 10°C. Alternatively, the sulfinate salt (28) may be suspended in an inert solvent such as methylene chloride at a temperature of from -78° to -50°C and treated with sulfuryl chloride. The sulfonyl chlorides (13) are isolated by aqueous work up and extraction with an organic solvent. The preparation of sulfonyl chlorides by sequential treatment of organo lithium species with sulfur dioxide and a chlorinating agent is taught in U.S. 4,127,405.

The sulfonyl chlorides (13) may also be prepared from the organolithium intermediate (27) by addition of the organolithium (27) to sulfuryl chloride as described by Equation 24. The reaction is carried out at from -30° to -20°C in a mixture of ether and hexane, preferably one part ether to one part hexane. The sulfonyl chlorides (13) are isolated by aqueous work up and extraction with an organic solvent. The preparation of sulfonyl chlorides from organolithium species by treatment with sulfuryl chloride is discussed in J. Chem. Soc. (C), 1265 (1968).

The intermediate bromoaryl ethers (26) are prepared by alkylation of the appropriate bromophenols by methods well known in the art.

Some of the sulfonyl chlorides (13) in which $R_1$ does not contain functionality reactive with chlorine or aqueous acid can be prepared by the method described by Equation 25.

Equation 25

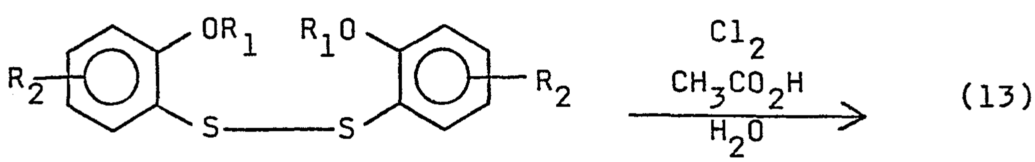


(29)

The disulfide intermediates (29) are treated with chlorine and water in a solvent such as acetic acid or a mixture of hydrochloric and nitric acids at a temperature of from 0° to 70°C for from one hour to several hours. The reaction mixture is diluted with cold water and the sulfonyl chloride (13) is separated by filtration, in the case of a solid product, or by decanting off the aqueous layer, in the case of a liquid product.

The disulfide intermediates (29) are prepared by alkylation of the bis-phenol disulfides of Formula (30) with the appropriate alkylating agent or activated aryl fluoride in the presence of a base such as potassium carbonate in a solvent such as methyl ethyl ketone or dimethylformamide, as illustrated by Equation 26 below.

Equation 26

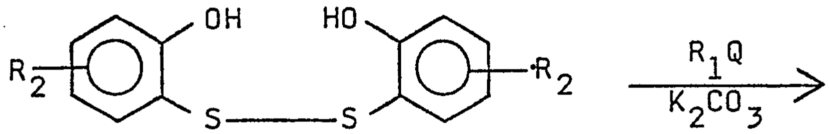


(29)

(30)

The bis-phenol disulfides (30) are themselves prepared from the appropriate mercaptophenols by oxidation with reagents such as oxygen in a basic medium or iodine in a solvent such as benzene or toluene.

Certain sulfonyl chlorides of Formula (13) in which $R_1$ is a substituent stable to acidic conditions, especially those in which $R_2$ is a 5-F, 5-Cl, 5-Br, 5-$CH_3$ or 5-$CF_3$ substituent, may be prepared by chlorosulfonation on the appropriately substituted aryl alkyl ether of Formula (31) as illustrated by Equation 27, below.

Equation 27

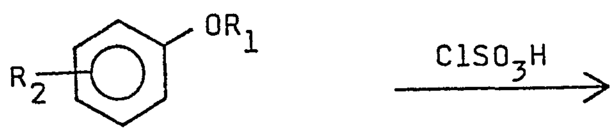


(13)

(31)

It is to be understood that an appropriate substitution pattern in substrates of Formula (31), consistent with the theories of electrophilic aromatic substitution (see, for instance, J. March, "Advanced

Organic Chemistry", pp. 383-390, McGraw-Hill, New York, 1968), is necessary to avoid the formation of undesired regioisomers. Alternatively, undesired regioisomers may be separated by physical purification methods, such as recrystallization, distillation or chromatography.

Chlorosulfonation of aromatic substrates is well-known (e.g., L. F. Fieser and M. Fieser, "Advanced Organic Chemistry," 696-698, Reinhold, New York, 1961). Chlorosulfonation can be accomplished by addition of the aryl alkyl ether to chlorosulfonic acid or vice versa, optionally in the presence of a cosolvent, such as an alkane or chlorinated alkane (e.g., hexane, 1-chlorobutane, methylene chloride, etc.). The reaction temperature is not critical, with a range of about -5° to 50° being usuable and ambient temperature (e.g., 20° to 30°C) being preferred for convenience.

Reaction time at ambient temperature is about 1 to 24 hours, depending on the exact substrate being chlorosulfonated, with an overnight period (about 16 hours) generally being sufficient. The aromatic sulfonyl chloride is conveniently isolated from the reaction mixture by pouring the mixture into ice-water, followed by extracting it with a water-immiscible organic solvent in which the aromatic sulfonyl chloride is soluble. Such solvents include 1-chlorobutane, methylene chloride, 1,2-dichloroethane, ethyl acetate, toluene, and diethyl ether.

Agriculturally suitable salts of compounds of Formula (1) are also useful herbicides and can be prepared in a number of ways known to the art. For example, metal salts can be made by contacting compounds of Formula (1) with a solution of an alkali or alkaline earth metal salt having a sufficiently basic anion (e.g., alkoxide, carbonate or hydroxide).

Quaternary amine salts can be made by similar techniques.

Salts of compounds of Formula (1) can also be prepared by exchange of one cation for another. Cationic exchange can be effected by direct contact of an aqueous solution of a salt of a compound of Formula (1) (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula (1) (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula (1) with a suitable acid, e.g., p-toluenesulfonic acid, trichloroacetic acid or the like.

The compounds of this invention and their preparation are further illustrated by the following examples wherein temperatures are given in degrees centigrade and all parts are by weight unless otherwise indicated.

## Example 1

## 1-(Cyclopropylmethoxy)-2-nitrobenzene

A solution of 42 g (0.30 mol) 2-nitrophenol and 65 g (0.47 mol) potassium carbonate in 175 ml dimethylformamide was stirred at room temperature for 60 minutes. To this solution was added 28 g (0.30 mol) chloromethylcyclopropane and the resultant solution was heated to reflux for sixteen hours. The reaction mixture was cooled and the solvent was removed *in vacuo*. The residue was diluted with water and extracted three times with ether. The combined organic solutions were washed with water and saturated sodium chloride solution (brine), dried over magnesium sulfate and the solvent was removed *in vacuo* to give 47 g (0.24 mol) 1-(cyclopropylmethoxy)-2-nitrobenzene as a brown oil.

NMR ($CDCl_3$)δ: 0.3-0.9 (m, 4H);
0.9-1.6 (m, 1H);
3.9 (d, 2H); and
6.8-7.9 (m, 4H).

## Example 2

## 2-(Cyclopropylmethoxy)benzenamine

A solution of 44 g (0.23 mol) 1-(cyclopropylmethoxy)-2-nitrobenzene, 103 ml glacial acetic acid and 1000 ml absolute ethanol was heated to reflux. Iron powder (51 g, 0.91 mol) was added in portions, over thirty minutes. The thick gray mixture was heated under reflux for three hours, cooled, filtered through a pad of celite and the solvent was removed *in vacuo*. The brown residue was diluted with water and extracted with ether four times. The combined organic extracts were washed with water and brine and dried over sodium sulfate. The solvent was removed *in vacuo* to yield a brown oil (36.7 g, 0.22 mol).

NMR ($CDCl_3$)δ: 0.3-0.9 (m, 4H);
1.5 (m, 1H);
3.9 (d, 2H);
4.2 (s, 2H); and
6-8 (br s, 4H).

IR (neat) $cm^{-1}$: 3400, 3300 (d) ($NH_2$).

## Example 3

2-(Cyclopropylmethoxy)benzenesulfonamide

A solution of 2-(cyclopropylmethoxy)benzenamine (30 g, 0.18 mol) in 180 ml 20% sulfuric acid was cooled to 5°C and to this was added 12.4 g (0.18 mol) sodium nitrite dissolved in 29 ml water. The diazonium salt solution was then added, after stirring for twenty minutes, to a solution of 14 ml condensed sulfur dioxide gas (0.30 mol), 15.3 g cupric chloride dihydrate (0.09 mol) in 160 ml glacial acetic acid and 36 ml concentrated hydrochloric acid. After stirring for thirty minutes, 200 ml *n*-butyl chloride and 200 ml water was added and the reaction mixture was heated for sixteen hours at 35°C. The solution was poured into 2 liters of water and extracted several times with *n*-butyl chloride. The combined organic layers were washed with water, saturated sodium bicarbonate solution and brine; dried over magnesium sulfate and concentrated *in vacuo*. The infrared spectrum showed the absence of an $NH_2$ stretch at 3400 $cm^{-1}$. The crude sulfonyl chloride (26.9 g) was dissolved in 500 ml ether and cooled to 0°. To this cold solution was added, dropwise, 6.6 ml condensed ammonia gas. The mixture was warmed to ambient temperature and stirred overnight. The water soluble solid was filtered off and discarded. The ethereal solution was washed with water and brine, dried over magnesium sulfate and concentrated *in vacuo* to yield 18.7 g of crude sulfon-

amide. Recrystallization from butyl chloride/carbon tetrachloride gave 8.2 g (20%) of the desired product, m.p. 120-122°C.

NMR (DMSO-$d_6$)δ: 0.3-0.6 (m, 4H);
1.5 (m, 1H);
3.2 (s, 2H);
4.0 (d, 2H); and
6.8-7.8 (m, 4H).

IR (nujol) $cm^{-1}$: 3400, 3300 (d, $SO_2NH_2$).

Example 4

2-(Cyclopropylmethoxy)benzenesulfonyl isocyanate

A solution of 2-(cyclopropylmethoxy)benzenesulfonamide (4.8 g, 0.021 mol) in 25 ml thionyl chloride was heated to reflux for 20 hours. The solution was cooled and the thionyl chloride removed *in vacuo*. To the residue was added 38 ml of a 10.7% by weight solution of phosgene in toluene, and 3 drops pyridine. The solution was heated to reflux for three hours, cooled and filtered under $N_2$. The solvent was removed *in vacuo* and the residue was taken up in 30 ml of dry acetonitrile, and used without further purification.

IR (neat) $cm^{-1}$: 2210 ($SO_2NCO$).

Example 5

2-(Cyclopropylmethoxy)-N-[(4-methoxy-6-methylpyrimidin-2-yl)aminocarbonyl]benzenesulfonamide

To a solution of 0.7 g (0.005 mol) 2-amino-4-methoxy-6-methylpyrimidine in 5 ml acetonitrile was added 10 ml of the sulfonyl isocyanate solution from the previous example. The solution was stirred and heated to reflux, then cooled and filtered to remove unreacted heterocyclic amine. The filtrate was concentrated *in vacuo* and the product was triturated with

*n*-butyl chloride and filtered to yield 1.5 g solid, m.p. 110°C.

NMR (DMSO-$d_6$): 0.2-1.0 (m, 4H);
1.5 (m, 1H);
2.2 (br s, 3H);
3.8 (t, 2H);
4.0 (s, 3H);
6.5 (s, 1H);
7.0-8.0 (m, 4H); and
10.2 (s, 1H).

IR (nujol) 1710 $cm^{-1}$.

## Example 6

### 2-Hydroxybenzenesulfonamide

To a solution of 50 ml of 85% potassium hydroxide in 100 ml of water was added 49 g (0.195 mol) of 2-hydroxybenzenesulfonamide methanesulfonate. The solution was stirred for four hours at room temperature then washed with ether. The aqueous solution was cooled to 0°C and was acidified with concentrated hydrochloric acid. The mixture was extracted with ethyl acetate and the extracts were combined. The extracts were washed with water and brine and dried over magnesium sulfate. Evaporation of the solvents under reduced pressure yielded 32.2 g (95%) of the title compound; m.p. 134-138°C.

## Example 7

### Methyl 2-[[2-[(butylaminocarbonyl)aminosulfonyl]-4-chlorophenoxy]]acetate

To 36 g of methyl 2-(2-aminosulfonyl-4-chlorophenoxy)acetate (*J. Chem. Soc. Perkins Trans. I.*, 2451 (1974)) and 15 g of anhydrous potassium carbonate was added 15 g of butyl isocyanate. The mixture was stirred and heated to reflux for three hours, allowed to

cool to room temperature and stirred for an additional sixteen hours. After evaporation of the solvent the residue was stirred in 200 ml of water and the mixture acidified to pH 3 by the addition of hydrochloric acid. The product precipitated as a solid and a tar. The solid was removed by filtration. The tar was dissolved in methanol and this solution diluted with water to yield additional solid product that was then combined with the first solid. The product (42.7 g) thus obtained melted at 119-123° and showed infrared absorption peaks at 1700 $cm^{-1}$ and 1720 $cm^{-1}$ consistent with two carbonyl groups.

Example 8

Methyl 2-(4-chloro-2-isocyanatosulfonylphenoxy)acetate

Into a mixture of 25 g of methyl 2-[2-(n-butylaminocarbonyl)aminosulfonyl-4-chlorophenoxy]acetate and 200 ml of xylene heated to reflux (135°) was passed phosgene until the reflux temperature dropped to 120° whereupon the addition was discontinued. The addition was resumed when the reflux temperature rose to 130° and again discontinued at 120°. When the reflux temperature did not rise above 120°, without further addition of phosgene, the reaction was considered complete. An infrared absorption spectrum of the reaction mixture showed the absence of absorption peaks at 3200 $cm^{-1}$ and the appearance of a strong absorption peak at 2220 $cm^{-1}$ consistent with the conversion of the reactant to the desired sulfonyl isocyanate. The solvent and butyl isocyanate by-product were removed *in vacuo* and the residue (21.0 g) thus obtained was suitable for use as an intermediate reagent for the preparation of herbicidal compounds of this invention.

Example 9

Methyl [4-chloro-2-[[(4-methoxy-6-methyl-2-pyrimidinyl)aminocarbonyl]aminosulfonyl]phenoxy]acetate

To 1.4 g of 2-amino-4-methoxy-6-methylpyrimidine in 25 ml of anhydrous acetonitrile at ambient temperature was added with stirring 3.0 g of methyl 2-(4-chloro-2-isocyanatosulfonylphenoxy)acetate. The mixture was stirred for sixteen hours and filtered to yield the product as a white crystalline solid melting at 169-173°. It showed absorption peaks by infrared analysis at 1650 and 1700 $cm^{-1}$, consistent with the urea function and 1720 $cm^{-1}$ for the ester group.

NMR (TFA-d): 2.5 (s, 3, $CH_3$);
3.8 (s, 3, $OCH_3$);
4.02 (s, 3, $OCH_3$); and
6.6-8.0 (broad, 4, aryl H).

Example 10

2-(2-Hydroxyethoxy)benzenesulfonamide

A mixture of 74.6 g (0.43 mol) of 2-hydroxybenzenesulfonamide, 45.3 g (0.51 mol) of ethylene carbonate and 0.9 g of imidazole was heated to 160° with vigorous stirring. After about four hours the carbon dioxide evolution had subsided and no unreacted 2-hydroxybenzenesulfonamide was present in the reaction mixture. The mixture was allowed to cool and about 400 ml of diethyl ether was added to the thick oil. The two-phase mixture was stirred vigorously and was heated to reflux for about one hour. The diethyl ether was decanted off and a fresh portion of diethyl ether was added. The mixture was again stirred and heated to reflux. After the diethyl ether was decanted off a fresh portion of diethyl ether was added and the mixture was allowed to stand at ambient temperature for about 18 hours. The resulting light

brown solid was isolated by filtration and was recrystallized from nitromethane to afford 49 g (53%) of the title compound; m.p. 94-99°C. The infrared spectrum exhibited a broad peak at approximately 3400 $cm^{-1}$ consistent with the primary alcohol of the product and peaks at 3370 $cm^{-1}$ and 3270 $cm^{-1}$ consistent with the $NH_2$ of a primary sulfonamide.

NMR ($DMSO-d_6$): δ 3.7-3.95 (m, 2, $-C\underline{H}_2OH$); 4.05-4.3 (m, 2, $ArOC\underline{H}_2-$); 6.3-7.05 (broad s, 2, $NH_2$); and 7.05-7.9 (m, 4, aromatic H).

Example 11

2-(2-Hydroxyethoxy)benzenesulfonamide, Methanesulfonate ester

A mixture of 24.5 g (0.113 mol) of 2-(2-hydroxyethoxy)benzenesulfonamide and about 0.1 g of 4-dimethylaminopyridine was dissolved in 200 ml of dry tetrahydrofuran. The solution was cooled to 4° under a calcium sulfate drying tube and 9.6 ml (14.2 g, 0.124 mol) of methanesulfonyl chloride was added. The slow addition of 24 ml (17.1 g, 0.169 mol) of triethylamine was then begun. The rate of addition was controlled such that the reaction temperature was maintained between 0° and 10°. After the addition was complete the mixture was allowed to warm to ambient temperature and stirred for 3 hours. The reaction mixture was then poured into a mixture of 500 ml of 5% hydrochloric acid and about 1000 g of ice. The solid product was collected by filtration, washed with water and then washed with a small amount of diethyl ether. After the product had dried it was recrystallized from 2-propanol to afford 15.3 g (46%) of the title compound; m.p. 120-125°C. The infrared spectrum exhibited peaks

at 3290 $cm^{-1}$ and 3270 $cm^{-1}$ consistent with the $NH_2$ of a primary sulfonamide and peaks at 1345 $cm^{-1}$, 1325 $cm^{-1}$, 1175 $cm^{-1}$ and 1160 $cm^{-1}$ consistent with the $SO_2$ groups of a sulfonamide and a sulfonate ester.

NMR ($DMSO-d_6$): δ 3.25 (s, 3, $OSO_2CH_3$);
4.3-4.8 (m, 4, $OCH_2CH_2O$);
7.0 (broad s, 2, $NH_2$); and
7.15-8.0 (m, 4, aromatic H).

## Example 12

N-[(4-Methoxy-6-methylpyrimidin-2-yl)aminocarbonyl]-2-(2-methylsulfonyloxyethoxy)benzenesulfonamide

To 1.5 g (0.01 mol) of 2-(2-hydroxyethoxy)benzenesulfonamide methanesulfonate ester suspended in 100 ml of dry dichloromethane under a nitrogen atmosphere was added dropwise 3.0 ml (0.012 mol) of a 2M solution of trimethylaluminum in toluene. Gas evolution and a mild exotherm occured during the addition of the trimethylaluminum solution and the mixture slowly became homogeneous. After stirring the solution at ambient temperature for 1.5 hours, 1.1 g (0.011 mol) of methyl 4-methoxy-6-methylpyrimidin-2-ylcarbamate was added in one portion. The resulting mixture was heated to reflux under a nitrogen atmosphere for 18 hours. The mixture was then cooled and 50 ml of 5% hydrochloric acid was slowly added. The organic layer was separated and the aqueous layer was extracted with two 50 ml portions of dichloromethane. The organic solutions were combined and washed with 50 ml of saturated sodium chloride solution. After drying over anhydrous magnesium sulfate, the solvents were evaporated to yield 2.2 g of the crude product which was recrystallized to yield 0.4 g of the title compound; m.p. 176-179°C. The infrared spectrum exhibited a band at 1700 $cm^{-1}$, consistent with the carbonyl group of a sulfonylurea.

NMR (DMSO-$d_6$): δ 2.4 (s, 3, pyrimidine $CH_3$);
3.1 (s, 3, $OSO_2CH_3$);
3.9 (s, 3, $OCH_3$);
4.4 (s, 4, $OCH_2CH_2O$);
6.55 (s, 1, pyrimidine 5-H);
7.1-8.05 (m, 4, aromatic H);
10.45 (broad s, 1, NH); and
13.15 (broad s, 1, NH).

## Example 13

2-(2-Hydroxyethoxy)benzenesulfonyl isocyanate methanesulfonate ester

A mixture of 15.0 g (0.051 mol) of 2-(2-hydroxyethoxy)benzenesulfonamide methanesulfonate ester and 90 ml of xylenes was heated to reflux to dissolve the sulfonamide. Heating was interrupted while 6.9 ml (6.05 g, 0.061 mol) of *n*-butyl isocyanate and about 0.1 g of 1,4-diazabicyclo[2.2.2]octane were added. The mixture was heated again to reflux. Approximately 15 ml of condensed phosgene gas was added in portions and heating was continued for about three hours. An additional 10 ml of phosgene was added and heating was stopped. The xylene solution was decanted away from an insoluble brown residue and the xylenes were evaporated under reduced pressure. The presence of the sulfonyl isocyanate group was confirmed by an intense band in the infrared spectrum at 2210 $cm^{-1}$. The crude product was used without further purification.

## Example 14

N-[(4,6-Dimethylpyrimidin-2-yl)aminocarbonyl]-2-(2-hydroxyethoxy)benzenesulfonamide

To a suspension of 1.2 g (0.0097 mol) of 2-amino-4,6-dimethylpyrimidine and a few crystals of 1,4-diazabicyclo[2.2.2]octane in 10 ml of dichloromethane was

added a solution of approximately 0.01 mol of 2-(2-hydroxyethoxy)benzenesulfonyl isocyanate methanesulfonate ester dissolved in 10 ml of dichloromethane. A mild exotherm was noted upon mixing and a homogeneous solution resulted. The mixture was refluxed for eight hours then stirred at ambient temperature for five days. The solution was filtered and the filtrate was evaporated under reduced pressure. Trituration of the residue with diethyl ether afforded a crude solid which was recrystallized from a mixture of 1-chlorobutane and acetonitrile to yield 2.0 g of the title compound; m.p. 183-190°C. The infrared spectrum exhibited a broad band at 3390 $cm^{-1}$ consistent with a primary alcohol and a band at 1695 $cm^{-1}$ consistent with the carbonyl group of a sulfonylurea. The mass spectrum exhibited a molecular ion at m/e = 366 and fragmentation consistent with the assigned structure.

NMR (DMSO-$d_6$): δ 2.45 (s, 6, pyrimidine $CH_3$);
3.7-3.9 (m, 2, -C$\underline{H}_2$OH);
4.25-4.5 (m, 2, ArOC$\underline{H}_2$-);
7.05 (s, 1, pyrimidine 5-H);
7.05-7.4 and 7.65-8.1 (m, 4, aromatic H);
10.55 (broad s, 1, NH); and
13.2 (broad s, 1, NH).

By the methods discussed above and the examples provided, the following compounds can be made.

Table I

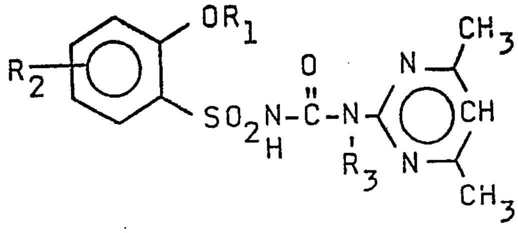

| $R_1$ | $R_2$ | $R_3$ | m.p.(°C) |
|---|---|---|---|
| $-CH(CH_3)CO_2CH_3$ | H | H | 188-194° |
| $-CH_2CO_2C_2H_5$ | H | H | |
| $-CH(CH_3)CO_2C_2H_5$ | H | H | |
| $-CH_2-$ 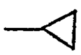 | H | H | 148-167° |
| $-CH(CH_3)-$ 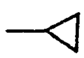 | H | H | |
| 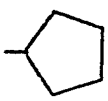 | H | H | 200-201° |
| $-CH_2CN$ | H | H | |
| $-CH(CH_3)CN$ | H | H | |
| $-CH(OCH_3)CN$ | H | H | |
| $-CH(OCH_3)CO_2CH_3$ | H | H | |
| $-CH(OCH_3)CO_2C_2H_5$ | H | H | |
| $-CH_2SO_2N(CH_3)_2$ | H | H | |
| $-CH(CH_3)SO_2N(CH_3)_2$ | H | H | |
| $-CH_2CH_2N(CH_3)_2$ | H | H | |
| $-CH_2CH_2CN$ | H | H | |
| $-CH_2CH_2CO_2CH_3$ | H | H | |
| $-CH_2CH_2CO_2C_2H_5$ | H | H | |
| $-CH_2CH_2SO_2N(CH_3)_2$ | H | H | |
| $-CH_2-$ 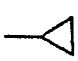 | $3-OCH_3$ | H | |
| $-CH_2-$ 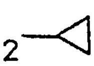 | $4-OCH_3$ | H | |

Table I (continued)

| $R_1$ | $R_2$ | $R_3$ | m.p.(°C) |
|---|---|---|---|
| $-CH_2$ 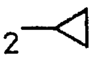 | 5-$OCH_3$ | H | |
| $-CH_2$ 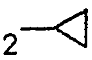 | 6-$OCH_3$ | H | |
| $-CH_2$ 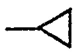 | 3-$CF_3$ | H | |
| $-CH_2$ 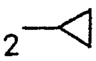 | 4-$CF_3$ | H | |
| $-CH_2$ 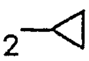 | 5-$CF_3$ | H | |
| $-CH_2$ 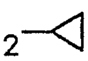 | 6-$CF_3$ | H | |
| $-CH_2$ 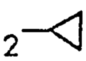 | 3-Cl | H | |
| $-CH_2$ 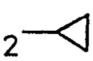 | 4-Cl | H | |
| $-CH_2$ 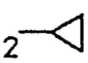 | 5-Cl | H | |
| $-CH_2$ 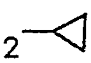 | 6-Cl | H | |
| $-CH_2$ 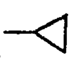 | 5-Br | H | |
| $-CH_2$ 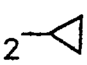 | 6-Br | H | |
| $-CH_2$ 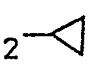 | 3-Br | H | |
| $-CH_2$ 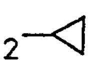 | 5-F | H | |
| $-CH_2$ 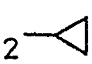 | 6-F | H | |
| $-CH_2C\equiv CH$ | 3-$OCH_3$ | H | |
| $-CH_2C\equiv CH$ | 4-$OCH_3$ | H | |
| $-CH_2C\equiv CH$ | 5-$OCH_3$ | H | |
| $-CH_2C\equiv CH$ | 6-$OCH_3$ | H | |
| $-CH_2C\equiv CH$ | 3-$CF_3$ | H | |
| $-CH_2C\equiv CH$ | 5-$CF_3$ | H | |

Table I (continued)

| $R_1$ | $R_2$ | $R_3$ | m.p.(°C) |
|---|---|---|---|
| $-CH_2C\equiv CH$ | 3-Cl | H | |
| $-CH_2C\equiv CH$ | 5-Cl | H | |
| $-CH_2C\equiv CH$ | 6-Cl | H | |
| $-CH_2C\equiv CH$ | $5-CH_3$ | H | |
| $-CH_2C\equiv CH$ | 5-Br | H | |
| $-CH_2C\equiv CH$ | 5-F | H | |
| $-CH(CH_3)CO_2CH_3$ | $3-OCH_3$ | H | |
| $-CH(CH_3)CO_2CH_3$ | $5-OCH_3$ | H | |
| $-CH(CH_3)CO_2CH_3$ | $6-OCH_3$ | H | |
| $-CH(CH_3)CO_2CH_3$ | $5-CF_3$ | H | |
| $-CH(CH_3)CO_2CH_3$ | 5-Cl | H | |
| $-CH(CH_3)CO_2CH_3$ | $5-CH_3$ | H | |
| $-CH_2CO_2C_2H_5$ | $5-OCH_3$ | H | |
| $-CH_2CO_2C_2H_5$ | $5-CF_3$ | H | |
| $-CH_2CO_2C_2H_5$ | 5-Br | H | |
| $-CH(CH_3)CO_2C_2H_5$ | $5-OCH_3$ | H | |
| $-CH(CH_3)CO_2C_2H_5$ | $5-CF_3$ | H | |
| $-CH(CH_3)CO_2C_2H_5$ | 5-Cl | H | |
| $-CH(CH_3)CO_2C_2H_5$ | $5-CH_3$ | H | |
| $-CH(CH_3)CO_2C_2H_5$ | 5-F | H | |
| $-CH_2CN$ | $5-OCH_3$ | H | |
| $-CH_2CN$ | $6-OCH_3$ | H | |
| $-CH_2CN$ | $5-CF_3$ | H | |
| $-CH_2CN$ | 5-Cl | H | |
| $-CH(CH_3)CN$ | $5-OCH_3$ | H | |
| $-CH_2SO_2N(CH_3)_2$ | $5-CF_3$ | H | |
| $-CH(CH_3)SO_2N(CH_3)_2$ | $5-OCH_3$ | H | |
| $-CH_2CH_2N(CH_3)_2$ | 5-Cl | H | |
| $-CH_2CH_2N(CH_3)_2$ | $5-CF_3$ | H | |
| $-CH_2CH_2N(CH_3)_2$ | $5-OCH_3$ | H | |
| $-CH_2CH_2CN$ | $5-CF_3$ | H | |

Table I (continued)

| $R_1$ | $R_2$ | $R_3$ | m.p.(°C) |
|---|---|---|---|
| $-CH_2CH_2CN$ | 5-$OCH_3$ | H | |
| $-CH_2CH_2CN$ | 5-Br | H | |
| $-CH_2CH_2CO_2CH_3$ | 3-$OCH_3$ | H | |
| $-CH_2CH_2CO_2CH_3$ | 5-$CF_3$ | H | |
| $-CH_2CH_2CO_2CH_3$ | 6-$OCH_3$ | H | |
| $-CH_2CH_2CO_2CH_3$ | 5-Cl | H | |
| $-CH_2CH_2CO_2C_2H_5$ | 3-Cl | H | |
| $-CH_2CH_2CO_2C_2H_5$ | 5-$CF_3$ | H | |
| $-CH_2CH_2CO_2C_2H_5$ | 5-$OCH_3$ | H | |
| $-CH_2CH_2SO_2N(CH_3)_2$ | 5-$CF_3$ | H | |
| $-CH_2CH_2SO_2N(CH_3)_2$ | 5-Cl | H | |
| $-CH_2C{\equiv}CH$ | H | $CH_3$ | |
| $-CH(CH_3)CO_2CH_3$ | H | $CH_3$ | |
| $-CH_2CO_2C_2H_5$ | H | $CH_3$ | |
| $-CH(CH_3)CO_2C_2H_5$ | H | $CH_3$ | |
| $-CH_2$ 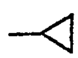 | H | $CH_3$ | |
| $-CH(CH_3)$ 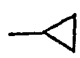 | H | $CH_3$ | |
| $-CH_2CN$ | H | $CH_3$ | |
| $-CH(CH_3)CN$ | H | $CH_3$ | |
| $-CH_2CH_2CN$ | H | $CH_3$ | |
| $-CH_2CH_2CO_2CH_3$ | H | $CH_3$ | |
| $-CH_2CH_2N(CH_3)_2$ | H | $CH_3$ | |
| $-CH_2CH_2CO_2C_2H_5$ | H | $CH_3$ | |
| $-CH_2SO_2N(CH_3)_2$ | H | $CH_3$ | |
| $-CH(CH_3)SO_2N(CH_3)_2$ | H | $CH_3$ | |
| $-CH_2CH_2SO_2N(CH_3)_2$ | H | $CH_3$ | |
| 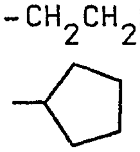 | H | $CH_3$ | |
| $-CH_2C{\equiv}CH$ | 5-Cl | $CH_3$ | |

Table I (continued)

| $R_1$ | $R_2$ | $R_3$ | m.p.(°C) |
|---|---|---|---|
| $-CH(CH_3)CO_2CH_3$ | 5-F | $CH_3$ | |
| $-CH_2CO_2C_2H_5$ | $5-CH_3$ | $CH_3$ | |
| $-CH(CH_3)CO_2C_2H_5$ | $5-OCH_3$ | $CH_3$ | |
| 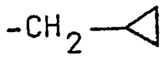 | $5-OCH_3$ | $CH_3$ | |
| 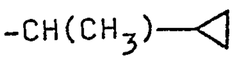 | $5-CF_3$ | $CH_3$ | |
| $-CH_2CN$ | $5-OCH_3$ | $CH_3$ | |
| $-CH_2CH_2CN$ | $5-CH_3$ | $CH_3$ | |
| $-CH(CH_3)CN$ | $5-CF_3$ | $CH_3$ | |
| $-CH_2CH_2CO_2CH_3$ | $5-OCH_3$ | $CH_3$ | |
| $-CH_2CH_2CO_2C_2H_5$ | $5-CF_3$ | $CH_3$ | |
| $-CH_2SO_2N(CH_3)_2$ | $5-OCH_3$ | $CH_3$ | |
| $OCH_3$<br>$-CHCO_2CH_3$ | H | $CH_3$ | |
| $OCH_3$<br>$-CHCO_2C_2H_5$ | H | $CH_3$ | |
| $OCH_3$<br>$-CHCN$ | H | $CH_3$ | |
| 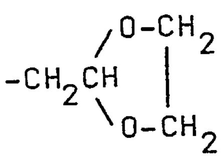 | H | H | |
| 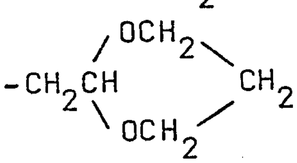 | H | H | |
| 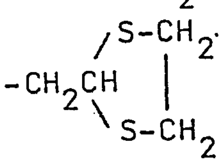 | H | H | |
| 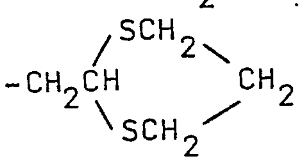 | H | H | |

Table I (continued)

| $R_1$ | $R_2$ | $R_3$ | m.p.(°C) |
|---|---|---|---|
| 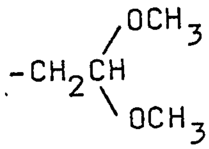 | H | H | |
| 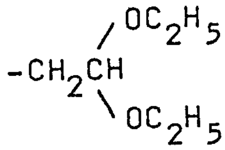 | H | H | |
| 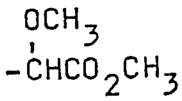 | 5-$CF_3$ | H | |
| 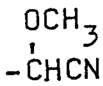 | 5-Cl | H | |
| 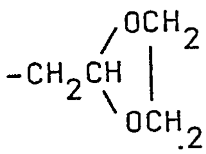 | 5-$CF_3$ | H | |
| 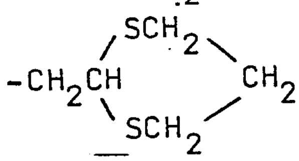 | 3-$OCH_3$ | H | |
| 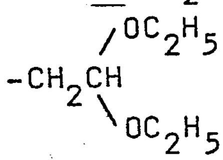 | 5-$CF_3$ | H | |
| 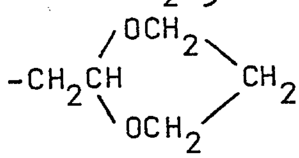 | H | $CH_3$ | |
| $CH_2CH_2CH_2CH_2CH_3$ | H | H | |
| 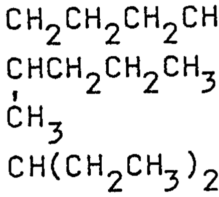 | H | H | 179-181° |
| $CH(CH_2CH_3)_2$ | H | H | 196-197° |
| $CH_2CH_2CH_2CH_2CH_3$ | H | $CH_3$ | |
| 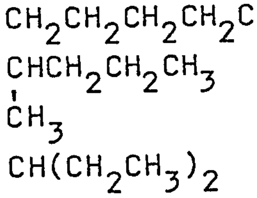 | H | $CH_3$ | |
| $CH(CH_2CH_3)_2$ | H | $CH_3$ | |
| $CH_2CH_2CH_2CH_2CH_3$ | 5-$OCH_3$ | H | |

Table I (continued)

| $R_1$ | $R_2$ | $R_3$ | m.p.(°C) |
|---|---|---|---|
| $CH_2CH_2CH_2CH_2CH_3$ | 5-$CF_3$ | H | |
| $CH_2CH_2CH_2CH_2CH_3$ | 5-Cl | H | |
| $CH_2CH_2CH_2CH_2CH_3$ | 5-$CH_3$ | H | |
| $CH_2CH_2CH_2CH_2CH_3$ | 5-Br | H | |
| $CH_2CH_2CH_2CH_2CH_3$ | 5-F | H | |
| $CH_2CH_2CH_2CH_2CH_3$ | 6-$CH_3$ | H | |
| $CH_2CH_2CH_2CH_2CH_3$ | 6-$OCH_3$ | H | |
| $CH_2CH_2CH_2CH_2CH_3$ | 6-F | H | |
| $-CH_2CH_2-OH$ | H | H | 183-190° |
| $CH_3$<br>$-CH-CH_2OH$ | H | H | |
| $CH_3$ $CH_3$<br>$-CH—CH-OH$ | H | H | |
| $CH_3$ $CH_3$<br>$-CH—C-OH$<br>$CH_3$ | H | H | |
| $CH_3$<br>$-CH_2-CH-OH$ | H | H | |
| $CH_3$<br>$-CH_2-C-OH$<br>$CH_3$ | H | H | |
| $-CH_2-CH_2-CH_2-OH$ | H | H | |
| $CH_3$<br>$-CH-CH_2-CH_2-OH$ | H | H | |
| $CH_3$<br>$-CH_2-CH-CH_2-OH$ | H | H | |
| $CH_3$<br>$-CH_2-CH_2-CH-OH$ | H | H | |
| $-CH_2-CH_2-OH$ | H | $CH_3$ | |
| $CH_3$<br>$-CH-CH_2-OH$ | H | $CH_3$ | |

Table I (continued)

| $R_1$ | $R_2$ | $R_3$ | m.p.(°C) |
|---|---|---|---|
| 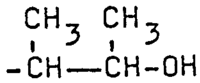 | H | $CH_3$ | |
| 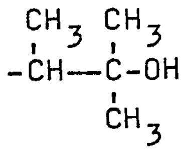 | H | $CH_3$ | |
| 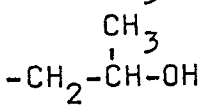 | H | $CH_3$ | |
| 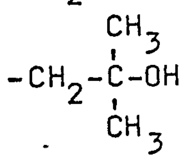 | H | $CH_3$ | |
| $-CH_2-CH_2-CH_2-OH$ | H | $CH_3$ | |
| 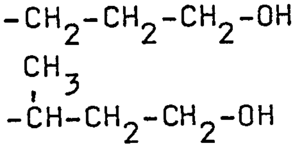 | H | $CH_3$ | |
| 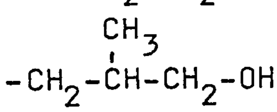 | H | $CH_3$ | |
| 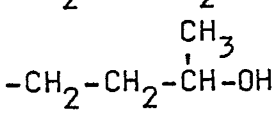 | H | $CH_3$ | |
| $-CH_2-CH_2-OH$ | 3-F | H | |
| $-CH_2-CH_2-OH$ | 5-F | H | |
| $-CH_2-CH_2-OH$ | 5-Cl | H | |
| $-CH_2-CH_2-OH$ | 5-Br | H | |
| $-CH_2-CH_2-OH$ | $5-CH_3$ | H | |
| $-CH_2-CH_2-OH$ | $3-OCH_3$ | H | |
| $-CH_2-CH_2-OH$ | $5-OCH_3$ | H | |
| $-CH_2-CH_2-OH$ | $6-OCH_3$ | H | |
| $-CH_2-CH_2-OH$ | $5-CF_3$ | H | |
| $-CH_2-CH_2-CH_2-OH$ | 5-F | H | |
| $-CH_2-CH_2-CH_2-OH$ | 5-Cl | H | |
| $-CH_2-CH_2-CH_2-OH$ | 5-Br | H | |
| $-CH_2-CH_2-CH_2-OH$ | $5-CH_3$ | H | |
| $-CH_2-CH_2-CH_2-OH$ | $3-OCH_3$ | H | |
| $-CH_2-CH_2-CH_2-OH$ | $5-OCH_3$ | H | |

Table I (continued)

| $R_1$ | $R_2$ | $R_3$ | m.p.(°C) |
|---|---|---|---|
| $-CH_2-CH_2-CH_2-OH$ | $6-OCH_3$ | H | |
| $-CH_2-CH_2-CH_2-OH$ | $5-CF_3$ | H | |
| $CH_3$<br>$-CH-CH_2-OH$ | $5-CF_3$ | H | |
| $CH_3$<br>$-CH-CH_2-OH$ | 5-Br | H | |
| $CH_3$<br>$-CH_2CH-OH$ | $5-CF_3$ | H | |
| $-CH_2-CH_2-OH$ | $5-CF_3$ | $CH_3$ | |
| $-CH_2-CH_2-OH$ | 5-Br | $CH_3$ | |
| $-CH_2-CH_2-OH$ | $3-OCH_3$ | $CH_3$ | |
| $-CH_2-CH_2-CH_2-OH$ | $5-CF_3$ | $CH_3$ | |
| $-CH_2-CH_2-OC(O)CH_3$ | H. | H | |
| $-CH_2-CH_2-OC(O)C_2H_5$ | H | H | |
| $-CH_2-CH_2-OC(O)CH_2-CH_2-CH_3$ | H | H | |
| $-CH_2-CH_2-OC(O)-CH(CH_3)_2$ | H | H | |
| $-CH_2-CH_2-OC(O)C_6H_5$ | H | H | |
| $-CH_2-CH_2-OC(O)NHCH_3$ | H | H | |
| $-CH_2-CH_2-OC(O)NHC_2H_5$ | H | H | |
| $-CH_2-CH_2-OC(O)N(CH_3)_2$ | H | H | |
| $CH_3$<br>$-CH_2-CH_2-OC(O)N$<br>$OCH_3$ | H | H | |
| $-CH_2-CH_2-OC(O)NC_6H_5$ | H | H | |
| $-CH_2-CH_2-OSO_2CF_3$ | H | H | |
| $-CH_2-CH_2-OSO_2CH_3$ | H | H | |
| $-CH_2-CH_2-OSO_2C_2H_5$ | H | H | |
| $-CH_2-CH_2-OSO_2C_6H_5$ | H | H | |
| $-CH_2-CH_2-OSO_2-$ 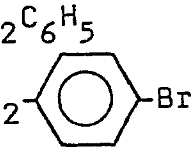 | H | H | |
| $-CH_2-CH_2-OSO_2-$ 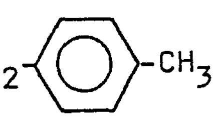 | H | H | |

Table I (continued)

| $R_1$ | $R_2$ | $R_3$ | m.p.(°C) |
|---|---|---|---|
| $-CH_2-CH_2-OSO_2CH_3$ | 5-Br | H | |
| $-CH_2-CH_2-OSO_2CH_3$ | $5-CF_3$ | H | |
| $-CH_2-CH_2OSO_2CF_3$ | $5-CF_3$ | H | |
| $-CH_2-CH_2-OC(O)CH_3$ | $5-CF_3$ | H | |
| $-CH_2-CH_2-OSO_2CH_3$ | H | $CH_3$ | |
| $-CH_2-CH_2-OC(O)CH_3$ | H | $CH_3$ | |
| $-CH_2-CH_2-CH_2-OSO_2CH_3$ | $5-CF_3$ | H | |
| $-CH_2-CH_2-CH_2-OSO_2CH_3$ | H | $CH_3$ | |
| $-CH_2-CH_2-CH_2-OC(O)CH_3$ | H | $CH_3$ | |
| $CH_3$<br>$-CH_2CH-OSO_2CH_3$ | H | H | |
| $CH_3$ $CH_3$<br>$-CH—CH-OSO_2CH_3$ | H | H | |
| $CH_3$<br>$-CH_2CH_2-CH-OSO_2CH_3$ | H | H | |
| $CH_3$<br>$-CH_2CHCH_2-OSO_2CH_3$ | H | H | |
| $-CH_2C{\equiv}CH$ | H | H | 222-224 |

Table II

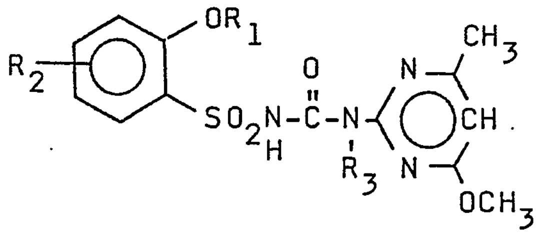

| $R_1$ | $R_2$ | $R_3$ | m.p.(°C) |
|---|---|---|---|
| $-CH_2CO_2CH_3$ | H | H | 174-176° |
| $-CH(CH_3)CO_2CH_3$ | H | H | 162-165° |
| $-CH_2CO_2C_2H_5$ | H | H | |
| $-CH(CH_3)CO_2C_2H_5$ | H | H | |
| $-CH_2$ 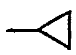 | H | H | 110° |
| $-CH(CH_3)$ 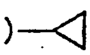 | H | H | |
| 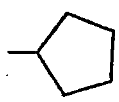 | H | H | |
| $-CH_2CN$ | H | H | |
| $-CH(CH_3)CN$ | H | H | |
| $-CH(OCH_3)CN$ | H | H | |
| $-CH(OCH_3)CO_2CH_3$ | H | H | 176-179° |
| $-CH(OCH_3)CO_2C_2H_5$ | H | H | |
| $-CH_2SO_2N(CH_3)_2$ | H | H | |
| $-CH(CH_3)SO_2N(CH_3)_2$ | H | H | |
| $-CH_2CH_2N(CH_3)_2$ | H | H | |
| $-CH_2CH_2CN$ | H | H | |
| $-CH_2CH_2CO_2CH_3$ | H | H | |
| $-CH_2CH_2CO_2C_2H_5$ | H | H | |
| $-CH_2CH_2SO_2N(CH_3)_2$ | H | H | |
| $-CH_2$ 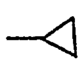 | 3-$OCH_3$ | H | |
| $-CH_2$ 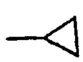 | 4-$OCH_3$ | H | |

Table II (continued)

| $R_1$ | $R_2$ | $R_3$ | m.p.(°C) |
|---|---|---|---|
| $-CH_2$ 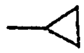 | 5-$OCH_3$ | H | |
| $-CH_2$ 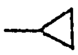 | 6-$OCH_3$ | H | |
| $-CH_2$ 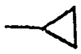 | 3-$CF_3$ | H | |
| $-CH_2$ 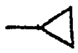 | 4-$CF_3$ | H | |
| $-CH_2$ 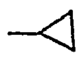 | 5-$CF_3$ | H | |
| $-CH_2$ 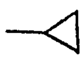 | 6-$CF_3$ | H | |
| $-CH_2$ 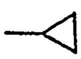 | 3-Cl | H | 84-90° |
| $-CH_2$ 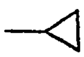 | 4-Cl | H | |
| $-CH_2$ 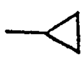 | 5-Cl | H | |
| $-CH_2$ 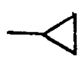 | 6-Cl | H | |
| $-CH_2$ 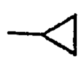 | 5-Br | H | |
| $-CH_2$ 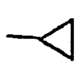 | 6-Br | H | |
| $-CH_2$ 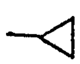 | 3-Br | H | |
| $-CH_2$ 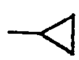 | 5-F | H | |
| $-CH_2$ 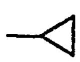 | 6-F | H | |
| $-CH_2C\equiv CH$ | 3-$OCH_3$ | H | |
| $-CH_2C\equiv CH$ | 4-$OCH_3$ | H | |
| $-CH_2C\equiv CH$ | 5-$OCH_3$ | H | |
| $-CH_2C\equiv CH$ | 6-$OCH_3$ | H | |
| $-CH_2C\equiv CH$ | 3-$CF_3$ | H | |
| $-CH_2C\equiv CH$ | 5-$CF_3$ | H | |

Table II (continued)

| $R_1$ | $R_2$ | $R_3$ | m.p.(°C) |
|---|---|---|---|
| $-CH_2C\equiv CH$ | 3-Cl | H | |
| $-CH_2C\equiv CH$ | 5-Cl | H | 169-173° |
| $-CH_2C\equiv CH$ | 6-Cl | H | |
| $-CH_2C\equiv CH$ | $5-CH_3$ | H | |
| $-CH_2C\equiv CH$ | 5-Br | H | |
| $-CH_2C\equiv CH$ | 5-F | H | |
| $-CH(CH_3)CO_2CH_3$ | $3-OCH_3$ | H | |
| $-CH(CH_3)CO_2CH_3$ | $5-OCH_3$ | H | |
| $-CH(CH_3)CO_2CH_3$ | $6-OCH_3$ | H | |
| $-CH(CH_3)CO_2CH_3$ | $5-CF_3$ | H | |
| $-CH(CH_3)CO_2CH_3$ | 5-Cl | H | |
| $-CH(CH_3)CO_2CH_3$ | $5-CH_3$ | H | |
| $-CH_2CO_2C_2H_5$ | $5-OCH_3$ | H | |
| $-CH_2CO_2C_2H_5$ | $5-CF_3$ | H | |
| $-CH_2CO_2C_2H_5$ | 5-Br | H | |
| $-CH(CH_3)CO_2C_2H_5$ | $5-OCH_3$ | H | |
| $-CH(CH_3)CO_2C_2H_5$ | $5-CF_3$ | H | |
| $-CH(CH_3)CO_2C_2H_5$ | 5-Cl | H | |
| $-CH(CH_3)CO_2C_2H_5$ | $5-CH_3$ | H | |
| $-CH(CH_3)CO_2C_2H_5$ | 5-F | H | |
| $-CH_2CN$ | $5-OCH_3$ | H | |
| $-CH_2CN$ | $6-OCH_3$ | H | |
| $-CH_2CN$ | $5-CF_3$ | H | |
| $-CH_2CN$ | 5-Cl | H | |
| $-CH(CH_3)CN$ | $5-OCH_3$ | H | |
| $-CH_2SO_2N(CH_3)_2$ | $5-CF_3$ | H | |
| $-CH(CH_3)SO_2N(CH_3)_2$ | $5-OCH_3$ | H | |
| $-CH_2CH_2N(CH_3)_2$ | 5-Cl | H | |
| $-CH_2CH_2N(CH_3)_2$ | $5-CF_3$ | H | |
| $-CH_2CH_2N(CH_3)_2$ | $5-OCH_3$ | H | |
| $-CH_2CH_2CN$ | $5-CF_3$ | H | |

Table II (continued)

| $R_1$ | $R_2$ | $R_3$ | m.p.(°C) |
|---|---|---|---|
| $-CH_2CH_2CN$ | 5-$OCH_3$ | H | |
| $-CH_2CH_2CN$ | 5-Br | H | |
| $-CH_2CH_2CO_2CH_3$ | 3-$OCH_3$ | H | |
| $-CH_2CH_2CO_2CH_3$ | 5-$CF_3$ | H | |
| $-CH_2CH_2CO_2CH_3$ | 6-$OCH_3$ | H | |
| $-CH_2CH_2CO_2CH_3$ | 5-Cl | H | |
| $-CH_2CH_2CO_2C_2H_5$ | 3-Cl | H | |
| $-CH_2CH_2CO_2C_2H_5$ | 5-$CF_3$ | H | |
| $-CH_2CH_2CO_2C_2H_5$ | 5-$OCH_3$ | H | |
| $-CH_2CH_2SO_2N(CH_3)_2$ | 5-$CF_3$ | H | |
| $-CH_2CH_2SO_2N(CH_3)_2$ | 5-Cl | H | |
| $-CH_2C{\equiv}CH$ | H | $CH_3$ | |
| $-CH(CH_3)CO_2CH_3$ | H | $CH_3$ | |
| $-CH_2CO_2C_2H_5$ | H | $CH_3$ | |
| $-CH(CH_3)CO_2C_2H_5$ | H | $CH_3$ | |
| $-CH_2$ 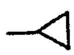 | H | $CH_3$ | |
| $-CH(CH_3)$ 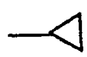 | H | $CH_3$ | |
| $-CH_2CN$ | H | $CH_3$ | |
| $-CH(CH_3)CN$ | H | $CH_3$ | |
| $-CH_2CH_2CN$ | H | $CH_3$ | |
| $-CH_2CH_2CO_2CH_3$ | H | $CH_3$ | |
| $-CH_2CH_2N(CH_3)_2$ | H | $CH_3$ | |
| $-CH_2CH_2CO_2C_2H_5$ | H | $CH_3$ | |
| $-CH_2SO_2N(CH_3)_2$ | H | $CH_3$ | |
| $-CH(CH_3)SO_2N(CH_3)_2$ | H | $CH_3$ | |
| $-CH_2CH_2SO_2N(CH_3)_2$ | H | $CH_3$ | |
| 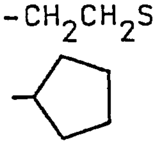 | H | $CH_3$ | |
| $-CH_2C{\equiv}CH$ | 5-Cl | $CH_3$ | |

Table II (continued)

| $R_1$ | $R_2$ | $R_3$ | m.p.(°C) |
|---|---|---|---|
| $-CH(CH_3)CO_2CH_3$ | 5-F | $CH_3$ | |
| $-CH_2CO_2C_2H_5$ | 5-$CH_3$ | $CH_3$ | |
| $-CH(CH_3)CO_2C_2H_5$ | 5-$OCH_3$ | $CH_3$ | |
| 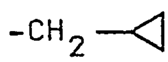 | 5-$OCH_3$ | $CH_3$ | |
| 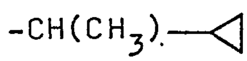 | 5-$CF_3$ | $CH_3$ | |
| $-CH_2CN$ | 5-$OCH_3$ | $CH_3$ | |
| $-CH_2CH_2CN$ | 5-$CH_3$ | $CH_3$ | |
| $-CH(CH_3)CN$ | 5-$CF_3$ | $CH_3$ | |
| $-CH_2CH_2CO_2CH_3$ | 5-$OCH_3$ | $CH_3$ | |
| $-CH_2CH_2CO_2C_2H_5$ | 5-$CF_3$ | $CH_3$ | |
| $-CH_2SO_2N(CH_3)_2$ | 5-$OCH_3$ | $CH_3$ | |
| 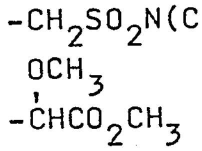 | H | $CH_3$ | |
| 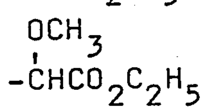 | H | $CH_3$ | |
| 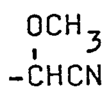 | H | $CH_3$ | |
| 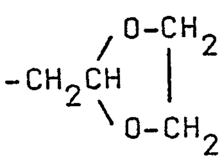 | H | H | |
| 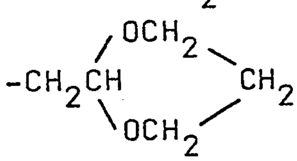 | H | H | |
| 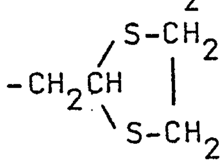 | H | H | |
| 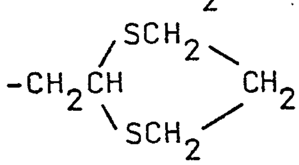 | H | H | |

Table II (continued)

| $R_1$ | $R_2$ | $R_3$ | m.p.(°C) |
|---|---|---|---|
| 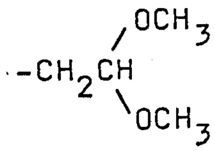 | H | H | |
| 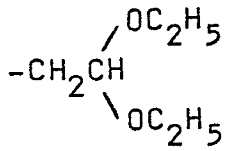 | H | H | |
| 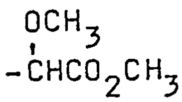 | 5-$CF_3$ | H | |
| 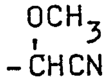 | 5-Cl | H | |
| 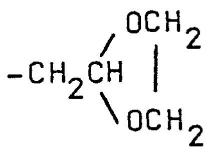 | 5-$CF_3$ | H | |
| 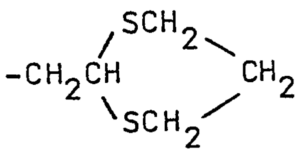 | 3-$OCH_3$ | H | |
| 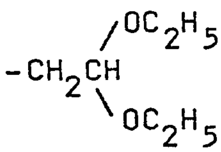 | 5-$CF_3$ | H | |
| 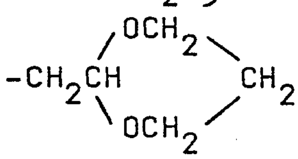 | H | $CH_3$ | |
| $CH_2CH_2CH_2CH_2CH_3$ | H | H | 163-165° |
| 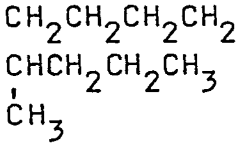 | H | H | 200-202° |
| $CH(CH_2CH_3)_2$ | H | H | 186-189° |
| $CH_2CH_2CH_2CH_2CH_3$ | H | $CH_3$ | |
| 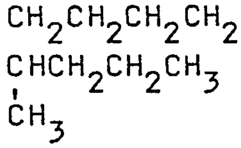 | H | $CH_3$ | |
| $CH(CH_2CH_3)_2$ | H | $CH_3$ | |
| $CH_2CH_2CH_2CH_2CH_3$ | 5-$OCH_3$ | H | |

Table II (continued)

| $R_1$ | $R_2$ | $R_3$ | m.p.(°C) |
|---|---|---|---|
| $CH_2CH_2CH_2CH_2CH_3$ | 5-$CF_3$ | H | |
| $CH_2CH_2CH_2CH_2CH_3$ | 5-Cl | H | |
| $CH_2CH_2CH_2CH_2CH_3$ | 5-$CH_3$ | H | |
| $CH_2CH_2CH_2CH_2CH_3$ | 5-Br | H | |
| $CH_2CH_2CH_2CH_2CH_3$ | 5-F | H | |
| $CH_2CH_2CH_2CH_2CH_3$ | 6-$CH_3$ | H | |
| $CH_2CH_2CH_2CH_2CH_3$ | 6-$OCH_3$ | H | |
| $CH_2CH_2CH_2CH_2CH_3$ | 6-F | H | |
| $-CH_2CH_2-OH$ | H | H | 200-204° |
| $CH_3$<br>$-CH-CH_2OH$ | H | H | |
| $CH_3$ $CH_3$<br>$-CH—CH-OH$ | H | H | |
| $CH_3$ $CH_3$<br>$-CH—C-OH$<br>$CH_3$ | H | H | |
| $CH_3$<br>$-CH_2-CH-OH$ | H | H | |
| $CH_3$<br>$-CH_2-C-OH$<br>$CH_3$ | H | H | |
| $-CH_2-CH_2-CH_2-OH$ | H | H | |
| $CH_3$<br>$-CH-CH_2-CH_2-OH$ | H | H | |
| $CH_3$<br>$-CH_2-CH-CH_2-OH$ | H | H | |
| $CH_3$<br>$-CH_2-CH_2-CH-OH$ | H | H | |
| $-CH_2-CH_2-OH$ | H | $CH_3$ | |
| $CH_3$<br>$-CH-CH_2-OH$ | H | $CH_3$ | |

Table II (continued)

| $R_1$ | $R_2$ | $R_3$ | m.p.(°C) |
|---|---|---|---|
| 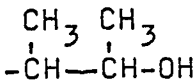 | H | $CH_3$ | |
| 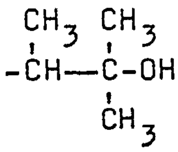 | H | $CH_3$ | |
| 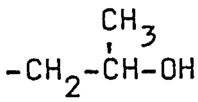 | H | $CH_3$ | |
| 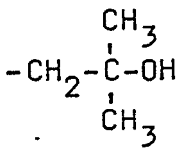 | H | $CH_3$ | |
| $-CH_2-CH_2-CH_2-OH$ | H | $CH_3$ | |
| 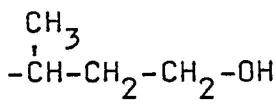 | H | $CH_3$ | |
| 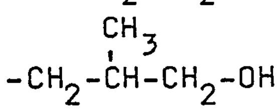 | H | $CH_3$ | |
| 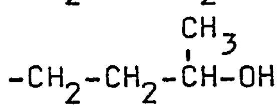 | H | $CH_3$ | |
| $-CH_2-CH_2-OH$ | 3-F | H | |
| $-CH_2-CH_2-OH$ | 5-F | H | |
| $-CH_2-CH_2-OH$ | 5-Cl | H | |
| $-CH_2-CH_2-OH$ | 5-Br | H | |
| $-CH_2-CH_2-OH$ | $5-CH_3$ | H | |
| $-CH_2-CH_2-OH$ | $3-OCH_3$ | H | |
| $-CH_2-CH_2-OH$ | $5-OCH_3$ | H | |
| $-CH_2-CH_2-OH$ | $6-OCH_3$ | H | |
| $-CH_2-CH_2-OH$ | $5-CF_3$ | H | |
| $-CH_2-CH_2-CH_2-OH$ | 5-F | H | |
| $-CH_2-CH_2-CH_2-OH$ | 5-Cl | H | |
| $-CH_2-CH_2-CH_2-OH$ | 5-Br | H | |
| $-CH_2-CH_2-CH_2-OH$ | $5-CH_3$ | H | |
| $-CH_2-CH_2-CH_2-OH$ | $3-OCH_3$ | H | |
| $-CH_2-CH_2-CH_2-OH$ | $5-OCH_3$ | H | |

Table II (continued)

| $R_1$ | $R_2$ | $R_3$ | m.p.(°C) |
|---|---|---|---|
| $-CH_2-CH_2-CH_2-OH$ | $6-OCH_3$ | H | |
| $-CH_2-CH_2-CH_2-OH$ | $5-CF_3$ | H | |
| $CH_3$<br>$-CH-CH_2-OH$ | $5-CF_3$ | H | |
| $CH_3$<br>$-CH-CH_2-OH$ | 5-Br | H | |
| $CH_3$<br>$-CH_2CH-OH$ | $5-CF_3$ | H | |
| $-CH_2-CH_2-OH$ | $5-CF_3$ | $CH_3$ | |
| $-CH_2-CH_2-OH$ | 5-Br | $CH_3$ | |
| $-CH_2-CH_2-OH$ | $3-OCH_3$ | $CH_3$ | |
| $-CH_2-CH_2-CH_2-OH$ | $5-CF_3$ | $CH_3$ | |
| $-CH_2-CH_2-OC(O)CH_3$ | H | H | |
| $-CH_2-CH_2-OC(O)C_2H_5$ | H | H | |
| $-CH_2-CH_2-OC(O)CH_2-CH_2-CH_3$ | H | H | |
| $-CH_2-CH_2-OC(O)-CH(CH_3)_2$ | H | H | |
| $-CH_2-CH_2-OC(O)C_6H_5$ | H | H | |
| $-CH_2-CH_2-OC(O)NHCH_3$ | H | H | |
| $-CH_2-CH_2-OC(O)NHC_2H_5$ | H | H | |
| $-CH_2-CH_2-OC(O)N(CH_3)_2$ | H | H | |
| $/CH_3$<br>$-CH_2-CH_2-OC(O)N$<br>$\backslash OCH_3$ | H | H | |
| $-CH_2-CH_2-OC(O)NC_6H_5$ | H | H | |
| $-CH_2-CH_2-OSO_2CF_3$ | H | H | |
| $-CH_2-CH_2-OSO_2CH_3$ | H | H | 176-179° |
| $-CH_2-CH_2-OSO_2C_2H_5$ | H | H | |
| $-CH_2-CH_2-OSO_2C_6H_5$ | H | H | |
| $-CH_2-CH_2-OSO_2-$ 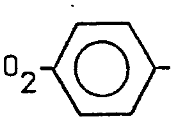 Br | H | H | |
| $-CH_2-CH_2-OSO_2-$ 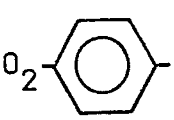 $CH_3$ | H | H | |

Table II (continued)

| $R_1$ | $R_2$ | $R_3$ | m.p.(°C) |
|---|---|---|---|
| $-CH_2-CH_2-OSO_2CH_3$ | 5-Br | H | |
| $-CH_2-CH_2-OSO_2CH_3$ | $5-CF_3$ | H | |
| $-CH_2-CH_2OSO_2CF_3$ | $5-CF_3$ | H | |
| $-CH_2-CH_2-OC(O)CH_3$ | $5-CF_3$ | H | |
| $-CH_2-CH_2-OSO_2CH_3$ | H | $CH_3$ | |
| $-CH_2-CH_2-OC(O)CH_3$ | H | $CH_3$ | |
| $-CH_2-CH_2-CH_2-OSO_2CH_3$ | $5-CF_3$ | H | |
| $-CH_2-CH_2-CH_2-OSO_2CH_3$ | H | $CH_3$ | |
| $-CH_2-CH_2-CH_2-OC(O)CH_3$ | H | $CH_3$ | |
| $CH_3$<br>$-CH_2CH-OSO_2CH_3$ | H | H | |
| $CH_3$ $CH_3$<br>$-CH—CH-OSO_2CH_3$ | H | H | |
| $CH_3$<br>$-CH_2CH_2-CH-OSO_2CH_3$ | H | H | |
| $CH_3$<br>$-CH_2CHCH_2-OSO_2CH_3$ | H | H | |
| $-CH_2C≡CH$ | H | H | 189-193 |

Table III

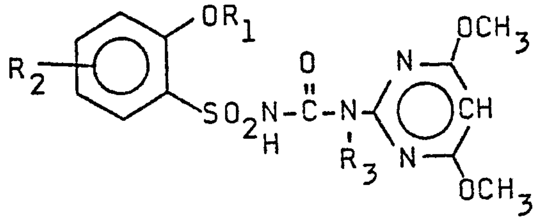

| $R_1$ | $R_2$ | $R_3$ | m.p.(°C) |
|---|---|---|---|
| $-CH_2CO_2CH_3$ | H | H | 190-192° |
| $-CH(CH_3)CO_2CH_3$ | H | H | 185-191° |
| $-CH_2CO_2C_2H_5$ | H | H | |
| $-CH(CH_3)CO_2C_2H_5$ | H | H | |
| $-CH_2$ 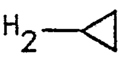 | H | H | 133-140° |
| $-CH(CH_3)$ 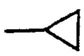 | H | H | |
| 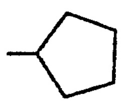 | H | H | 211-212° |
| $-CH_2CN$ | H | H | |
| $-CH(CH_3)CN$ | H | H | |
| $-CH(OCH_3)CN$ | H | H | |
| $-CH(OCH_3)CO_2CH_3$ | H | H | 190-193° |
| $-CH(OCH_3)CO_2C_2H_5$ | H | H | |
| $-CH_2SO_2N(CH_3)_2$ | H | H | |
| $-CH(CH_3)SO_2N(CH_3)_2$ | H | H | |
| $-CH_2CH_2N(CH_3)_2$ | H | H | |
| $-CH_2CH_2CN$ | H | H | |
| $-CH_2CH_2CO_2CH_3$ | H | H | |
| $-CH_2CH_2CO_2C_2H_5$ | H | H | |
| $-CH_2CH_2SO_2N(CH_3)_2$ | H | H | |
| $-CH_2$ 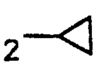 | 3-$OCH_3$ | H | |
| $-CH_2$ 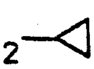 | 4-$OCH_3$ | H | |

Table II (continued)

| $R_1$ | $R_2$ | $R_3$ | m.p.(°C) |
|---|---|---|---|
| $-CH_2-CH_2-OSO_2CH_3$ | 5-Br | H | |
| $-CH_2-CH_2-OSO_2CH_3$ | $5-CF_3$ | H | |
| $-CH_2-CH_2OSO_2CF_3$ | $5-CF_3$ | H | |
| $-CH_2-CH_2-OC(O)CH_3$ | $5-CF_3$ | H | |
| $-CH_2-CH_2-OSO_2CH_3$ | H | $CH_3$ | |
| $-CH_2-CH_2-OC(O)CH_3$ | H | $CH_3$ | |
| $-CH_2-CH_2-CH_2-OSO_2CH_3$ | $5-CF_3$ | H | |
| $-CH_2-CH_2-CH_2-OSO_2CH_3$ | H | $CH_3$ | |
| $-CH_2-CH_2-CH_2-OC(O)CH_3$ | H | $CH_3$ | |
| $-CH_2CH(CH_3)-OSO_2CH_3$ | H | H | |
| $-CH(CH_3)-CH(CH_3)-OSO_2CH_3$ | H | H | |
| $-CH_2CH_2-CH(CH_3)-OSO_2CH_3$ | H | H | |
| $-CH_2CH(CH_3)CH_2-OSO_2CH_3$ | H | H | |
| $-CH_2C\equiv CH$ | H | H | 189-193 |

Table III

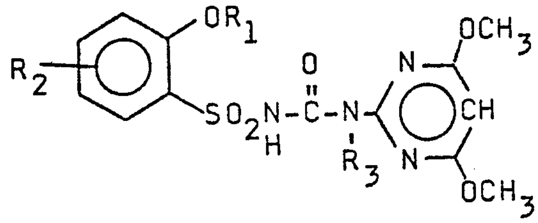

| $R_1$ | $R_2$ | $R_3$ | m.p.(°C) |
|---|---|---|---|
| $-CH_2CO_2CH_3$ | H | H | 190-192° |
| $-CH(CH_3)CO_2CH_3$ | H | H | 185-191° |
| $-CH_2CO_2C_2H_5$ | H | H | |
| $-CH(CH_3)CO_2C_2H_5$ | H | H | |
| $-CH_2-$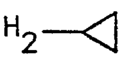 | H | H | 133-140° |
| $-CH(CH_3)-$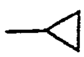 | H | H | |
| 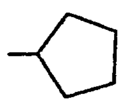 | H | H | 211-212° |
| $-CH_2CN$ | H | H | |
| $-CH(CH_3)CN$ | H | H | |
| $-CH(OCH_3)CN$ | H | H | |
| $-CH(OCH_3)CO_2CH_3$ | H | H | 190-193° |
| $-CH(OCH_3)CO_2C_2H_5$ | H | H | |
| $-CH_2SO_2N(CH_3)_2$ | H | H | |
| $-CH(CH_3)SO_2N(CH_3)_2$ | H | H | |
| $-CH_2CH_2N(CH_3)_2$ | H | H | |
| $-CH_2CH_2CN$ | H | H | |
| $-CH_2CH_2CO_2CH_3$ | H | H | |
| $-CH_2CH_2CO_2C_2H_5$ | H | H | |
| $-CH_2CH_2SO_2N(CH_3)_2$ | H | H | |
| $-CH_2-$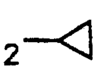 | $3-OCH_3$ | H | |
| $-CH_2-$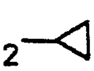 | $4-OCH_3$ | H | |

Table III (continued)

| $R_1$ | $R_2$ | $R_3$ | m.p.(°C) |
|---|---|---|---|
| $-CH_2$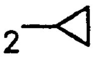 | 5-$OCH_3$ | H | |
| $-CH_2$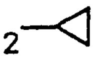 | 6-$OCH_3$ | H | |
| $-CH_2$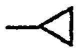 | 3-$CF_3$ | H | |
| $-CH_2$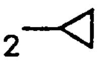 | 4-$CF_3$ | H | |
| $-CH_2$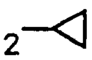 | 5-$CF_3$ | H | |
| $-CH_2$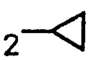 | 6-$CF_3$ | H | |
| $-CH_2$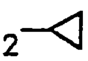 | 3-Cl | H | |
| $-CH_2$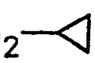 | 4-Cl | H | |
| $-CH_2$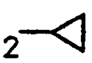 | 5-Cl | H | |
| $-CH_2$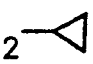 | 6-Cl | H | |
| $-CH_2$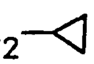 | 5-Br | H | |
| $-CH_2$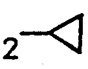 | 6-Br | H | |
| $-CH_2$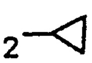 | 3-Br | H | |
| $-CH_2$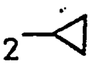 | 5-F | H | |
| $-CH_2$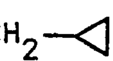 | 6-F | H | |
| $-CH_2C{\equiv}CH$ | 3-$OCH_3$ | H | |
| $-CH_2C{\equiv}CH$ | 4-$OCH_3$ | H | |
| $-CH_2C{\equiv}CH$ | 5-$OCH_3$ | H | |
| $-CH_2C{\equiv}CH$ | 6-$OCH_3$ | H | |
| $-CH_2C{\equiv}CH$ | 3-$CF_3$ | H | |
| $-CH_2C{\equiv}CH$ | 5-$CF_3$ | H | |

Table III (continued)

| $R_1$ | $R_2$ | $R_3$ | m.p.(°C) |
|---|---|---|---|
| $-CH_2C≡CH$ | 3-Cl | H | |
| $-CH_2C≡CH$ | 5-Cl | H | |
| $-CH_2C≡CH$ | 6-Cl | H | |
| $-CH_2C≡CH$ | $5-CH_3$ | H | |
| $-CH_2C≡CH$ | 5-Br | H | |
| $-CH_2C≡CH$ | 5-F | H | |
| $-CH(CH_3)CO_2CH_3$ | $3-OCH_3$ | H | |
| $-CH(CH_3)CO_2CH_3$ | $5-OCH_3$ | H | |
| $-CH(CH_3)CO_2CH_3$ | $6-OCH_3$ | H | |
| $-CH(CH_3)CO_2CH_3$ | $5-CF_3$ | H | |
| $-CH(CH_3)CO_2CH_3$ | 5-Cl | H | |
| $-CH(CH_3)CO_2CH_3$ | $5-CH_3$ | H | |
| $-CH_2CO_2C_2H_5$ | $5-OCH_3$ | H | |
| $-CH_2CO_2C_2H_5$ | $5-CF_3$ | H | |
| $-CH_2CO_2C_2H_5$ | 5-Br | H | |
| $-CH(CH_3)CO_2C_2H_5$ | $5-OCH_3$ | H | |
| $-CH(CH_3)CO_2C_2H_5$ | $5-CF_3$ | H | |
| $-CH(CH_3)CO_2C_2H_5$ | 5-Cl | H | |
| $-CH(CH_3)CO_2C_2H_5$ | $5-CH_3$ | H | |
| $-CH(CH_3)CO_2C_2H_5$ | 5-F | H | |
| $-CH_2CN$ | $5-OCH_3$ | H | |
| $-CH_2CN$ | $6-OCH_3$ | H | |
| $-CH_2CN$ | $5-CF_3$ | H | |
| $-CH_2CN$ | 5-Cl | H | |
| $-CH(CH_3)CN$ | $5-OCH_3$ | H | |
| $-CH_2SO_2N(CH_3)_2$ | $5-CF_3$ | H | |
| $-CH(CH_3)SO_2N(CH_3)_2$ | $5-OCH_3$ | H | |
| $-CH_2CH_2N(CH_3)_2$ | 5-Cl | H | |
| $-CH_2CH_2N(CH_3)_2$ | $5-CF_3$ | H | |
| $-CH_2CH_2N(CH_3)_2$ | $5-OCH_3$ | H | |
| $-CH_2CH_2CN$ | $5-CF_3$ | H | |

Table III (continued)

| $R_1$ | $R_2$ | $R_3$ | m.p.(°C) |
|---|---|---|---|
| $-CH_2CH_2CN$ | 5-$OCH_3$ | H | |
| $-CH_2CH_2CN$ | 5-Br | H | |
| $-CH_2CH_2CO_2CH_3$ | 3-$OCH_3$ | H | |
| $-CH_2CH_2CO_2CH_3$ | 5-$CF_3$ | H | |
| $-CH_2CH_2CO_2CH_3$ | 6-$OCH_3$ | H | |
| $-CH_2CH_2CO_2CH_3$ | 5-Cl | H | |
| $-CH_2CH_2CO_2C_2H_5$ | 3-Cl | H | |
| $-CH_2CH_2CO_2C_2H_5$ | 5-$CF_3$ | H | |
| $-CH_2CH_2CO_2C_2H_5$ | 5-$OCH_3$ | H | |
| $-CH_2CH_2SO_2N(CH_3)_2$ | 5-$CF_3$ | H | |
| $-CH_2CH_2SO_2N(CH_3)_2$ | 5-Cl | H | |
| $-CH_2C{\equiv}CH$ | H | $CH_3$ | |
| $-CH(CH_3)CO_2CH_3$ | H | $CH_3$ | |
| $-CH_2CO_2C_2H_5$ | H | $CH_3$ | |
| $-CH(CH_3)CO_2C_2H_5$ | H | $CH_3$ | |
| $-CH_2$ 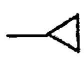 | H | $CH_3$ | |
| $-CH(CH_3)$ 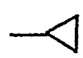 | H | $CH_3$ | |
| $-CH_2CN$ | H | $CH_3$ | |
| $-CH(CH_3)CN$ | H | $CH_3$ | |
| $-CH_2CH_2CN$ | H | $CH_3$ | |
| $-CH_2CH_2CO_2CH_3$ | H | $CH_3$ | |
| $-CH_2CH_2N(CH_3)_2$ | H | $CH_3$ | |
| $-CH_2CH_2CO_2C_2H_5$ | H | $CH_3$ | |
| $-CH_2SO_2N(CH_3)_2$ | H | $CH_3$ | |
| $-CH(CH_3)SO_2N(CH_3)_2$ | H | $CH_3$ | |
| $-CH_2CH_2SO_2N(CH_3)_2$ | H | $CH_3$ | |
| 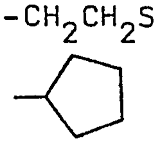 | H | $CH_3$ | |
| $-CH_2C{\equiv}CH$ | 5-Cl | $CH_3$ | |

Table III (continued)

| $R_1$ | $R_2$ | $R_3$ | m.p.(°C) |
|---|---|---|---|
| $-CH(CH_3)CO_2CH_3$ | 5-F | $CH_3$ | |
| $-CH_2CO_2C_2H_5$ | $5-CH_3$ | $CH_3$ | |
| $-CH(CH_3)CO_2C_2H_5$ | $5-OCH_3$ | $CH_3$ | |
| $-CH_2$ 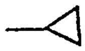 | $5-OCH_3$ | $CH_3$ | |
| $-CH(CH_3)$ 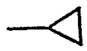 | $5-CF_3$ | $CH_3$ | |
| $-CH_2CN$ | $5-OCH_3$ | $CH_3$ | |
| $-CH_2CH_2CN$ | $5-CH_3$ | $CH_3$ | |
| $-CH(CH_3)CN$ | $5-CF_3$ | $CH_3$ | |
| $-CH_2CH_2CO_2CH_3$ | $5-OCH_3$ | $CH_3$ | |
| $-CH_2CH_2CO_2C_2H_5$ | $5-CF_3$ | $CH_3$ | |
| $-CH_2SO_2N(CH_3)_2$ | $5-OCH_3$ | $CH_3$ | |
| $-\overset{OCH_3}{C}HCO_2CH_3$ | H | $CH_3$ | |
| $-\overset{OCH_3}{C}HCO_2C_2H_5$ | H | $CH_3$ | |
| $-\overset{OCH_3}{C}HCN$ | H | $CH_3$ | |
| 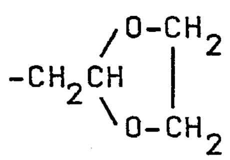 | H | H | 192-197° |
| 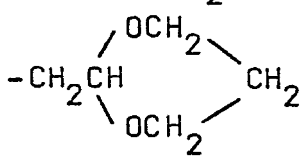 | H | H | |
| 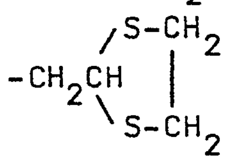 | H | H | |
| 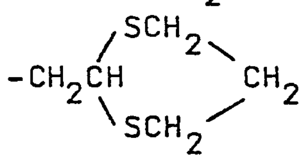 | H | H | |

Table III (continued)

| $R_1$ | $R_2$ | $R_3$ | m.p.(°C) |
|---|---|---|---|
| 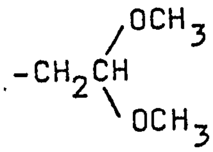 | H | H | |
| 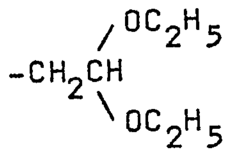 | H | H | |
| 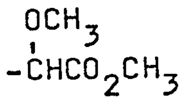 | 5-$CF_3$ | H | |
| 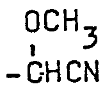 | 5-Cl | H | |
| 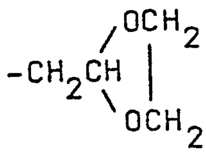 | 5-$CF_3$ | H | |
| 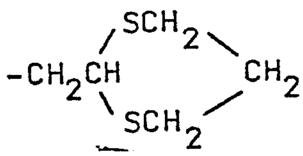 | 3-$OCH_3$ | H | |
| 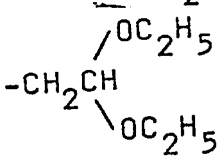 | 5-$CF_3$ | H | |
| 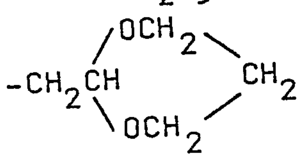 | H | $CH_3$ | |
| $CH_2CH_2CH_2CH_2CH_3$ | H | H | 180-183° |
| 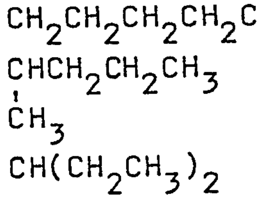 | H | H | 204-206° |
| $CH(CH_2CH_3)_2$ | H | H | 192-194° |
| $CH_2CH_2CH_2CH_2CH_3$ | H | $CH_3$ | |
| 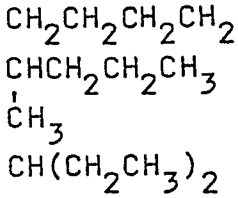 | H | $CH_3$ | |
| $CH(CH_2CH_3)_2$ | H | $CH_3$ | |
| $CH_2CH_2CH_2CH_2CH_3$ | 5-$OCH_3$ | H | |

Table III (continued)

| $R_1$ | $R_2$ | $R_3$ | m.p.(°C) |
|---|---|---|---|
| $CH_2CH_2CH_2CH_2CH_3$ | 5-$CF_3$ | H | |
| $CH_2CH_2CH_2CH_2CH_3$ | 5-Cl | H | |
| $CH_2CH_2CH_2CH_2CH_3$ | 5-$CH_3$ | H | |
| $CH_2CH_2CH_2CH_2CH_3$ | 5-Br | H | |
| $CH_2CH_2CH_2CH_2CH_3$ | 5-F | H | |
| $CH_2CH_2CH_2CH_2CH_3$ | 6-$CH_3$ | H | |
| $CH_2CH_2CH_2CH_2CH_3$ | 6-$OCH_3$ | H | |
| $CH_2CH_2CH_2CH_2CH_3$ | 6-F | H | |
| $-CH_2CH_2-OH$ | H | H | 195-199° |
| 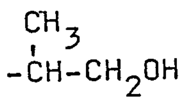 | H | H | |
| 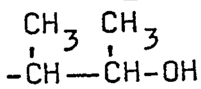 | H | H | |
| 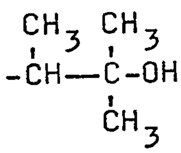 | H | H | |
| 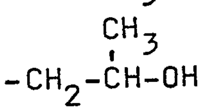 | H | H | |
| 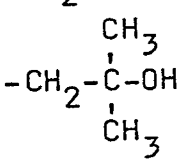 | H | H | |
| $-CH_2-CH_2-CH_2-OH$ | H | H | |
| 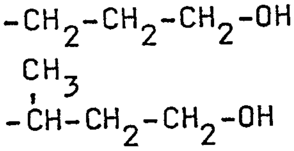 | H | H | |
| 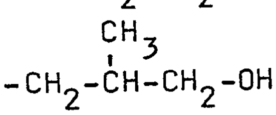 | H | H | |
| 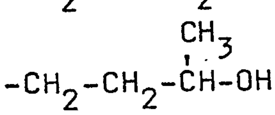 | H | H | |
| $-CH_2-CH_2-OH$ | H | $CH_3$ | |
| 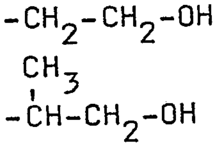 | H | $CH_3$ | |

Table III (continued)

| $R_1$ | $R_2$ | $R_3$ | m.p.(°C) |
|---|---|---|---|
| $CH_3$ $CH_3$<br>$-CH—CH-OH$ | H | $CH_3$ | |
| $CH_3$ $CH_3$<br>$-CH—C-OH$<br>$CH_3$ | H | $CH_3$ | |
| $CH_3$<br>$-CH_2-CH-OH$ | H | $CH_3$ | |
| $CH_3$<br>$-CH_2-C-OH$<br>$CH_3$ | H | $CH_3$ | |
| $-CH_2-CH_2-CH_2-OH$ | H | $CH_3$ | |
| $CH_3$<br>$-CH-CH_2-CH_2-OH$ | H | $CH_3$ | |
| $CH_3$<br>$-CH_2-CH-CH_2-OH$ | H | $CH_3$ | |
| $CH_3$<br>$-CH_2-CH_2-CH-OH$ | H | $CH_3$ | |
| $-CH_2-CH_2-OH$ | 3-F | H | |
| $-CH_2-CH_2-OH$ | 5-F | H | |
| $-CH_2-CH_2-OH$ | 5-Cl | H | |
| $-CH_2-CH_2-OH$ | 5-Br | H | |
| $-CH_2-CH_2-OH$ | 5-$CH_3$ | H | |
| $-CH_2-CH_2-OH$ | 3-$OCH_3$ | H | |
| $-CH_2-CH_2-OH$ | 5-$OCH_3$ | H | |
| $-CH_2-CH_2-OH$ | 6-$OCH_3$ | H | |
| $-CH_2-CH_2-OH$ | 5-$CF_3$ | H | |
| $-CH_2-CH_2-CH_2-OH$ | 5-F | H | |
| $-CH_2-CH_2-CH_2-OH$ | 5-Cl | H | |
| $-CH_2-CH_2-CH_2-OH$ | 5-Br | H | |
| $-CH_2-CH_2-CH_2-OH$ | 5-$CH_3$ | H | |
| $-CH_2-CH_2-CH_2-OH$ | 3-$OCH_3$ | H | |
| $-CH_2-CH_2-CH_2-OH$ | 5-$OCH_3$ | H | |

Table III (continued)

| $R_1$ | $R_2$ | $R_3$ | m.p.(°C) |
|---|---|---|---|
| $-CH_2-CH_2-CH_2-OH$ | $6-OCH_3$ | H | |
| $-CH_2-CH_2-CH_2-OH$ | $5-CF_3$ | H | |
| $CH_3$<br>$-CH-CH_2-OH$ | $5-CF_3$ | H | |
| $CH_3$<br>$-CH-CH_2-OH$ | 5-Br | H | |
| $CH_3$<br>$-CH_2CH-OH$ | $5-CF_3$ | H | |
| $-CH_2-CH_2-OH$ | $5-CF_3$ | $CH_3$ | |
| $-CH_2-CH_2-OH$ | 5-Br | $CH_3$ | |
| $-CH_2-CH_2-OH$ | $3-OCH_3$ | $CH_3$ | |
| $-CH_2-CH_2-CH_2-OH$ | $5-CF_3$ | $CH_3$ | |
| $-CH_2-CH_2-OC(O)CH_3$ | H | H | |
| $-CH_2-CH_2-OC(O)C_2H_5$ | H | H | |
| $-CH_2-CH_2-OC(O)CH_2-CH_2-CH_3$ | H | H | |
| $-CH_2-CH_2-OC(O)-CH(CH_3)_2$ | H | H | |
| $-CH_2-CH_2-OC(O)C_6H_5$ | H | H | |
| $-CH_2-CH_2-OC(O)NHCH_3$ | H | H | |
| $-CH_2-CH_2-OC(O)NHC_2H_5$ | H | H | |
| $-CH_2-CH_2-OC(O)N(CH_3)_2$ | H | H | |
| $/CH_3$<br>$-CH_2-CH_2-OC(O)N$<br>$\backslash OCH_3$ | H | H | |
| $-CH_2-CH_2-OC(O)NC_6H_5$ | H | H | |
| $-CH_2-CH_2-OSO_2CF_3$ | H | H | |
| $-CH_2-CH_2-OSO_2CH_3$ | H | H | |
| $-CH_2-CH_2-OSO_2C_2H_5$ | H | H | |
| $-CH_2-CH_2-OSO_2C_6H_5$ | H | H | |
| $-CH_2-CH_2-OSO_2-$ 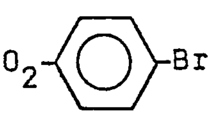 | H | H | |
| $-CH_2-CH_2-OSO_2-$ 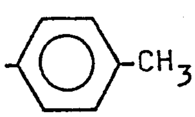 | H | H | |

Table III (continued)

| $R_1$ | $R_2$ | $R_3$ | m.p.(°C) |
|---|---|---|---|
| $-CH_2-CH_2-OSO_2CH_3$ | 5-Br | H | |
| $-CH_2-CH_2-OSO_2CH_3$ | 5-$CF_3$ | H | |
| $-CH_2-CH_2OSO_2CF_3$ | 5-$CF_3$ | H | |
| $-CH_2-CH_2-OC(O)CH_3$ | 5-$CF_3$ | H | |
| $-CH_2-CH_2-OSO_2CH_3$ | H | $CH_3$ | |
| $-CH_2-CH_2-OC(O)CH_3$ | H | $CH_3$ | |
| $-CH_2-CH_2-CH_2-OSO_2CH_3$ | 5-$CF_3$ | H | |
| $-CH_2-CH_2-CH_2-OSO_2CH_3$ | H | $CH_3$ | |
| $-CH_2-CH_2-CH_2-OC(O)CH_3$ | H | $CH_3$ | |
| $CH_3$<br>$-CH_2CH-OSO_2CH_3$ | H | H | |
| $CH_3$ $CH_3$<br>$-CH—CH-OSO_2CH_3$ | H | H | |
| $CH_3$<br>$-CH_2CH_2-CH-OSO_2CH_3$ | H | H | |
| $CH_3$<br>$-CH_2CHCH_2-OSO_2CH_3$ | H | H | |
| $-CH_2C\equiv CH$ | H | H | 218-223 |

## Table IV

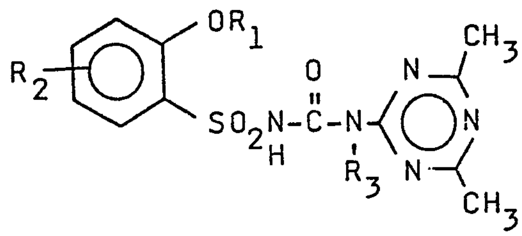

| $R_1$ | $R_2$ | $R_3$ | m.p.(°C) |
|---|---|---|---|
| $-CH(CH_3)CO_2CH_3$ | H | H | |
| $-CH_2CO_2C_2H_5$ | H | H | |
| $-CH(CH_3)CO_2C_2H_5$ | H | H | |
| $-CH_2$ 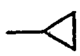 | H | H | |
| $-CH(CH_3)$ 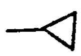 | H | H | |
| 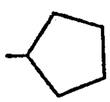 | H | H | |
| $-CH_2CN$ | H | H | |
| $-CH(CH_3)CN$ | H | H | |
| $-CH(OCH_3)CN$ | H | H | |
| $-CH(OCH_3)CO_2CH_3$ | H | H | |
| $-CH(OCH_3)CO_2C_2H_5$ | H | H | |
| $-CH_2SO_2N(CH_3)_2$ | H | H | |
| $-CH(CH_3)SO_2N(CH_3)_2$ | H | H | |
| $-CH_2CH_2N(CH_3)_2$ | H | H | |
| $-CH_2CH_2CN$ | H | H | |
| $-CH_2CH_2CO_2CH_3$ | H | H | |
| $-CH_2CH_2CO_2C_2H_5$ | H | H | |
| $-CH_2CH_2SO_2N(CH_3)_2$ | H | H | |
| $-CH_2$ 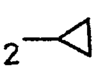 | $3-OCH_3$ | H | |
| $-CH_2$ 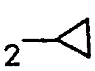 | $4-OCH_3$ | H | |

Table IV (continued)

| $R_1$ | $R_2$ | $R_3$ | m.p.(°C) |
|---|---|---|---|
| $-CH_2$ 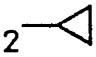 | 5-$OCH_3$ | H | |
| $-CH_2$ 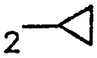 | 6-$OCH_3$ | H | |
| $-CH_2$ 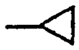 | 3-$CF_3$ | H | |
| $-CH_2$ 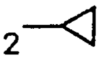 | 4-$CF_3$ | H | |
| $-CH_2$ 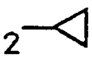 | 5-$CF_3$ | H | |
| $-CH_2$ 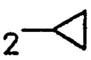 | 6-$CF_3$ | H | |
| $-CH_2$ 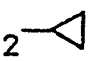 | 3-Cl | H | |
| $-CH_2$ 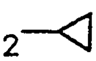 | 4-Cl | H | |
| $-CH_2$ 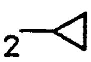 | 5-Cl | H | |
| $-CH_2$ 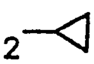 | 6-Cl | H | |
| $-CH_2$ 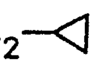 | 5-Br | H | |
| $-CH_2$ 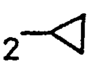 | 6-Br | H | |
| $-CH_2$ 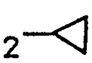 | 3-Br | H | |
| $-CH_2$ 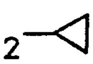 | 5-F | H | |
| $-CH_2$ 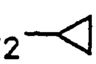 | 6-F | H | |
| $-CH_2C \equiv CH$ | 3-$OCH_3$ | H | |
| $-CH_2C \equiv CH$ | 4-$OCH_3$ | H | |
| $-CH_2C \equiv CH$ | 5-$OCH_3$ | H | |
| $-CH_2C \equiv CH$ | 6-$OCH_3$ | H | |
| $-CH_2C \equiv CH$ | 3-$CF_3$ | H | |
| $-CH_2C \equiv CH$ | 5-$CF_3$ | H | |

Table IV (continued)

| $R_1$ | $R_2$ | $R_3$ | m.p.(°C) |
|---|---|---|---|
| $-CH_2C{\equiv}CH$ | 3-Cl | H | |
| $-CH_2C{\equiv}CH$ | 5-Cl | H | |
| $-CH_2C{\equiv}CH$ | 6-Cl | H | |
| $-CH_2C{\equiv}CH$ | $5-CH_3$ | H | |
| $-CH_2C{\equiv}CH$ | 5-Br | H | |
| $-CH_2C{\equiv}CH$ | 5-F | H | |
| $-CH(CH_3)CO_2CH_3$ | $3-OCH_3$ | H | |
| $-CH(CH_3)CO_2CH_3$ | $5-OCH_3$ | H | |
| $-CH(CH_3)CO_2CH_3$ | $6-OCH_3$ | H | |
| $-CH(CH_3)CO_2CH_3$ | $5-CF_3$ | H | |
| $-CH(CH_3)CO_2CH_3$ | 5-Cl | H | |
| $-CH(CH_3)CO_2CH_3$ | $5-CH_3$ | H | |
| $-CH_2CO_2C_2H_5$ | $5-OCH_3$ | H | |
| $-CH_2CO_2C_2H_5$ | $5-CF_3$ | H | |
| $-CH_2CO_2C_2H_5$ | 5-Br | H | |
| $-CH(CH_3)CO_2C_2H_5$ | $5-OCH_3$ | H | |
| $-CH(CH_3)CO_2C_2H_5$ | $5-CF_3$ | H | |
| $-CH(CH_3)CO_2C_2H_5$ | 5-Cl | H | |
| $-CH(CH_3)CO_2C_2H_5$ | $5-CH_3$ | H | |
| $-CH(CH_3)CO_2C_2H_5$ | 5-F | H | |
| $-CH_2CN$ | $5-OCH_3$ | H | |
| $-CH_2CN$ | $6-OCH_3$ | H | |
| $-CH_2CN$ | $5-CF_3$ | H | |
| $-CH_2CN$ | 5-Cl | H | |
| $-CH(CH_3)CN$ | $5-OCH_3$ | H | |
| $-CH_2SO_2N(CH_3)_2$ | $5-CF_3$ | H | |
| $-CH(CH_3)SO_2N(CH_3)_2$ | $5-OCH_3$ | H | |
| $-CH_2CH_2N(CH_3)_2$ | 5-Cl | H | |
| $-CH_2CH_2N(CH_3)_2$ | $5-CF_3$ | H | |
| $-CH_2CH_2N(CH_3)_2$ | $5-OCH_3$ | H | |
| $-CH_2CH_2CN$ | $5-CF_3$ | H | |

Table IV (continued)

| $R_1$ | $R_2$ | $R_3$ | m.p.(°C) |
|---|---|---|---|
| $-CH_2CH_2CN$ | 5-$OCH_3$ | H | |
| $-CH_2CH_2CN$ | 5-Br | H | |
| $-CH_2CH_2CO_2CH_3$ | 3-$OCH_3$ | H | |
| $-CH_2CH_2CO_2CH_3$ | 5-$CF_3$ | H | |
| $-CH_2CH_2CO_2CH_3$ | 6-$OCH_3$ | H | |
| $-CH_2CH_2CO_2CH_3$ | 5-Cl | H | |
| $-CH_2CH_2CO_2C_2H_5$ | 3-Cl | H | |
| $-CH_2CH_2CO_2C_2H_5$ | 5-$CF_3$ | H | |
| $-CH_2CH_2CO_2C_2H_5$ | 5-$OCH_3$ | H | |
| $-CH_2CH_2SO_2N(CH_3)_2$ | 5-$CF_3$ | H | |
| $-CH_2CH_2SO_2N(CH_3)_2$ | 5-Cl | H | |
| $-CH_2C\equiv CH$ | H | $CH_3$ | |
| $-CH(CH_3)CO_2CH_3$ | H | $CH_3$ | |
| $-CH_2CO_2C_2H_5$ | H | $CH_3$ | |
| $-CH(CH_3)CO_2C_2H_5$ | H | $CH_3$ | |
| $-CH_2$ 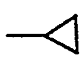 | H | $CH_3$ | |
| $-CH(CH_3)$ 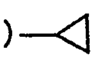 | H | $CH_3$ | |
| $-CH_2CN$ | H | $CH_3$ | |
| $-CH(CH_3)CN$ | H | $CH_3$ | |
| $-CH_2CH_2CN$ | H | $CH_3$ | |
| $-CH_2CH_2CO_2CH_3$ | H | $CH_3$ | |
| $-CH_2CH_2N(CH_3)_2$ | H | $CH_3$ | |
| $-CH_2CH_2CO_2C_2H_5$ | H | $CH_3$ | |
| $-CH_2SO_2N(CH_3)_2$ | H | $CH_3$ | |
| $-CH(CH_3)SO_2N(CH_3)_2$ | H | $CH_3$ | |
| $-CH_2CH_2SO_2N(CH_3)_2$ | H | $CH_3$ | |
| 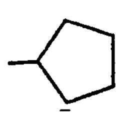 | H | $CH_3$ | |
| $-CH_2C\equiv CH$ | 5-Cl | $CH_3$ | |

Table IV (continued)

| $R_1$ | $R_2$ | $R_3$ | m.p.(°C) |
|---|---|---|---|
| $-CH(CH_3)CO_2CH_3$ | 5-F | $CH_3$ | |
| $-CH_2CO_2C_2H_5$ | 5-$CH_3$ | $CH_3$ | |
| $-CH(CH_3)CO_2C_2H_5$ | 5-$OCH_3$ | $CH_3$ | |
| 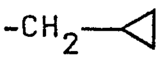 | 5-$OCH_3$ | $CH_3$ | |
| 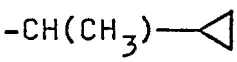 | 5-$CF_3$ | $CH_3$ | |
| $-CH_2CN$ | 5-$OCH_3$ | $CH_3$ | |
| $-CH_2CH_2CN$ | 5-$CH_3$ | $CH_3$ | |
| $-CH(CH_3)CN$ | 5-$CF_3$ | $CH_3$ | |
| $-CH_2CH_2CO_2CH_3$ | 5-$OCH_3$ | $CH_3$ | |
| $-CH_2CH_2CO_2C_2H_5$ | 5-$CF_3$ | $CH_3$ | |
| $-CH_2SO_2N(CH_3)_2$ | 5-$OCH_3$ | $CH_3$ | |
| 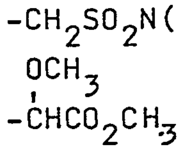 | H | $CH_3$ | |
| 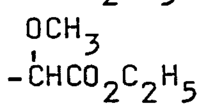 | H | $CH_3$ | |
| 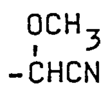 | H | $CH_3$ | |
| 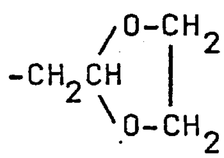 | H | H | |
| 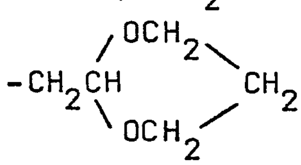 | H | H | |
| 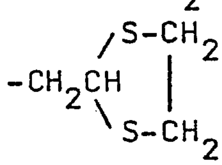 | H | H | |
| 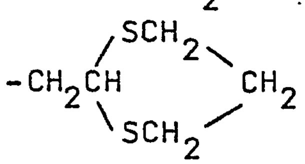 | H | H | |

Table IV (continued)

| $R_1$ | $R_2$ | $R_3$ | m.p.(°C) |
|---|---|---|---|
| 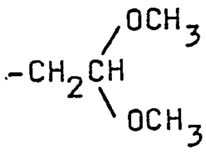 | H | H | |
| 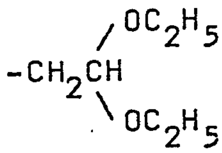 | H | H | |
| 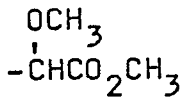 | 5-$CF_3$ | H | |
| 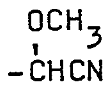 | 5-Cl | H | |
| 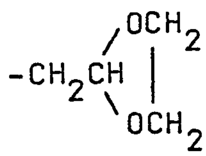 | 5-$CF_3$ | H | |
| 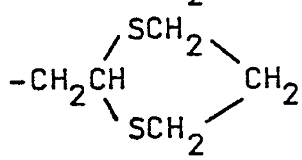 | 3-$OCH_3$ | H | |
| 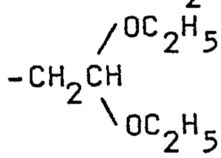 | 5-$CF_3$ | H | |
| 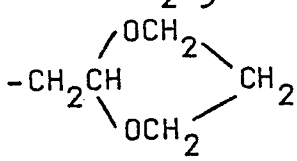 | H | $CH_3$ | |
| $CH_2CH_2CH_2CH_2CH_3$ | H | H | |
| 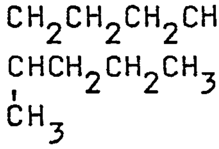 | H | H | |
| $CH(CH_2CH_3)_2$ | H | H | 193°(d) |
| $CH_2CH_2CH_2CH_2CH_3$ | H | $CH_3$ | |
| 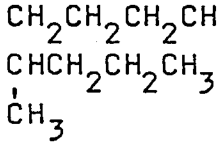 | H | $CH_3$ | |
| $CH(CH_2CH_3)_2$ | H | $CH_3$ | |
| $CH_2CH_2CH_2CH_2CH_3$ | 5-$OCH_3$ | H | |

Table IV (continued)

| $R_1$ | $R_2$ | $R_3$ | m.p.(°C) |
|---|---|---|---|
| $CH_2CH_2CH_2CH_2CH_3$ | 5-$CF_3$ | H | |
| $CH_2CH_2CH_2CH_2CH_3$ | 5-Cl | H | |
| $CH_2CH_2CH_2CH_2CH_3$ | 5-$CH_3$ | H | |
| $CH_2CH_2CH_2CH_2CH_3$ | 5-Br | H | |
| $CH_2CH_2CH_2CH_2CH_3$ | 5-F | H | |
| $CH_2CH_2CH_2CH_2CH_3$ | 6-$CH_3$ | H | |
| $CH_2CH_2CH_2CH_2CH_3$ | 6-$OCH_3$ | H | |
| $CH_2CH_2CH_2CH_2CH_3$ | 6-F | H | |
| $-CH_2CH_2-OH$ | H | H | |
| $CH_3$<br>$-CH-CH_2OH$ | H | H | |
| $CH_3$ $CH_3$<br>$-CH—CH-OH$ | H | H | |
| $CH_3$ $CH_3$<br>$-CH—C-OH$<br>$CH_3$ | H | H | |
| $CH_3$<br>$-CH_2-CH-OH$ | H | H | |
| $CH_3$<br>$-CH_2-C-OH$<br>$CH_3$ | H | H | |
| $-CH_2-CH_2-CH_2-OH$ | H | H | |
| $CH_3$<br>$-CH-CH_2-CH_2-OH$ | H | H | |
| $CH_3$<br>$-CH_2-CH-CH_2-OH$ | H | H | |
| $CH_3$<br>$-CH_2-CH_2-CH-OH$ | H | H | |
| $-CH_2-CH_2-OH$ | H | $CH_3$ | |
| $CH_3$<br>$-CH-CH_2-OH$ | H | $CH_3$ | |

Table IV (continued)

| $R_1$ | $R_2$ | $R_3$ | m.p.(°C) |
|---|---|---|---|
| $CH_3$ $CH_3$<br>-CH—CH-OH | H | $CH_3$ | |
| $CH_3$ $CH_3$<br>-CH—C-OH<br>$CH_3$ | H | $CH_3$ | |
| $CH_3$<br>$-CH_2-CH-OH$ | H | $CH_3$ | |
| $CH_3$<br>$-CH_2-C-OH$<br>$CH_3$ | H | $CH_3$ | |
| $-CH_2-CH_2-CH_2-OH$ | H | $CH_3$ | |
| $CH_3$<br>$-CH-CH_2-CH_2-OH$ | H | $CH_3$ | |
| $CH_3$<br>$-CH_2-CH-CH_2-OH$ | H | $CH_3$ | |
| $CH_3$<br>$-CH_2-CH_2-CH-OH$ | H | $CH_3$ | |
| $-CH_2-CH_2-OH$ | 3-F | H | |
| $-CH_2-CH_2-OH$ | 5-F | H | |
| $-CH_2-CH_2-OH$ | 5-Cl | H | |
| $-CH_2-CH_2-OH$ | 5-Br | H | |
| $-CH_2-CH_2-OH$ | $5-CH_3$ | H | |
| $-CH_2-CH_2-OH$ | $3-OCH_3$ | H | |
| $-CH_2-CH_2-OH$ | $5-OCH_3$ | H | |
| $-CH_2-CH_2-OH$ | $6-OCH_3$ | H | |
| $-CH_2-CH_2-OH$ | $5-CF_3$ | H | |
| $-CH_2-CH_2-CH_2-OH$ | 5-F | H | |
| $-CH_2-CH_2-CH_2-OH$ | 5-Cl | H | |
| $-CH_2-CH_2-CH_2-OH$ | 5-Br | H | |
| $-CH_2-CH_2-CH_2-OH$ | $5-CH_3$ | H | |
| $-CH_2-CH_2-CH_2-OH$ | $3-OCH_3$ | H | |
| $-CH_2-CH_2-CH_2-OH$ | $5-OCH_3$ | H | |

Table IV (continued)

| $R_1$ | $R_2$ | $R_3$ | m.p.(°C) |
|---|---|---|---|
| $-CH_2-CH_2-CH_2-OH$ | $6-OCH_3$ | H | |
| $-CH_2-CH_2-CH_2-OH$ | $5-CF_3$ | H | |
| $CH_3$<br>$-CH-CH_2-OH$ | $5-CF_3$ | H | |
| $CH_3$<br>$-CH-CH_2-OH$ | 5-Br | H | |
| $CH_3$<br>$-CH_2CH-OH$ | $5-CF_3$ | H | |
| $-CH_2-CH_2-OH$ | $5-CF_3$ | $CH_3$ | |
| $-CH_2-CH_2-OH$ | 5-Br | $CH_3$ | |
| $-CH_2-CH_2-OH$ | $3-OCH_3$ | $CH_3$ | |
| $-CH_2-CH_2-CH_2-OH$ | $5-CF_3$ | $CH_3$ | |
| $-CH_2-CH_2-OC(O)CH_3$ | H | H | |
| $-CH_2-CH_2-OC(O)C_2H_5$ | H | H | |
| $-CH_2-CH_2-OC(O)CH_2-CH_2-CH_3$ | H | H | |
| $-CH_2-CH_2-OC(O)-CH(CH_3)_2$ | H | H | |
| $-CH_2-CH_2-OC(O)C_6H_5$ | H | H | |
| $-CH_2-CH_2-OC(O)NHCH_3$ | H | H | |
| $-CH_2-CH_2-OC(O)NHC_2H_5$ | H | H | |
| $-CH_2-CH_2-OC(O)N(CH_3)_2$ | H | H | |
| $CH_3$<br>$-CH_2-CH_2-OC(O)N$<br>$OCH_3$ | H | H | |
| $-CH_2-CH_2-OC(O)NC_6H_5$ | H | H | |
| $-CH_2-CH_2-OSO_2CF_3$ | H | H | |
| $-CH_2-CH_2-OSO_2CH_3$ | H | H | |
| $-CH_2-CH_2-OSO_2C_2H_5$ | H | H | |
| $-CH_2-CH_2-OSO_2C_6H_5$ | H | H | |
| 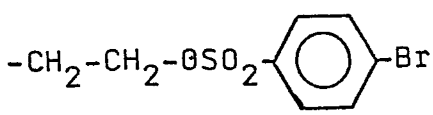 | H | H | |
| 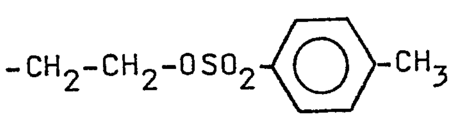 | H | H | |

Table IV (continued)

| $R_1$ | $R_2$ | $R_3$ | m.p.(°C) |
|---|---|---|---|
| $-CH_2-CH_2-OSO_2CH_3$ | 5-Br | H | |
| $-CH_2-CH_2-OSO_2CH_3$ | $5-CF_3$ | H | |
| $-CH_2-CH_2OSO_2CF_3$ | $5-CF_3$ | H | |
| $-CH_2-CH_2-OC(O)CH_3$ | $5-CF_3$ | H | |
| $-CH_2-CH_2-OSO_2CH_3$ | H | $CH_3$ | |
| $-CH_2-CH_2-OC(O)CH_3$ | H | $CH_3$ | |
| $-CH_2-CH_2-CH_2-OSO_2CH_3$ | $5-CF_3$ | H | |
| $-CH_2-CH_2-CH_2-OSO_2CH_3$ | H | $CH_3$ | |
| $-CH_2-CH_2-CH_2-OC(O)CH_3$ | H | $CH_3$ | |
| $-CH_2\overset{CH_3}{\overset{\vert}{C}}H-OSO_2CH_3$ | H | H | |
| $-\overset{CH_3}{\overset{\vert}{C}}H-\overset{CH_3}{\overset{\vert}{C}}H-OSO_2CH_3$ | H | H | |
| $-CH_2CH_2-\overset{CH_3}{\overset{\vert}{C}}H-OSO_2CH_3$ | H | H | |
| $-CH_2\overset{CH_3}{\overset{\vert}{C}}HCH_2-OSO_2CH_3$ | H | H | |
| $-CH_2C\equiv CH$ | H | H | |

Table V

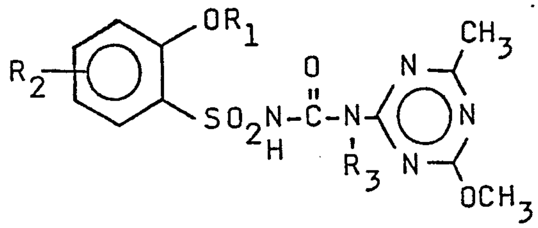

| $R_1$ | $R_2$ | $R_3$ | m.p.(°C) |
|---|---|---|---|
| $-CH_2CO_2CH_3$ | H | H | 173-175° |
| $-CH(CH_3)CO_2CH_3$ | H | H | 156-163° |
| $-CH_2CO_2C_2H_5$ | H | H | |
| $-CH(CH_3)CO_2C_2H_5$ | H | H | |
| $-CH_2-$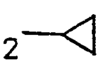 | H | H | 110° |
| $-CH(CH_3)-$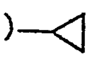 | H | H | |
| 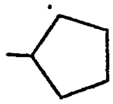 | H | H | 110°(d) |
| $-CH_2CN$ | H | H | |
| $-CH(CH_3)CN$ | H | H | |
| $-CH(OCH_3)CN$ | H | H | |
| $-CH(OCH_3)CO_2CH_3$ | H | H | |
| $-CH(OCH_3)CO_2C_2H_5$ | H | H | |
| $-CH_2SO_2N(CH_3)_2$ | H | H | |
| $-CH(CH_3)SO_2N(CH_3)_2$ | H | H | |
| $-CH_2CH_2N(CH_3)_2$ | H | H | |
| $-CH_2CH_2CN$ | H | H | |
| $-CH_2CH_2CO_2CH_3$ | H | H | |
| $-CH_2CH_2CO_2C_2H_5$ | H | H | |
| $-CH_2CH_2SO_2N(CH_3)_2$ | H | H | |
| $-CH_2-$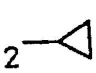 | 3-$OCH_3$ | H | |
| $-CH_2-$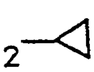 | 4-$OCH_3$ | H | |

Table V (continued)

| $R_1$ | $R_2$ | $R_3$ | m.p.(°C) |
|---|---|---|---|
| $-CH_2-$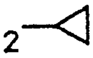 | 5-$OCH_3$ | H | |
| $-CH_2-$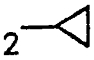 | 6-$OCH_3$ | H | |
| $-CH_2-$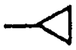 | 3-$CF_3$ | H | |
| $-CH_2-$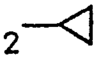 | 4-$CF_3$ | H | |
| $-CH_2-$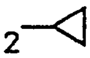 | 5-$CF_3$ | H | |
| $-CH_2-$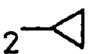 | 6-$CF_3$ | H | |
| $-CH_2-$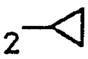 | 3-Cl | H | |
| $-CH_2-$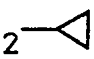 | 4-Cl | H | |
| $-CH_2-$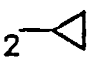 | 5-Cl | H | |
| $-CH_2-$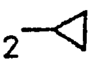 | 6-Cl | H | |
| $-CH_2-$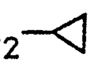 | 5-Br | H | |
| $-CH_2-$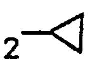 | 6-Br | H | |
| $-CH_2-$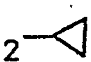 | 3-Br | H | |
| $-CH_2-$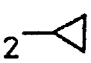 | 5-F | H | |
| $-CH_2-$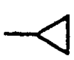 | 6-F | H | |
| $-CH_2C{\equiv}CH$ | 3-$OCH_3$ | H | |
| $-CH_2C{\equiv}CH$ | 4-$OCH_3$ | H | |
| $-CH_2C{\equiv}CH$ | 5-$OCH_3$ | H | |
| $-CH_2C{\equiv}CH$ | 6-$OCH_3$ | H | |
| $-CH_2C{\equiv}CH$ | 3-$CF_3$ | H | |
| $-CH_2C{\equiv}CH$ | 5-$CF_3$ | H | |

Table V (continued)

| $R_1$ | $R_2$ | $R_3$ | m.p.(°C) |
|---|---|---|---|
| $-CH_2C{\equiv}CH$ | 3-Cl | H | |
| $-CH_2C{\equiv}CH$ | 5-Cl | H | |
| $-CH_2C{\equiv}CH$ | 6-Cl | H | |
| $-CH_2C{\equiv}CH$ | $5-CH_3$ | H | |
| $-CH_2C{\equiv}CH$ | 5-Br | H | |
| $-CH_2C{\equiv}CH$ | 5-F | H | |
| $-CH(CH_3)CO_2CH_3$ | $3-OCH_3$ | H | |
| $-CH(CH_3)CO_2CH_3$ | $5-OCH_3$ | H | |
| $-CH(CH_3)CO_2CH_3$ | $6-OCH_3$ | H | |
| $-CH(CH_3)CO_2CH_3$ | $5-CF_3$ | H | |
| $-CH(CH_3)CO_2CH_3$ | 5-Cl | H | |
| $-CH(CH_3)CO_2CH_3$ | $5-CH_3$ | H | |
| $-CH_2CO_2C_2H_5$ | $5-OCH_3$ | H | |
| $-CH_2CO_2C_2H_5$ | $5-CF_3$ | H | |
| $-CH_2CO_2C_2H_5$ | 5-Br | H | |
| $-CH(CH_3)CO_2C_2H_5$ | $5-OCH_3$ | H | |
| $-CH(CH_3)CO_2C_2H_5$ | $5-CF_3$ | H | |
| $-CH(CH_3)CO_2C_2H_5$ | 5-Cl | H | |
| $-CH(CH_3)CO_2C_2H_5$ | $5-CH_3$ | H | |
| $-CH(CH_3)CO_2C_2H_5$ | 5-F | H | |
| $-CH_2CN$ | $5-OCH_3$ | H | |
| $-CH_2CN$ | $6-OCH_3$ | H | |
| $-CH_2CN$ | $5-CF_3$ | H | |
| $-CH_2CN$ | 5-Cl | H | |
| $-CH(CH_3)CN$ | $5-OCH_3$ | H | |
| $-CH_2SO_2N(CH_3)_2$ | $5-CF_3$ | H | |
| $-CH(CH_3)SO_2N(CH_3)_2$ | $5-OCH_3$ | H | |
| $-CH_2CH_2N(CH_3)_2$ | 5-Cl | H | |
| $-CH_2CH_2N(CH_3)_2$ | $5-CF_3$ | H | |
| $-CH_2CH_2N(CH_3)_2$ | $5-OCH_3$ | H | |
| $-CH_2CH_2CN$ | $5-CF_3$ | H | |

Table V (continued)

| $R_1$ | $R_2$ | $R_3$ | m.p.(°C) |
|---|---|---|---|
| $-CH_2CH_2CN$ | 5-$OCH_3$ | H | |
| $-CH_2CH_2CN$ | 5-Br | H | |
| $-CH_2CH_2CO_2CH_3$ | 3-$OCH_3$ | H | |
| $-CH_2CH_2CO_2CH_3$ | 5-$CF_3$ | H | |
| $-CH_2CH_2CO_2CH_3$ | 6-$OCH_3$ | H | |
| $-CH_2CH_2CO_2CH_3$ | 5-Cl | H | |
| $-CH_2CH_2CO_2C_2H_5$ | 3-Cl | H | |
| $-CH_2CH_2CO_2C_2H_5$ | 5-$CF_3$ | H | |
| $-CH_2CH_2CO_2C_2H_5$ | 5-$OCH_3$ | H | |
| $-CH_2CH_2SO_2N(CH_3)_2$ | 5-$CF_3$ | H | |
| $-CH_2CH_2SO_2N(CH_3)_2$ | 5-Cl | H | |
| $-CH_2C{\equiv}CH$ | H | $CH_3$ | |
| $-CH(CH_3)CO_2CH_3$ | H | $CH_3$ | |
| $-CH_2CO_2C_2H_5$ | H | $CH_3$ | |
| $-CH(CH_3)CO_2C_2H_5$ | H | $CH_3$ | |
| $-CH_2$ 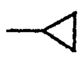 | H | $CH_3$ | |
| $-CH(CH_3)$ 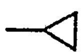 | H | $CH_3$ | |
| $-CH_2CN$ | H | $CH_3$ | |
| $-CH(CH_3)CN$ | H | $CH_3$ | |
| $-CH_2CH_2CN$ | H | $CH_3$ | |
| $-CH_2CH_2CO_2CH_3$ | H | $CH_3$ | |
| $-CH_2CH_2N(CH_3)_2$ | H | $CH_3$ | |
| $-CH_2CH_2CO_2C_2H_5$ | H | $CH_3$ | |
| $-CH_2SO_2N(CH_3)_2$ | H | $CH_3$ | |
| $-CH(CH_3)SO_2N(CH_3)_2$ | H | $CH_3$ | |
| $-CH_2CH_2SO_2N(CH_3)_2$ | H | $CH_3$ | |
| 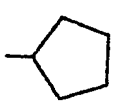 | H | $CH_3$ | |
| $-CH_2C{\equiv}CH$ | 5-Cl | $CH_3$ | |

Table V (continued)

| $R_1$ | $R_2$ | $R_3$ | m.p.(°C) |
|---|---|---|---|
| $-CH(CH_3)CO_2CH_3$ | 5-F | $CH_3$ | |
| $-CH_2CO_2C_2H_5$ | 5-$CH_3$ | $CH_3$ | |
| $-CH(CH_3)CO_2C_2H_5$ | 5-$OCH_3$ | $CH_3$ | |
| 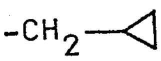 | 5-$OCH_3$ | $CH_3$ | |
| 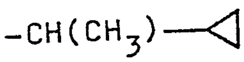 | 5-$CF_3$ | $CH_3$ | |
| $-CH_2CN$ | 5-$OCH_3$ | $CH_3$ | |
| $-CH_2CH_2CN$ | 5-$CH_3$ | $CH_3$ | |
| $-CH(CH_3)CN$ | 5-$CF_3$ | $CH_3$ | |
| $-CH_2CH_2CO_2CH_3$ | 5-$OCH_3$ | $CH_3$ | |
| $-CH_2CH_2CO_2C_2H_5$ | 5-$CF_3$ | $CH_3$ | |
| $-CH_2SO_2N(CH_3)_2$ | 5-$OCH_3$ | $CH_3$ | |
| 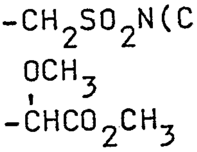 | H | $CH_3$ | |
| 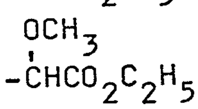 | H | $CH_3$ | |
| 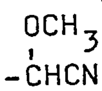 | H | $CH_3$ | |
| 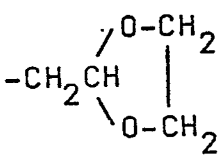 | H | H | |
| 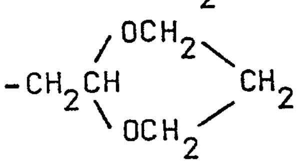 | H | H | |
| 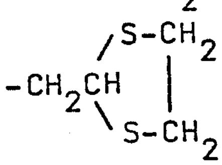 | H | H | |
| 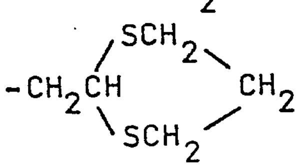 | H | H | |

Table V (continued)

| $R_1$ | $R_2$ | $R_3$ | m.p.(°C) |
|---|---|---|---|
| 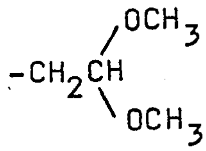 | H | H | |
| 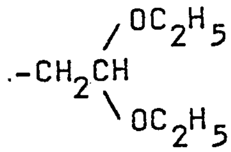 | H | H | |
| $OCH_3$<br>$-CHCO_2CH_3$ | 5-$CF_3$ | H | |
| $OCH_3$<br>$-CHCN$ | 5-Cl | H | |
| 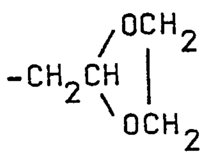 | 5-$CF_3$ | H | |
| 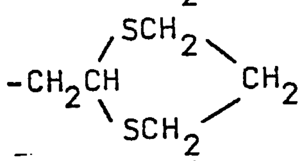 | 3-$OCH_3$ | H | |
| 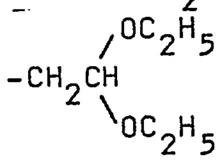 | 5-$CF_3$ | H | |
| 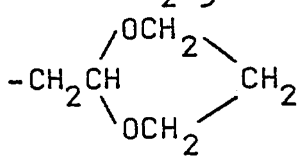 | H | $CH_3$ | |
| $CH_2CH_2CH_2CH_2CH_3$ | H | H | >250° |
| $CHCH_2CH_2CH_3$<br>$CH_3$ | H | H | 149-151° |
| $CH(CH_2CH_3)_2$ | H | H | 158-160° |
| $CH_2CH_2CH_2CH_2CH_3$ | H | $CH_3$ | |
| $CHCH_2CH_2CH_3$<br>$CH_3$ | H | $CH_3$ | |
| $CH(CH_2CH_3)_2$ | H | $CH_3$ | |
| $CH_2CH_2CH_2CH_2CH_3$ | 5-$OCH_3$ | H | |

Table V (continued)

| $R_1$ | $R_2$ | $R_3$ | m.p.(°C) |
|---|---|---|---|
| $CH_2CH_2CH_2CH_2CH_3$ | 5-$CF_3$ | H | |
| $CH_2CH_2CH_2CH_2CH_3$ | 5-Cl | H | |
| $CH_2CH_2CH_2CH_2CH_3$ | 5-$CH_3$ | H | |
| $CH_2CH_2CH_2CH_2CH_3$ | 5-Br | H | |
| $CH_2CH_2CH_2CH_2CH_3$ | 5-F | H | |
| $CH_2CH_2CH_2CH_2CH_3$ | 6-$CH_3$ | H | |
| $CH_2CH_2CH_2CH_2CH_3$ | 6-$OCH_3$ | H | |
| $CH_2CH_2CH_2CH_2CH_3$ | 6-F | H | |
| $-CH_2CH_2-OH$ | H | H | 171-174° |
| $CH_3$<br>$-CH-CH_2OH$ | H | H | |
| $CH_3$ $CH_3$<br>$-CH—CH-OH$ | H | H | |
| $CH_3$ $CH_3$<br>$-CH—C-OH$<br>$CH_3$ | H | H | |
| $CH_3$<br>$-CH_2-CH-OH$ | H | H | |
| $CH_3$<br>$-CH_2-C-OH$<br>$CH_3$ | H | H | |
| $-CH_2-CH_2-CH_2-OH$ | H | H | |
| $CH_3$<br>$-CH-CH_2-CH_2-OH$ | H | H | |
| $CH_3$<br>$-CH_2-CH-CH_2-OH$ | H | H | |
| $CH_3$<br>$-CH_2-CH_2-CH-OH$ | H | H | |
| $-CH_2-CH_2-OH$ | H | $CH_3$ | |
| $CH_3$<br>$-CH-CH_2-OH$ | H | $CH_3$ | |

Table V (continued)

| $R_1$ | $R_2$ | $R_3$ | m.p.(°C) |
|---|---|---|---|
| $-CH(CH_3)-CH(CH_3)-OH$ | H | $CH_3$ | |
| $-CH(CH_3)-C(CH_3)_2-OH$ | H | $CH_3$ | |
| $-CH_2-CH(CH_3)-OH$ | H | $CH_3$ | |
| $-CH_2-C(CH_3)_2-OH$ | H | $CH_3$ | |
| $-CH_2-CH_2-CH_2-OH$ | H | $CH_3$ | |
| $-CH(CH_3)-CH_2-CH_2-OH$ | H | $CH_3$ | |
| $-CH_2-CH(CH_3)-CH_2-OH$ | H | $CH_3$ | |
| $-CH_2-CH_2-CH(CH_3)-OH$ | H | $CH_3$ | |
| $-CH_2-CH_2-OH$ | 3-F | H | |
| $-CH_2-CH_2-OH$ | 5-F | H | |
| $-CH_2-CH_2-OH$ | 5-Cl | H | |
| $-CH_2-CH_2-OH$ | 5-Br | H | |
| $-CH_2-CH_2-OH$ | 5-$CH_3$ | H | |
| $-CH_2-CH_2-OH$ | 3-$OCH_3$ | H | |
| $-CH_2-CH_2-OH$ | 5-$OCH_3$ | H | |
| $-CH_2-CH_2-OH$ | 6-$OCH_3$ | H | |
| $-CH_2-CH_2-OH$ | 5-$CF_3$ | H | |
| $-CH_2-CH_2-CH_2-OH$ | 5-F | H | |
| $-CH_2-CH_2-CH_2-OH$ | 5-Cl | H | |
| $-CH_2-CH_2-CH_2-OH$ | 5-Br | H | |
| $-CH_2-CH_2-CH_2-OH$ | 5-$CH_3$ | H | |
| $-CH_2-CH_2-CH_2-OH$ | 3-$OCH_3$ | H | |
| $-CH_2-CH_2-CH_2-OH$ | 5-$OCH_3$ | H | |

Table V (continued)

| $R_1$ | $R_2$ | $R_3$ | m.p.(°C) |
|---|---|---|---|
| $-CH_2-CH_2-CH_2-OH$ | $6-OCH_3$ | H | |
| $-CH_2-CH_2-CH_2-OH$ | $5-CF_3$ | H | |
| $CH_3$<br>$-CH-CH_2-OH$ | $5-CF_3$ | H | |
| $CH_3$<br>$-CH-CH_2-OH$ | 5-Br | H | |
| $CH_3$<br>$-CH_2CH-OH$ | $5-CF_3$ | H | |
| $-CH_2-CH_2-OH$ | $5-CF_3$ | $CH_3$ | |
| $-CH_2-CH_2-OH$ | 5-Br | $CH_3$ | |
| $-CH_2-CH_2-OH$ | $3-OCH_3$ | $CH_3$ | |
| $-CH_2-CH_2-CH_2-OH$ | $5-CF_3$ | $CH_3$ | |
| $-CH_2-CH_2-OC(O)CH_3$ | H | H | |
| $-CH_2-CH_2-OC(O)C_2H_5$ | H | H | |
| $-CH_2-CH_2-OC(O)CH_2-CH_2-CH_3$ | H | H | |
| $-CH_2-CH_2-OC(O)-CH(CH_3)_2$ | H | H | |
| $-CH_2-CH_2-OC(O)C_6H_5$ | H | H | |
| $-CH_2-CH_2-OC(O)NHCH_3$ | H | H | |
| $-CH_2-CH_2-OC(O)NHC_2H_5$ | H | H | |
| $-CH_2-CH_2-OC(O)N(CH_3)_2$ | H | H | |
| $CH_3$<br>$-CH_2-CH_2-OC(O)N$<br>$OCH_3$ | H | H | |
| $-CH_2-CH_2-OC(O)NC_6H_5$ | H | H | |
| $-CH_2-CH_2-OSO_2CF_3$ | H | H | |
| $-CH_2-CH_2-OSO_2CH_3$ | H | H | |
| $-CH_2-CH_2-OSO_2C_2H_5$ | H | H | |
| $-CH_2-CH_2-OSO_2C_6H_5$ | H | H | |
| $-CH_2-CH_2-OSO_2$ 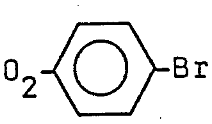 | H | H | |
| $-CH_2-CH_2-OSO_2$ 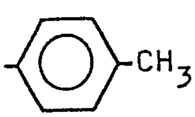 | H | H | |

Table V (continued)

| $R_1$ | $R_2$ | $R_3$ | m.p.(°C) |
|---|---|---|---|
| $-CH_2-CH_2-OSO_2CH_3$ | 5-Br | H | |
| $-CH_2-CH_2-OSO_2CH_3$ | 5-$CF_3$ | H | |
| $-CH_2-CH_2OSO_2CF_3$ | 5-$CF_3$ | H | |
| $-CH_2-CH_2-OC(O)CH_3$ | 5-$CF_3$ | H | |
| $-CH_2-CH_2-OSO_2CH_3$ | H | $CH_3$ | |
| $-CH_2-CH_2-OC(O)CH_3$ | H | $CH_3$ | |
| $-CH_2-CH_2-CH_2-OSO_2CH_3$ | 5-$CF_3$ | H | |
| $-CH_2-CH_2-CH_2-OSO_2CH_3$ | H | $CH_3$ | |
| $-CH_2-CH_2-CH_2-OC(O)CH_3$ | H | $CH_3$ | |
| $CH_3$<br>$-CH_2CH-OSO_2CH_3$ | H | H | |
| $CH_3$ $CH_3$<br>$-CH—CH-OSO_2CH_3$ | H | H | |
| $CH_3$<br>$-CH_2CH_2-CH-OSO_2CH_3$ | H | H | |
| $CH_3$<br>$-CH_2CHCH_2-OSO_2CH_3$ | H | H | |
| $-CH_2C{\equiv}CH$ | H | H | 172-174 |

Table VI

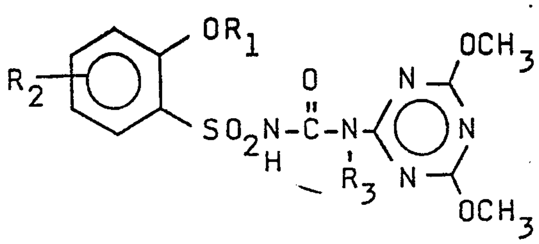

| $R_1$ | $R_2$ | $R_3$ | m.p.(°C) |
|---|---|---|---|
| $-CH_2CO_2CH_3$ | H | H | 171-173° |
| $-CH(CH_3)CO_2CH_3$ | H | H | 101-107°(d) |
| $-CH_2CO_2C_2H_5$ | H | H | |
| $-CH(CH_3)CO_2C_2H_5$ | H | H | |
| $-CH_2$ 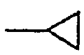 | H | H | 74-81° |
| $-CH(CH_3)$ 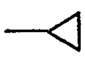 | H | H | |
| 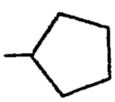 | H | H | 158-166° |
| $-CH_2CN$ | H | H | |
| $-CH(CH_3)CN$ | H | H | |
| $-CH(OCH_3)CN$ | H | H | |
| $-CH(OCH_3)CO_2CH_3$ | H | H | |
| $-CH(OCH_3)CO_2C_2H_5$ | H | H | |
| $-CH_2SO_2N(CH_3)_2$ | H | H | |
| $-CH(CH_3)SO_2N(CH_3)_2$ | H | H | |
| $-CH_2CH_2N(CH_3)_2$ | H | H | |
| $-CH_2CH_2CN$ | H | H | |
| $-CH_2CH_2CO_2CH_3$ | H | H | |
| $-CH_2CH_2CO_2C_2H_5$ | H | H | |
| $-CH_2CH_2SO_2N(CH_3)_2$ | H | H | |
| $-CH_2$ 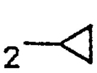 | 3-$OCH_3$ | H | |
| $-CH_2$ 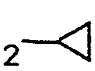 | 4-$OCH_3$ | H | |

Table VI (continued)

| $R_1$ | $R_2$ | $R_3$ | m.p.(°C) |
|---|---|---|---|
| $-CH_2-$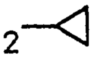 | $5-OCH_3$ | H | |
| $-CH_2-$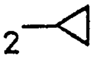 | $6-OCH_3$ | H | |
| $-CH_2-$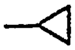 | $3-CF_3$ | H | |
| $-CH_2-$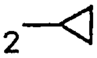 | $4-CF_3$ | H | |
| $-CH_2-$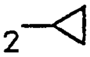 | $5-CF_3$ | H | |
| $-CH_2-$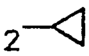 | $6-CF_3$ | H | |
| $-CH_2-$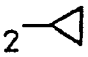 | 3-Cl | H | |
| $-CH_2-$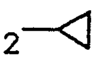 | 4-Cl | H | |
| $-CH_2-$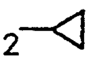 | 5-Cl | H | |
| $-CH_2-$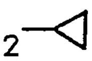 | 6-Cl | H | |
| $-CH_2-$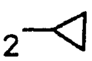 | 5-Br | H | |
| $-CH_2-$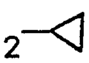 | 6-Br | H | |
| $-CH_2-$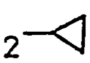 | 3-Br | H | |
| $-CH_2-$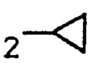 | 5-F | H | |
| $-CH_2-$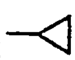 | 6-F | H | |
| $-CH_2C{\equiv}CH$ | $3-OCH_3$ | H | |
| $-CH_2C{\equiv}CH$ | $4-OCH_3$ | H | |
| $-CH_2C{\equiv}CH$ | $5-OCH_3$ | H | |
| $-CH_2C{\equiv}CH$ | $6-OCH_3$ | H | |
| $-CH_2C{\equiv}CH$ | $3-CF_3$ | H | |
| $-CH_2C{\equiv}CH$ | $5-CF_3$ | H | |

Table VI (continued)

| $R_1$ | $R_2$ | $R_3$ | m.p.(°C) |
|---|---|---|---|
| $-CH_2C{\equiv}CH$ | 3-Cl | H | |
| $-CH_2C{\equiv}CH$ | 5-Cl | H | |
| $-CH_2C{\equiv}CH$ | 6-Cl | H | |
| $-CH_2C{\equiv}CH$ | $5-CH_3$ | H | |
| $-CH_2C{\equiv}CH$ | 5-Br | H | |
| $-CH_2C{\equiv}CH$ | 5-F | H | |
| $-CH(CH_3)CO_2CH_3$ | $3-OCH_3$ | H | |
| $-CH(CH_3)CO_2CH_3$ | $5-OCH_3$ | H | |
| $-CH(CH_3)CO_2CH_3$ | $6-OCH_3$ | H | |
| $-CH(CH_3)CO_2CH_3$ | $5-CF_3$ | H | |
| $-CH(CH_3)CO_2CH_3$ | 5-Cl | H | |
| $-CH(CH_3)CO_2CH_3$ | $5-CH_3$ | H | |
| $-CH_2CO_2C_2H_5$ | $5-OCH_3$ | H | |
| $-CH_2CO_2C_2H_5$ | $5-CF_3$ | H | |
| $-CH_2CO_2C_2H_5$ | 5-Br | H | |
| $-CH(CH_3)CO_2C_2H_5$ | $5-OCH_3$ | H | |
| $-CH(CH_3)CO_2C_2H_5$ | $5-CF_3$ | H | |
| $-CH(CH_3)CO_2C_2H_5$ | 5-Cl | H | |
| $-CH(CH_3)CO_2C_2H_5$ | $5-CH_3$ | H | |
| $-CH(CH_3)CO_2C_2H_5$ | 5-F | H | |
| $-CH_2CN$ | $5-OCH_3$ | H | |
| $-CH_2CN$ | $6-OCH_3$ | H | |
| $-CH_2CN$ | $5-CF_3$ | H | |
| $-CH_2CN$ | 5-Cl | H | |
| $-CH(CH_3)CN$ | $5-OCH_3$ | H | |
| $-CH_2SO_2N(CH_3)_2$ | $5-CF_3$ | H | |
| $-CH(CH_3)SO_2N(CH_3)_2$ | $5-OCH_3$ | H | |
| $-CH_2CH_2N(CH_3)_2$ | 5-Cl | H | |
| $-CH_2CH_2N(CH_3)_2$ | $5-CF_3$ | H | |
| $-CH_2CH_2N(CH_3)_2$ | $5-OCH_3$ | H | |
| $-CH_2CH_2CN$ | $5-CF_3$ | H | |

Table VI (continued)

| $R_1$ | $R_2$ | $R_3$ | m.p.(°C) |
|---|---|---|---|
| $-CH_2CH_2CN$ | 5-$OCH_3$ | H | |
| $-CH_2CH_2CN$ | 5-Br | H | |
| $-CH_2CH_2CO_2CH_3$ | 3-$OCH_3$ | H | |
| $-CH_2CH_2CO_2CH_3$ | 5-$CF_3$ | H | |
| $-CH_2CH_2CO_2CH_3$ | 6-$OCH_3$ | H | |
| $-CH_2CH_2CO_2CH_3$ | 5-Cl | H | |
| $-CH_2CH_2CO_2C_2H_5$ | 3-Cl | H | |
| $-CH_2CH_2CO_2C_2H_5$ | 5-$CF_3$ | H | |
| $-CH_2CH_2CO_2C_2H_5$ | 5-$OCH_3$ | H | |
| $-CH_2CH_2SO_2N(CH_3)_2$ | 5-$CF_3$ | H | |
| $-CH_2CH_2SO_2N(CH_3)_2$ | 5-Cl | H | |
| $-CH_2C{\equiv}CH$ | H | $CH_3$ | |
| $-CH(CH_3)CO_2CH_3$ | H | $CH_3$ | |
| $-CH_2CO_2C_2H_5$ | H | $CH_3$ | |
| $-CH(CH_3)CO_2C_2H_5$ | H | $CH_3$ | |
| $-CH_2$ 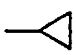 | H | $CH_3$ | |
| $-CH(CH_3)$ 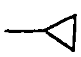 | H | $CH_3$ | |
| $-CH_2CN$ | H | $CH_3$ | |
| $-CH(CH_3)CN$ | H | $CH_3$ | |
| $-CH_2CH_2CN$ | H | $CH_3$ | |
| $-CH_2CH_2CO_2CH_3$ | H | $CH_3$ | |
| $-CH_2CH_2N(CH_3)_2$ | H | $CH_3$ | |
| $-CH_2CH_2CO_2C_2H_5$ | H | $CH_3$ | |
| $-CH_2SO_2N(CH_3)_2$ | H | $CH_3$ | |
| $-CH(CH_3)SO_2N(CH_3)_2$ | H | $CH_3$ | |
| $-CH_2CH_2SO_2N(CH_3)_2$ | H | $CH_3$ | |
| 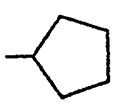 | H | $CH_3$ | |
| $-CH_2C{\equiv}CH$ | 5-Cl | $CH_3$ | |

Table VI (continued)

| $R_1$ | $R_2$ | $R_3$ | m.p.(°C) |
|---|---|---|---|
| $-CH(CH_3)CO_2CH_3$ | 5-F | $CH_3$ | |
| $-CH_2CO_2C_2H_5$ | $5-CH_3$ | $CH_3$ | |
| $-CH(CH_3)CO_2C_2H_5$ | $5-OCH_3$ | $CH_3$ | |
| 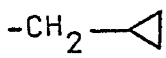 | $5-OCH_3$ | $CH_3$ | |
| 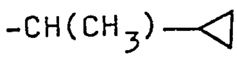 | $5-CF_3$ | $CH_3$ | |
| $-CH_2CN$ | $5-OCH_3$ | $CH_3$ | |
| $-CH_2CH_2CN$ | $5-CH_3$ | $CH_3$ | |
| $-CH(CH_3)CN$ | $5-CF_3$ | $CH_3$ | |
| $-CH_2CH_2CO_2CH_3$ | $5-OCH_3$ | $CH_3$ | |
| $-CH_2CH_2CO_2C_2H_5$ | $5-CF_3$ | $CH_3$ | |
| $-CH_2SO_2N(CH_3)_2$ | $5-OCH_3$ | $CH_3$ | |
| 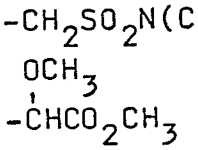 | H | $CH_3$ | |
| 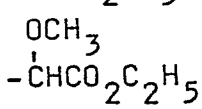 | H | $CH_3$ | |
| 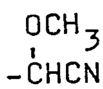 | H | $CH_3$ | |
| 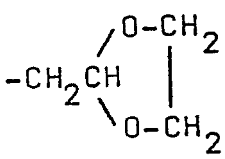 | H | H | 177-184° |
| 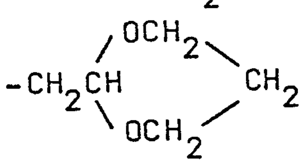 | H | H | |
| 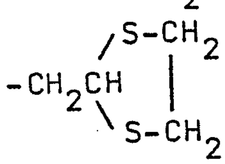 | H | H | |
| 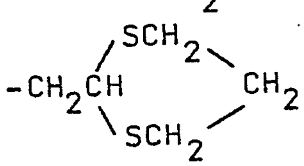 | H | H | |

Table VI (continued)

| $R_1$ | $R_2$ | $R_3$ | m.p.(°C) |
|---|---|---|---|
| 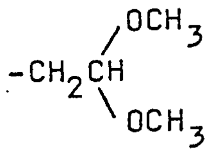 | H | H | |
| 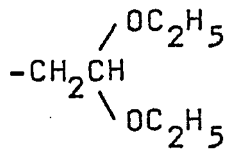 | H | H | |
| 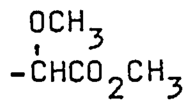 | 5-$CF_3$ | H | |
| 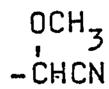 | 5-Cl | H | |
| 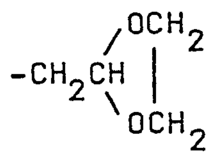 | 5-$CF_3$ | H | |
| 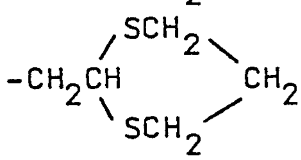 | 3-$OCH_3$ | H | |
| 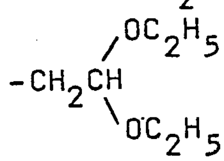 | 5-$CF_3$ | H | |
| 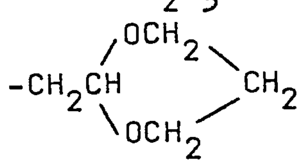 | H | $CH_3$ | |
| $CH_2CH_2CH_2CH_2CH_3$ | H | H | 108-114° |
| 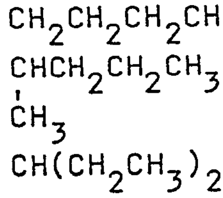 | H | H | 172-174° |
| $CH(CH_2CH_3)_2$ | H | H | 162-163° |
| $CH_2CH_2CH_2CH_2CH_3$ | H | $CH_3$ | |
| 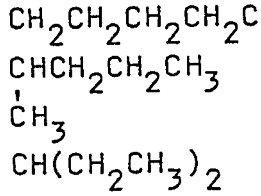 | H | $CH_3$ | |
| $CH(CH_2CH_3)_2$ | H | $CH_3$ | |
| $CH_2CH_2CH_2CH_2CH_3$ | 5-$OCH_3$ | H | |

Table VI (continued)

| $R_1$ | $R_2$ | $R_3$ | m.p.(°C) |
|---|---|---|---|
| $CH_2CH_2CH_2CH_2CH_3$ | 5-$CF_3$ | H | |
| $CH_2CH_2CH_2CH_2CH_3$ | 5-Cl | H | |
| $CH_2CH_2CH_2CH_2CH_3$ | 5-$CH_3$ | H | |
| $CH_2CH_2CH_2CH_2CH_3$ | 5-Br | H | |
| $CH_2CH_2CH_2CH_2CH_3$ | 5-F | H | |
| $CH_2CH_2CH_2CH_2CH_3$ | 6-$CH_3$ | H | |
| $CH_2CH_2CH_2CH_2CH_3$ | 6-$OCH_3$ | H | |
| $CH_2CH_2CH_2CH_2CH_3$ | 6-F | H | |
| $-CH_2CH_2-OH$ | H | H | 159-165° |
| $CH_3$<br>$-CH-CH_2OH$ | H | H | |
| $CH_3$ $CH_3$<br>$-CH—CH-OH$ | H | H | |
| $CH_3$ $CH_3$<br>$-CH—C-OH$<br>$CH_3$ | H | H | |
| $CH_3$<br>$-CH_2-CH-OH$ | H | H | |
| $CH_3$<br>$-CH_2-C-OH$<br>$CH_3$ | H | H | |
| $-CH_2-CH_2-CH_2-OH$ | H | H | |
| $CH_3$<br>$-CH-CH_2-CH_2-OH$ | H | H | |
| $CH_3$<br>$-CH_2-CH-CH_2-OH$ | H | H | |
| $CH_3$<br>$-CH_2-CH_2-CH-OH$ | H | H | |
| $-CH_2-CH_2-OH$ | H | $CH_3$ | |
| $CH_3$<br>$-CH-CH_2-OH$ | H | $CH_3$ | |

Table VI (continued)

| $R_1$ | $R_2$ | $R_3$ | m.p.(°C) |
|---|---|---|---|
| $CH_3$ $CH_3$<br>$-CH—CH-OH$ | H | $CH_3$ | |
| $CH_3$ $CH_3$<br>$-CH—C-OH$<br>$CH_3$ | H | $CH_3$ | |
| $CH_3$<br>$-CH_2-CH-OH$ | H | $CH_3$ | |
| $CH_3$<br>$-CH_2-C-OH$<br>$CH_3$ | H | $CH_3$ | |
| $-CH_2-CH_2-CH_2-OH$ | H | $CH_3$ | |
| $CH_3$<br>$-CH-CH_2-CH_2-OH$ | H | $CH_3$ | |
| $CH_3$<br>$-CH_2-CH-CH_2-OH$ | H | $CH_3$ | |
| $CH_3$<br>$-CH_2-CH_2-CH-OH$ | H | $CH_3$ | |
| $-CH_2-CH_2-OH$ | 3-F | H | |
| $-CH_2-CH_2-OH$ | 5-F | H | |
| $-CH_2-CH_2-OH$ | 5-Cl | H | |
| $-CH_2-CH_2-OH$ | 5-Br | H | |
| $-CH_2-CH_2-OH$ | $5-CH_3$ | H | |
| $-CH_2-CH_2-OH$ | $3-OCH_3$ | H | |
| $-CH_2-CH_2-OH$ | $5-OCH_3$ | H | |
| $-CH_2-CH_2-OH$ | $6-OCH_3$ | H | |
| $-CH_2-CH_2-OH$ | $5-CF_3$ | H | |
| $-CH_2-CH_2-CH_2-OH$ | 5-F | H | |
| $-CH_2-CH_2-CH_2-OH$ | 5-Cl | H | |
| $-CH_2-CH_2-CH_2-OH$ | 5-Br | H | |
| $-CH_2-CH_2-CH_2-OH$ | $5-CH_3$ | H | |
| $-CH_2-CH_2-CH_2-OH$ | $3-OCH_3$ | H | |
| $-CH_2-CH_2-CH_2-OH$ | $5-OCH_3$ | H | |

Table VI (continued)

| $R_1$ | $R_2$ | $R_3$ | m.p.(°C) |
|---|---|---|---|
| $-CH_2-CH_2-CH_2-OH$ | $6-OCH_3$ | H | |
| $-CH_2-CH_2-CH_2-OH$ | $5-CF_3$ | H | |
| $CH_3$<br>$-CH-CH_2-OH$ | $5-CF_3$ | H | |
| $CH_3$<br>$-CH-CH_2-OH$ | 5-Br | H | |
| $CH_3$<br>$-CH_2CH-OH$ | $5-CF_3$ | H | |
| $-CH_2-CH_2-OH$ | $5-CF_3$ | $CH_3$ | |
| $-CH_2-CH_2-OH$ | 5-Br | $CH_3$ | |
| $-CH_2-CH_2-OH$ | $3-OCH_3$ | $CH_3$ | |
| $-CH_2-CH_2-CH_2-OH$ | $5-CF_3$ | $CH_3$ | |
| $-CH_2-CH_2-OC(O)CH_3$ | H | H | |
| $-CH_2-CH_2-OC(O)C_2H_5$ | H | H | |
| $-CH_2-CH_2-OC(O)CH_2-CH_2-CH_3$ | H | H | |
| $-CH_2-CH_2-OC(O)-CH(CH_3)_2$ | H | H | |
| $-CH_2-CH_2-OC(O)C_6H_5$ | H | H | |
| $-CH_2-CH_2-OC(O)NHCH_3$ | H | H | |
| $-CH_2-CH_2-OC(O)NHC_2H_5$ | H | H | |
| $-CH_2-CH_2-OC(O)N(CH_3)_2$ | H | H | |
| $CH_3$<br>$-CH_2-CH_2-OC(O)N$<br>$OCH_3$ | H | H | |
| $-CH_2-CH_2-OC(O)NC_6H_5$ | H | H | |
| $-CH_2-CH_2-OSO_2CF_3$ | H | H | |
| $-CH_2-CH_2-OSO_2CH_3$ | H | H | |
| $-CH_2-CH_2-OSO_2C_2H_5$ | H | H | |
| $-CH_2-CH_2-OSO_2C_6H_5$ | H | H | |
| $-CH_2-CH_2-OSO_2-$ 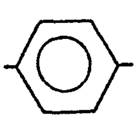 -Br | H | H | |
| $-CH_2-CH_2-OSO_2-$ 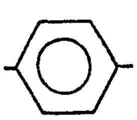 $-CH_3$ | H | H | |

Table VI (continued)

| $R_1$ | $R_2$ | $R_3$ | m.p.(°C) |
|---|---|---|---|
| $-CH_2-CH_2-OSO_2CH_3$ | 5-Br | H | |
| $-CH_2-CH_2-OSO_2CH_3$ | 5-$CF_3$ | H | |
| $-CH_2-CH_2OSO_2CF_3$ | 5-$CF_3$ | H | |
| $-CH_2-CH_2-OC(O)CH_3$ | 5-$CF_3$ | H | |
| $-CH_2-CH_2-OSO_2CH_3$ | H | $CH_3$ | |
| $-CH_2-CH_2-OC(O)CH_3$ | H | $CH_3$ | |
| $-CH_2-CH_2-CH_2-OSO_2CH_3$ | 5-$CF_3$ | H | |
| $-CH_2-CH_2-CH_2-OSO_2CH_3$ | H | $CH_3$ | |
| $-CH_2-CH_2-CH_2-OC(O)CH_3$ | H | $CH_3$ | |
| $CH_3$<br>$-CH_2CH-OSO_2CH_3$ | H | H | |
| $CH_3$ $CH_3$<br>$-CH—CH-OSO_2CH_3$ | H | H | |
| $CH_3$<br>$-CH_2CH_2-CH-OSO_2CH_3$ | H | H | |
| $CH_3$<br>$-CH_2CHCH_2-OSO_2CH_3$ | H | H | |
| $-CH_2C≡CH$ | H | H | 172-177 |

Table VII

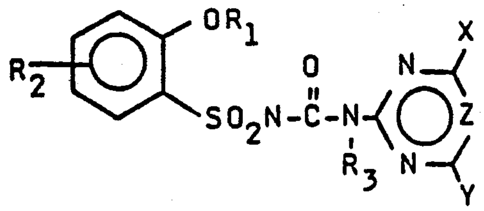

| $R_1$ | $R_2$ | $R_3$ | X | Y | Z | m.p.(°C) |
|---|---|---|---|---|---|---|
| $(CH_2)_4CH_3$ | H | H | $CH_3$ | $C_2H_5$ | CH | |
| $-CH_2$— 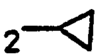 | H | H | $CH_3$ | $C_2H_5$ | N | |
| $(CH_2)_4CH_3$ | H | H | $CH_3$ | $OC_2H_5$ | CH | |
| $(CH_2)_4CH_3$ | H | H | $CH_3$ | $OC_2H_5$ | N | |
| $(CH_2)_4CH_3$ | H | H | $CH_3$ | $CH_2OCH_3$ | CH | |
| $CH_2$— 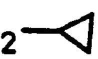 | H | H | $CH_3$ | $CH_2OCH_3$ | N | |
| $-CH_2$— 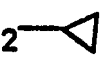 | H | H | $CH_3$ | $CH(OCH_3)_2$ | CH | |
| $(CH_2)_4CH_3$ | H | H | $CH_3$ | $CH(OCH_3)_2$ | N | |
| $-CH_2$— 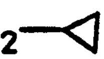 | H | H | $CH_3$ | 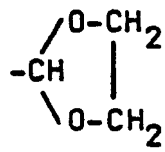 | CH | |
| $(CH_2)_4CH_3$ | H | H | $CH_3$ | 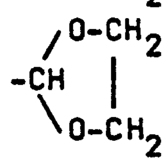 | N | |
| $(CH_2)_4CH_3$ | H | H | $CH_3$ | $NH_2$ | CH | |
| $(CH_2)_4CH_3$ | H | H | $CH_3$ | $NH_2$ | N | |
| $(CH_2)_4CH_3$ | H | H | $CH_3$ | $NHCH_3$ | CH | |
| $(CH_2)_4CH_3$ | H | H | $CH_3$ | $NHCH_3$ | N | |
| $(CH_2)_4CH_3$ | H | H | $CH_3$ | $N(CH_3)_2$ | CH | |
| $-CH_2$— 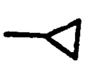 | H | H | $CH_3$ | $N(CH_3)_2$ | N | |
| $CH_2$— 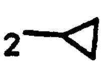 | H | H | $OCH_3$ | $C_2H_5$ | CH | |

Table VII (continued)

| $R_1$ | $R_2$ | $R_3$ | X | Y | Z | m.p.(°C) |
|---|---|---|---|---|---|---|
| $(CH_2)_4CH_3$ | H | H | $OCH_3$ | $C_2H_5$ | N | |
| $(CH_2)_4CH_3$ | H | H | $OCH_3$ | $OC_2H_5$ | CH | |
| $(CH_2)_4CH_3$ | H | H | $OCH_3$ | $OC_2H_5$ | N | |
| $(CH_2)_4CH_3$ | H | H | $OCH_3$ | $CH_2OCH_3$ | CH | |
| $(CH_2)_4CH_3$ | H | H | $OCH_3$ | $CH_2OCH_3$ | N | |
| 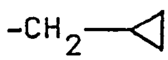 | H | H | $OCH_3$ | $CH(OCH_3)_2$ | CH | |
| 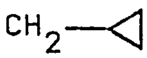 | H | H | $OCH_3$ | $CH(OCH_3)_2$ | N | |
| $(CH_2)_4CH_3$ | H | H | $OCH_3$ | 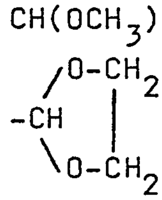 | CH | |
| $(CH_2)_4CH_3$ | H | H | $OCH_3$ | 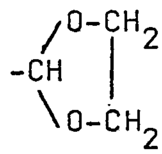 | N | |
| 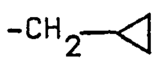 | H | H | $OCH_3$ | $NH_2$ | CH | |
| $(CH_2)_4CH_3$ | H | H | $OCH_3$ | $NH_2$ | N | |
| 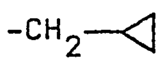 | H | H | $OCH_3$ | $NHCH_3$ | CH | |
| $(CH_2)_4CH_3$ | H | H | $OCH_3$ | $NHCH_3$ | N | |
| $(CH_2)_4CH_3$ | H | H | $OCH_3$ | $N(CH_3)_2$ | CH | |
| $(CH_2)_4CH_3$ | H | H | $OCH_3$ | $N(CH_3)_2$ | N | |
| 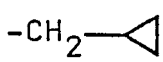 | H | H | Cl | $OCH_3$ | CH | |
| $(CH_2)_4CH_3$ | H | H | Cl | $OCH_3$ | CH | 141-142° |
| $(CH_2)_4CH_3$ | H | H | Cl | $CH_3$ | CH | |
| $(CH_2)_4CH_3$ | H | H | Cl | $OC_2H_5$ | CH | |
| 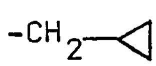 | H | H | Cl | $OC_2H_5$ | CH | |
| $(CH_2)_4CH_3$ | H | H | Cl | $NH_2$ | CH | |

Table VII (continued)

| $R_1$ | $R_2$ | $R_3$ | X | Y | Z | m.p. (°C) |
|---|---|---|---|---|---|---|
| $-CH_2$ 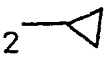 | H | H | Cl | $NH_2$ | CH | |
| $(CH_2)_4CH_3$ | H | H | Cl | $NHCH_3$ | CH | |
| $(CH_2)_4CH_3$ | H | H | Cl | $N(CH_3)_2$ | CH | |
| $C_6H_5$ | H | H | $OCH_3$ | $NH_2$ | CH | |
| 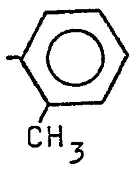 | H | H | $CH_3$ | $OC_2H_5$ | CH | |
| 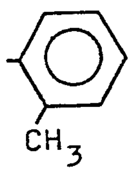 | H | H | Cl | $OCH_3$ | CH | |
| 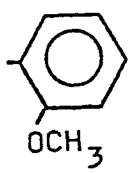 | H | H | $OCH_3$ | $OC_2H_5$ | CH | |
| $-CH_2CO_2C_2H_5$ | H | H | $CH_3$ | $OC_2H_5$ | CH | |
| $-CH_2CO_2CH_3$ | H | H | $OCH_3$ | $C_2H_5$ | N | |
| $-CH_2CO_2C_2H_5$ | H | H | $OCH_3$ | 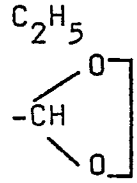 | CH | |
| $-CH_2CO_2CH_3$ | H | H | $OCH_3$ | $NH_2$ | N | |
| $-CH_2CO_2CH_3$ | H | H | Cl | $OC_2H_5$ | CH | |
| $-CH(CH_3)CO_2CH_3$ | H | H | $OCH_3$ | $C_2H_5$ | N | |
| $-CH(CH_3)CO_2C_2H_5$ | H | H | $CH_3$ | $OC_2H_5$ | CH | |
| $-CH(CH_3)CO_2CH_3$ | H | H | $OCH_3$ | $CH(OCH_3)_2$ | N | |
| $-CH(CH_3)CO_2CH_3$ | H | H | Cl | $OCH_3$ | CH | |
| $-CH(CH_3)CO_2C_2H_5$ | H | H | $OCH_3$ | $NH_2$ | N | |
| $-CH(CH_3)CO_2CH_3$ | H | H | $CH_3$ | $CH_2OCH_3$ | CH | |
| $-CH(CH_3)CO_2C_2H_5$ | H | H | $OCH_3$ | $N(CH_3)_2$ | CH | |
| $-CH(OCH_3)_2$ | H | H | $OCH_3$ | $OC_2H_5$ | N | |
| $-CH(OCH_3)_2$ | H | H | $OCH_3$ | $NH_2$ | CH | |

Table VII (continued)

| $R_1$ | $R_2$ | $R_3$ | X | Y | Z | m.p.(°C) |
|---|---|---|---|---|---|---|
| $CH(OC_2H_5)_2$ | H | H | $OCH_3$ | $CH_2OCH_3$ | CH | |
| $CH(OC_2H_5)_2$ | H | H | Cl | $OCH_3$ | CH | |
| $CH(OCH_3)_2$ | H | H | $OCH_3$ | 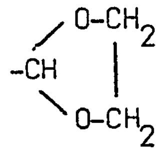 | CH | |
| $CH(OCH_3)_2$ | H | H | $OCH_3$ | $C_2H_5$ | N | |
| $-CH_2CN$ | H | H | $CH_3$ | $CH_2OCH_3$ | CH | |
| $-CH(CH_3)CN$ | H | H | $OCH_3$ | $OC_2H_5$ | N | |
| $-CH_2CH_2CN$ | H | H | $OCH_3$ | $NHCH_3$ | CH | |
| $-CH_2CH_2N(CH_3)_2$ | H | H | $OCH_3$ | $NH_2$ | CH | |
| $CH_2CH_2CN$ | H | H | Cl | $OCH_3$ | CH | |
| $CH_2CH_2CN$ | H | H | $CH_3$ | $OC_2H_5$ | N | |
| $CH_2CN$ | H | H | $OCH_3$ | 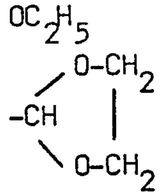 | CH | |
| $CH_2CN$ | H | H | $OCH_3$ | $C_2H_5$ | CH | |
| $-CH_2CH_2SO_2N(CH_3)_2$ | H | H | $OCH_3$ | $OC_2H_5$ | CH | |
| $-CH_2CH_2CO_2CH_3$ | H | H | Cl | $OCH_3$ | CH | |
| $-CH_2CH_2CO_2CH_3$ | H | H | $OCH_3$ | $NH_2$ | CH | |
| $-CH_2CH_2CO_2C_2H_5$ | H | H | $OCH_3$ | $N(CH_3)_2$ | CH | |
| $-CH_2CH_2CO_2C_2H_5$ | H | H | $CH_3$ | $OC_2H_5$ | N | |
| $C_6H_5$ | 5-$CF_3$ | H | $OCH_3$ | $OC_2H_5$ | CH | |
| 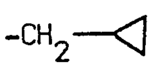 | 6-$OCH_3$ | H | Cl | $OCH_3$ | CH | |
| $(CH_2)_4CH_3$ | 5-Br | H | $OCH_3$ | $NH_2$ | N | |
| $(CH_2)_4CH_3$ | 5-$CF_3$ | H | $CH_3$ | $OC_2H_5$ | CH | |
| $(CH_2)_4CH_3$ | H | $CH_3$ | $OCH_3$ | 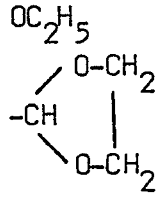 | CH | |
| $(CH_2)_4CH_3$ | H | $CH_3$ | Cl | $OCH_3$ | CH | |
| 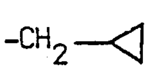 | H | $CH_3$ | $OCH_3$ | $OC_2H_5$ | N | |

Table VII (continued)

| $R_1$ | $R_2$ | $R_3$ | X | Y | Z | m.p.(°C) |
|---|---|---|---|---|---|---|
| 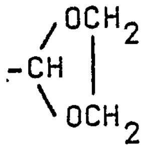 | H | H | Cl | $OCH_3$ | CH | |
| 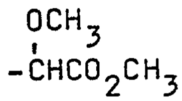 | H | H | Cl | $OCH_3$ | CH | |
| $CH(CH_3)CH_2CH_2CH_3$ | H | H | Cl | $OCH_3$ | CH | 155-160° |
| $CH(CH_2CH_3)_2$ | H | H | Cl | $OCH_3$ | CH | |
| 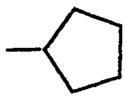 | H | H | Cl | $OCH_3$ | CH | |
| $-CH_2CH_2-OH$ | H | H | Cl | $OCH_3$ | CH | |
| 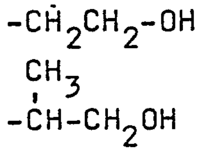 | H | H | Cl | $OCH_3$ | CH | |
| 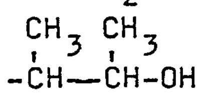 | H | H | Cl | $OCH_3$ | CH | |
| 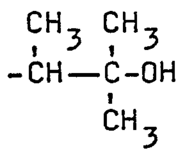 | H | H | Cl | $OCH_3$ | CH | |
| 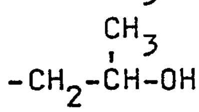 | H | H | Cl | $OCH_3$ | CH | |
| 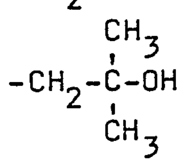 | H | H | Cl | $OCH_3$ | CH | |
| $-CH_2-CH_2-CH_2-OH$ | H | H | Cl | $OCH_3$ | CH | |
| 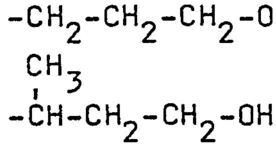 | H | H | Cl | $OCH_3$ | CH | |
| 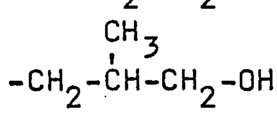 | H | H | Cl | $OCH_3$ | CH | |
| 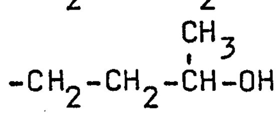 | H | H | Cl | $OCH_3$ | CH | |
| $-CH_2-CH_2-OSO_2CH_3$ | H | H | Cl | $OCH_3$ | CH | |
| $-CH_2-CH_2-CH_2-OSO_2CH_3$ | H | H | Cl | $OCH_3$ | CH | |

Table VII (continued)

| $R_1$ | $R_2$ | $R_3$ | X | Y | Z | m.p.(°C) |
|---|---|---|---|---|---|---|
| $-CH_2-CH_2-OC(O)CH_3$ | H | H | Cl | $OCH_3$ | CH | |
| $-CH_2-CH_2-CH_2OC(O)C_2H_5$ | H | H | Cl | $OCH_3$ | CH | |
| $-CH_2-CH_2-OC(O)NHCH_3$ | H | H | Cl | $OCH_3$ | CH | |
| $-CH_2-CH_2-OSO_2CF_3$ | H | H | Cl | $OCH_3$ | CH | |
| $-CH_2-CH_2-OH$ | H | $CH_3$ | Cl | $OCH_3$ | CH | |
| $-CH_2-CH_2-CH_2-OH$ | H | $CH_3$ | Cl | $OCH_3$ | CH | |
| $-CH_2-CH_2-OSO_2CH_3$ | H | $CH_3$ | Cl | $OCH_3$ | CH | |
| $-CH_2-CH_2-OH$ | $3-OCH_3$ | H | Cl | $OCH_3$ | CH | |
| $-CH_2-CH_2-OH$ | $5-CF_3$ | H | Cl | $OCH_3$ | CH | |
| $-CH_2-CH_2-CH_2-OH$ | $5-CF_3$ | H | Cl | $OCH_3$ | CH | |
| $-CH_2-CH_2-OSO_2CH_3$ | $5-CF_3$ | H | Cl | $OCH_3$ | CH | |
| $-CH_2-CH_2-OSO_2CH_3$ | $5-CF_3$ | $CH_3$ | Cl | $OCH_3$ | CH | |
| $-CH_2-CH_2OC(O)CH_3$ | $5-CF_3$ | H | Cl | $OCH_3$ | CH | |
| $-CH_2-CH_2-OH$ | H | H | Cl | $CH_3$ | CH | |
| $-CH_2-CH_2-OH$ | H | H | Cl | $OC_2H_5$ | CH | |
| $-CH_2-CH_2-OH$ | H | H | Cl | $NH_2$ | CH | |
| $-CH_2-CH_2-OH$ | H | H | Cl | $NHCH_3$ | CH | |
| $-CH_2-CH_2-OH$ | H | H | Cl | $N(CH_3)_2$ | CH | |
| $-CH_2-CH_2-OH$ | $5-CF_3$ | H | Cl | $NH_2$ | CH | |
| $-CH_2-CH_2OSO_2CH_3$ | H | H | Cl | $OC_2H_5$ | CH | |
| $-CH_2-CH_2OSO_2CH_3$ | H | H | Cl | $NH_2$ | CH | |
| $-CH_2-CH_2OSO_2CH_3$ | $5-CF_3$ | H | Cl | $NH_2$ | CH | |
| $-CH_2-CH_2-CH_2-OH$ | H | H | Cl | $CH_3$ | CH | |
| $-CH_2-CH_2-CH_2-OH$ | H | H | Cl | $OC_2H_5$ | CH | |
| $-CH_2-CH_2-CH_2-OH$ | H | H | Cl | $NH_2$ | CH | |
| $-CH_2-CH_2-OC(O)CH_3$ | H | H | Cl | $NHCH_3$ | CH | |
| $-CH_2-CH_2-OH$ | H | H | $CH_3$ | $C_2H_5$ | CH | |
| $-CH_2-CH_2-OH$ | H | H | $CH_3$ | $OC_2H_5$ | CH | |
| $-CH_2-CH_2-OH$ | H | H | $CH_3$ | $CH_2OCH_3$ | CH | |
| $-CH_2-CH_2-OH$ | H | H | $CH_3$ | $CH(OCH_3)_2$ | CH | |

Table VII (continued)

| $R_1$ | $R_2$ | $R_3$ | X | Y | Z | m.p.(°C) |
|---|---|---|---|---|---|---|
| $-CH_2-CH_2-OH$ | H | H | $CH_3$ | 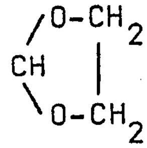 | CH | |
| $-CH_2-CH_2-OH$ | H | H | $CH_3$ | $NH_2$ | CH | |
| $-CH_2-CH_2-OH$ | H | H | $CH_3$ | $NHCH_3$ | CH | |
| $-CH_2-CH_2-OH$ | H | H | $CH_3$ | $N(CH_3)_2$ | CH | |
| $-CH_2-CH_2-OH$ | H | H | $OCH_3$ | $C_2H_5$ | CH | |
| $-CH_2-CH_2-OH$ | H | H | $OCH_3$ | $OC_2H_5$ | CH | |
| $-CH_2-CH_2-OH$ | H | H | $OCH_3$ | $CH_2OCH_3$ | CH | |
| $-CH_2-CH_2-OH$ | H | H | $OCH_3$ | $CH(OCH_3)_2$ | CH | |
| $-CH_2-CH_2-OH$ | H | H | $OCH_3$ | 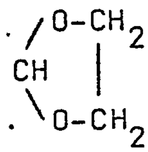 | CH | |
| $-CH_2-CH_2-OH$ | H | H | $OCH_3$ | $NH_2$ | CH | |
| $-CH_2-CH_2-OH$ | H | H | $OCH_3$ | $NHCH_3$ | CH | |
| $-CH_2-CH_2-OH$ | H | H | $OCH_3$ | $N(CH_3)_2$ | CH | |
| $-CH_2-CH_2-OH$ | H | H | $CH_3$ | $C_2H_5$ | N | |
| $-CH_2-CH_2-OH$ | H | H | $CH_3$ | $OC_2H_5$ | N | |
| $-CH_2-CH_2-OH$ | H | H | $CH_3$ | $CH_2OCH_3$ | N | |
| $-CH_2-CH_2-OH$ | H | H | $CH_3$ | $CH(OCH_3)_2$ | N | |
| $-CH_2-CH_2-OH$ | H | H | $CH_3$ | 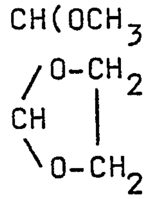 | N | |
| $-CH_2-CH_2-OH$ | H | H | $CH_3$ | $NH_2$ | N | |
| $-CH_2-CH_2-OH$ | H | H | $CH_3$ | $NHCH_3$ | N | |
| $-CH_2-CH_2-OH$ | H | H | $CH_3$ | $N(CH_3)_2$ | N | |
| $-CH_2-CH_2-OH$ | H | H | $OCH_3$ | $C_2H_5$ | N | |
| $-CH_2-CH_2-OH$ | H | H | $OCH_3$ | $OC_2H_5$ | N | |
| $-CH_2-CH_2-OH$ | H | H | $OCH_3$ | $CH_2OCH_3$ | N | |
| $-CH_2-CH_2-OH$ | H | H | $OCH_3$ | $CH(OCH_3)_2$ | N | |
| $-CH_2-CH_2-OH$ | H | H | $OCH_3$ | 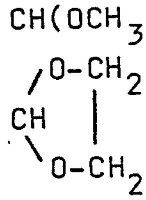 | N | |

Table VII (continued)

| $R_1$ | $R_2$ | $R_3$ | X | Y | Z | m.p. (°C) |
|---|---|---|---|---|---|---|
| $-CH_2-CH_2-OH$ | H | H | $OCH_3$ | $NH_2$ | N | |
| $-CH_2-CH_2-OH$ | H | H | $OCH_3$ | $NHCH_3$ | N | |
| $-CH_2-CH_2-OH$ | H | H | $OCH_3$ | $N(CH_3)_2$ | N | |
| $-CH_2-CH_2-CH_2-OH$ | H | H | $CH_3$ | $OC_2H_5$ | CH | |
| $-CH_2-CH_2-CH_2-OH$ | H | H | $CH_3$ | $CH_2OCH_3$ | CH | |
| $-CH_2-CH_2-CH_2-OH$ | H | H | $CH_3$ | $CH(OCH_3)_2$ | CH | |
| $-CH_2-CH_2-CH_2-OH$ | H | H | $CH_3$ | 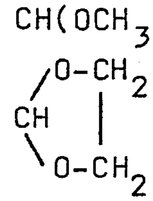 | CH | |
| $-CH_2-CH_2-CH_2-OH$ | H | H | $CH_3$ | $NH_2$ | CH | |
| $-CH_2-CH_2-CH_2-OH$ | H | H | $CH_3$ | $NHCH_3$ | CH | |
| $-CH_2-CH_2-CH_2-OH$ | H | H | $CH_3$ | $N(CH_3)_2$ | CH | |
| $-CH_2-CH_2-CH_2-OH$ | H | H | $OCH_3$ | $C_2H_5$ | CH | |
| $-CH_2-CH_2-CH_2-OH$ | H | H | $OCH_3$ | $OC_2H_5$ | CH | |
| $-CH_2-CH_2-CH_2-OH$ | H | H | $OCH_3$ | $CH_2OCH_3$ | CH | |
| $-CH_2-CH_2-CH_2-OH$ | H | H | $OCH_3$ | 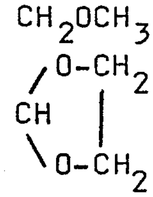 | CH | |
| $-CH_2-CH_2-CH_2-OH$ | H | H | $OCH_3$ | $NH_2$ | CH | |
| $-CH_2-CH_2-CH_2-OH$ | H | H | $OCH_3$ | $NHCH_3$ | CH | |
| $-CH_2-CH_2-CH_2-OH$ | H | H | $OCH_3$ | $N(CH_3)_2$ | CH | |
| $-CH_2-CH_2-CH_2-OH$ | H | H | $CH_3$ | $OC_2H_5$ | N | |
| $-CH_2-CH_2-CH_2-OH$ | H | H | $CH_3$ | $CH_2OCH_3$ | N | |
| $-CH_2-CH_2-CH_2-OH$ | H | H | $CH_3$ | $CH(OCH_3)_2$ | N | |
| $-CH_2-CH_2-CH_2-OH$ | H | H | $CH_3$ | $NH_2$ | N | |
| $-CH_2-CH_2-CH_2-OH$ | H | H | $CH_3$ | $NHCH_3$ | N | |
| $-CH_2-CH_2-CH_2-OH$ | H | H | $CH_3$ | $N(CH_3)_2$ | N | |
| $-CH_2-CH_2-CH_2-OH$ | H | H | $OCH_3$ | $C_2H_5$ | N | |
| $-CH_2-CH_2-CH_2-OH$ | H | H | $OCH_3$ | $OC_2H_5$ | N | |
| $-CH_2-CH_2-CH_2-OH$ | H | H | $OCH_3$ | $CH_2OCH_3$ | N | |
| $-CH_2-CH_2-CH_2-OH$ | H | H | $OCH_3$ | $CH(OCH_3)_2$ | N | |

Table VII (continued)

| $R_1$ | $R_2$ | $R_3$ | X | Y | Z | m.p. (°C) |
|---|---|---|---|---|---|---|
| $-CH_2-CH_2-CH_2-OH$ | H | H | $OCH_3$ | 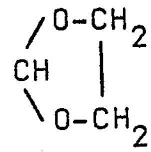 | N | |
| $-CH_2-CH_2-CH_2-OH$ | H | H | $OCH_3$ | $NH_2$ | N | |
| $-CH_2-CH_2-CH_2-OH$ | H | H | $OCH_3$ | $NHCH_3$ | N | |
| $-CH_2-CH_2-CH_2-OH$ | H | H | $OCH_3$ | $N(CH_3)_2$ | N | |
| $-CH_2-CH_2-OSO_2CH_3$ | H | H | $CH_3$ | $CH(OCH_3)_2$ | CH | |
| $-CH_2-CH_2-OSO_2CH_3$ | H | H | $CH_3$ | $NH_2$ | CH | |
| $-CH_2-CH_2-OSO_2CH_3$ | H | H | $CH_3$ | $N(CH_3)_2$ | CH | |
| $-CH_2-CH_2-OSO_2CH_3$ | H | H | $OCH_3$ | $CH_2OCH_3$ | CH | |
| $-CH_2-CH_2-OSO_2CH_3$ | H | H | $OCH_3$ | $NH_2$ | CH | |
| $-CH_2-CH_2-OSO_2CH_3$ | H | H | $CH_3$ | $CH(OCH_3)_2$ | N | |
| $-CH_2-CH_2-OSO_2CH_3$ | H | H | $CH_3$ | $NH_2$ | N | |
| $-CH_2-CH_2-OSO_2CH_3$ | H | H | $OCH_3$ | $CH_2OCH_3$ | N | |
| $-CH_2-CH_2-OSO_2CH_3$ | H | H | $OCH_3$ | $NHCH_3$ | N | |
| $-CH_2-CH_2-OC(O)CH_3$ | H | H | $CH_3$ | $CH(OCH_3)_2$ | CH | |
| $-CH_2-CH_2-OC(O)CH_3$ | H | H | $CH_3$ | $NH_2$ | CH | |
| $-CH_2-CH_2-OC(O)CH_3$ | H | H | $OCH_3$ | 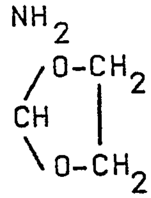 | CH | |
| $-CH_2-CH_2-OC(O)CH_3$ | H | H | $OCH_3$ | $NH_2$ | CH | |
| $-CH_2-CH_2-OC(O)CH_3$ | H | H | $CH_3$ | $CH(OCH_3)_2$ | N | |
| $-CH_2-CH_2-OC(O)CH_3$ | H | H | $OCH_3$ | $NH_2$ | N | |
| $-CH_2-CH_2-OH$ | 5-$CF_3$ | H | $CH_3$ | $NH_2$ | CH | |
| $-CH_2\overset{CH_3}{CH}-OH$ | H | H | $OCH_3$ | $CH(OCH_3)_2$ | N | |
| $-CH_2-CH_2-CH_2-OH$ | 5-$CF_3$ | H | $OCH_3$ | $NH_2$ | CH | |
| $-CH_2-CH_2-OSO_2CH_3$ | 5-$CF_3$ | H | $OCH_3$ | 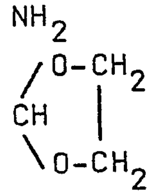 | CH | |

Table VII (continued)

| $R_1$ | $R_2$ | $R_3$ | X | Y | Z | m.p.(°C) |
|---|---|---|---|---|---|---|
| $CH_2C{\equiv}CH$ | H | H | $CH_3$ | $OCF_3$ | CH | |
| $CH_2C{\equiv}CH$ | H | H | $OCH_3$ | $OCF_3$ | CH | |
| $CH_2C{\equiv}CH$ | H | H | $CH_3$ | $OCF_2H$ | CH | |
| $CH_2C{\equiv}CH$ | H | H | $OCH_3$ | $OCF_2H$ | CH | |
| $CH_2C{\equiv}CH$ | H | H | $CH_3$ | $SCF_2H$ | CH | |
| $CH_2C{\equiv}CH$ | H | H | $OCH_3$ | $SCF_2H$ | CH | |
| $CH_2C{\equiv}CH$ | H | H | $CH_3$ | $OCF_2CHFCl$ | CH | |
| $CH_2C{\equiv}CH$ | H | H | $OCH_3$ | $OCF_2CHFCl$ | CH | |
| $CH_2C{\equiv}CH$ | H | H | $CH_3$ | $SCF_2CHFCl$ | CH | |
| $CH_2C{\equiv}CH$ | H | H | $OCH_3$ | $SCF_2CHFCl$ | CH | |
| $CH_2C{\equiv}CH$ | H | H | $CH_3$ | $OCF_2CHFBr$ | CH | |
| $CH_2C{\equiv}CH$ | H | H | $OCH_3$ | $OCF_2CHFBr$ | CH | |
| $CH_2C{\equiv}CH$ | H | H | $CH_3$ | $SCF_2CHFBr$ | CH | |
| $CH_2C{\equiv}CH$ | H | H | $OCH_3$ | $SCF_2CHFBr$ | CH | |
| $CH_2C{\equiv}CH$ | H | H | $CH_3$ | $OCF_2CF_2H$ | CH | |
| $CH_2C{\equiv}CH$ | H | H | $OCH_3$ | $OCF_2CF_2H$ | CH | |
| $CH_2C{\equiv}CH$ | H | H | $CH_3$ | $SCF_2CF_2H$ | CH | |
| $CH_2C{\equiv}CH$ | H | H | $OCH_3$ | $SCF_2CF_2H$ | CH | |
| $CH_2C{\equiv}CH$ | H | H | $CH_3$ | $OCF_2CHFCF_3$ | CH | |
| $CH_2C{\equiv}CH$ | H | H | $OCH_3$ | $OCF_2CHFCF_3$ | CH | |
| $CH_2C{\equiv}CH$ | H | H | $CH_3$ | $SCF_2CHFCF_3$ | CH | |
| $CH_2C{\equiv}CH$ | H | H | $OCH_3$ | $SCF_2CHFCF_3$ | CH | |
| $CH_2C{\equiv}CH$ | H | H | $OCF_2H$ | $OCF_2H$ | CH | |
| $CH_2C{\equiv}CH$ | H | H | $SCF_2H$ | $OCF_2H$ | CH | |
| $CH_2C{\equiv}CH$ | H | H | $SCF_2H$ | $OCF_2H$ | Z | |
| $CH_2C{\equiv}CH$ | H | H | $CH_3$ | $NH_2$ | CH | |
| $CH_2C{\equiv}CH$ | H | H | $OCH_3$ | $C_2H_5$ | CH | |
| $CH_2C{\equiv}CH$ | H | H | $OCH_3$ | $CH_2OCH_3$ | CH | |
| $CH_2C{\equiv}CH$ | H | H | $CH_3$ | $CH(OCH_3)_2$ | N | |
| $CH_2C{\equiv}CH$ | H | H | $CH_3$ | $OC_2H_5$ | N | |
| $CH_2C{\equiv}CH$ | H | H | $CH_3$ | $N(CH_3)_2$ | N | |

Table VIII

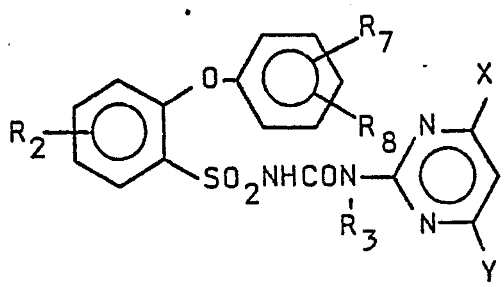

| $R_2$ | $R_3$ | $R_7$ | $R_8$ | X | Y | m.p.(°C) |
|---|---|---|---|---|---|---|
| H | $CH_3$ | 2-F | H | $CH_3$ | $CH_3$ | |
| H | $CH_3$ | 2-F | H | $CH_3$ | $OCH_3$ | |
| H | $CH_3$ | 2-F | H | $OCH_3$ | $OCH_3$ | |
| H | H | 2-F | H | $CH_3$ | $C_2H_5$ | |
| H | H | 2-F | H | $OCH_3$ | $C_2H_5$ | |
| H | H | 2-F | H | $CH_3$ | $OC_2H_5$ | |
| H | H | 2-F | H | $OCH_3$ | $OC_2H_5$ | |
| H | H | 2-F | H | Cl | $OCH_3$ | |
| H | H | 2-F | H | Cl | $CH_3$ | |
| H | H | 2-F | H | Cl | $OC_2H_5$ | |
| H | H | 2-F | H | Cl | $NH_2$ | |
| H | H | 2-F | H | Cl | $NHCH_3$ | |
| H | H | 2-F | H | Cl | $N(CH_3)_2$ | |
| H | H | 2-F | H | $CH_3$ | $CH_2OCH_3$ | |
| H | H | 2-F | H | $OCH_3$ | $CH_2OCH_3$ | |
| H | H | 2-F | H | $CH_3$ | $CH(OCH_3)_2$ | |
| H | H | 2-F | H | $OCH_3$ | $CH(OCH_3)_2$ | |
| H | H | 2-F | H | $CH_3$ | 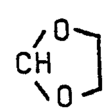 | |
| H | H | 2-F | H | $OCH_3$ | 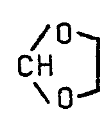 | |
| H | H | 2-F | H | $CH_3$ | $NH_2$ | |
| H | H | 2-F | H | $OCH_3$ | $NH_2$ | |
| H | H | 2-F | H | $CH_3$ | $NHCH_3$ | |

Table VIII (continued)

| $R_2$ | $R_3$ | $R_7$ | $R_8$ | X | Y | m.p.(°C) |
|---|---|---|---|---|---|---|
| H | H | 2-F | H | $OCH_3$ | $NHCH_3$ | |
| H | H | 2-F | H | $CH_3$ | $N(CH_3)_2$ | |
| H | H | 2-F | H | $OCH_3$ | $N(CH_3)_2$ | |
| H | H | $2-CH_3$ | H | $CH_3$ | $CH_3$ | |
| H | H | $2-CH_3$ | H | $CH_3$ | $OCH_3$ | 201-203° |
| H | H | $2-CH_3$ | H | $OCH_3$ | $OCH_3$ | 202-205° |
| H | H | $3-CH_3$ | H | $CH_3$ | $CH_3$ | |
| H | H | $3-CH_3$ | H | $CH_3$ | $OCH_3$ | |
| H | H | $3-CH_3$ | H | $OCH_3$ | $OCH_3$ | |
| H | H | $4-CH_3$ | H | $CH_3$ | $CH_3$ | |
| H | H | $4-CH_3$ | H | $CH_3$ | $OCH_3$ | |
| H | H | $4-CH_3$ | H | $OCH_3$ | $OCH_3$ | |
| H | H | $2-CO_2CH_3$ | H | $CH_3$ | $CH_3$ | |
| H | H | $2-CO_2CH_3$ | H | $CH_3$ | $OCH_3$ | 196-198° |
| H | H | $2-CO_2CH_3$ | H | $OCH_3$ | $OCH_3$ | |
| H | H | $3-CO_2CH_3$ | H | $CH_3$ | $CH_3$ | |
| H | H | $3-CO_2CH_3$ | H | $CH_3$ | $OCH_3$ | |
| H | H | $3-CO_2CH_3$ | H | $OCH_3$ | $OCH_3$ | |
| H | H | $4-CO_2CH_3$ | H | $CH_3$ | $CH_3$ | |
| H | H | $4-CO_2CH_3$ | H | $CH_3$ | $OCH_3$ | |
| H | H | $4-CO_2CH_3$ | H | $OCH_3$ | $OCH_3$ | |
| H | H | 2-Cl | H | $CH_3$ | $CH_3$ | |
| H | H | 2-Cl | H | $CH_3$ | $OCH_3$ | 225-227° |
| H | H | 2-Cl | H | $OCH_3$ | $OCH_3$ | 224-226° |
| H | H | 2-Cl | 4-Cl | $CH_3$ | $CH_3$ | 201-203° |
| H | H | 2-Cl | 4-Cl | $CH_3$ | $OCH_3$ | 217-219° |
| H | H | 2-Cl | 4-Cl | $OCH_3$ | $OCH_3$ | 185-187° |
| H | H | 2-Cl | 3-Cl | $CH_3$ | $CH_3$ | 172-189° |
| H | H | 2-Cl | 3-Cl | $CH_3$ | $OCH_3$ | 160-179° |
| H | H | 2-Cl | 3-Cl | $OCH_3$ | $OCH_3$ | 211-213° |
| H | H | $2-NO_2$ | H | $CH_3$ | $CH_3$ | |
| H | H | $2-NO_2$ | H | $CH_3$ | $OCH_3$ | |

Table VIII (continued)

| $R_2$ | $R_3$ | $R_7$ | $R_8$ | X | Y | m.p.(°C) |
|---|---|---|---|---|---|---|
| H | H | 2-$NO_2$ | H | $OCH_3$ | $OCH_3$ | |
| H | H | 2-$CF_3$ | H | $CH_3$ | $CH_3$ | |
| H | H | 2-$CF_3$ | H | $CH_3$ | $OCH_3$ | |
| H | H | 2-$CF_3$ | H | $OCH_3$ | $OCH_3$ | |
| H | H | 3-$NO_2$ | H | $CH_3$ | $CH_3$ | |
| H | H | 3-$NO_2$ | H | $CH_3$ | $OCH_3$ | |
| H | H | 3-$NO_2$ | H | $OCH_3$ | $OCH_3$ | |
| H | H | 2-$SO_2CH_3$ | H | $CH_3$ | $CH_3$ | |
| H | H | 2-$SO_2CH_3$ | H | $CH_3$ | $OCH_3$ | |
| H | H | 2-$SO_2CH_3$ | H | $OCH_3$ | $OCH_3$ | |
| H | H | 2-$CO_2CH_3$ | 4-$CH_3$ | $CH_3$ | $OCH_3$ | |
| H | H | 2-$OCH_3$ | H | $CH_3$ | $CH_3$ | |
| H | H | 2-$OCH_3$ | H | $CH_3$ | $OCH_3$ | 112-115° |
| H | H | 2-$OCH_3$ | H | $OCH_3$ | $OCH_3$ | 203-206° |
| H | H | 3-$OCH_3$ | H | $CH_3$ | $CH_3$ | |
| H | H | 3-$OCH_3$ | H | $CH_3$ | $OCH_3$ | 90-98° |
| H | H | 3-$OCH_3$ | H | $OCH_3$ | $OCH_3$ | 141°(d) |
| 5-Cl | H | 2-F | H | $CH_3$ | $CH_3$ | |
| 5-Cl | H | 2-F | H | $CH_3$ | $OCH_3$ | |
| 5-Cl | H | 2-F | H | $OCH_3$ | $OCH_3$ | |
| 6-Cl | H | 2-F | H | $CH_3$ | $OCH_3$ | |
| 5-$CH_3$ | H | 2-F | H | $CH_3$ | $OCH_3$ | |
| 6-$CH_3$ | H | 2-F | H | $CH_3$ | $OCH_3$ | |
| 5-$CF_3$ | H | 2-F | H | $CH_3$ | $OCH_3$ | |
| 5-$OCH_3$ | H | 2-F | H | $CH_3$ | $OCH_3$ | |
| 3-$OCH_3$ | H | 2-F | H | $CH_3$ | $OCH_3$ | |
| 3-Cl | H | 2-F | H | $CH_3$ | $OCH_3$ | |
| 5-$CF_3$ | H | 2-$SO_2CH_3$ | H | $CH_3$ | $OCH_3$ | |
| 5-$CF_3$ | H | 2-$SO_2CH_3$ | H | $OCH_3$ | $OCH_3$ | |
| H | $CH_3$ | 2-$CO_2CH_3$ | H | $OCH_3$ | $OCH_3$ | |
| H | $CH_3$ | 2-$CH_3$ | H | $OCH_3$ | $OCH_3$ | |
| H | $CH_3$ | 2-$CF_3$ | H | $OCH_3$ | $OCH_3$ | |

Table VIII (continued)

| $R_2$ | $R_3$ | $R_7$ | $R_8$ | X | Y | m.p.(°C) |
|---|---|---|---|---|---|---|
| H | $CH_3$ | 2-Cl | H | $OCH_3$ | $OCH_3$ | |
| H | H | $2\text{-}CO_2CH_3$ | H | $OCF_2H$ | $OCF_2H$ | |
| H | H | $2\text{-}CO_2CH_3$ | H | $OCF_2H$ | $SCF_2H$ | |
| H | H | $2\text{-}CO_2CH_3$ | H | $SCF_2H$ | $SCF_2H$ | |
| H | H | $2\text{-}CO_2CH_3$ | H | $CH_3$ | $OCF_3$ | |
| H | H | $2\text{-}CO_2CH_3$ | H | $OCH_3$ | $OCF_3$ | |
| H | H | $2\text{-}CO_2CH_3$ | H | $CH_3$ | $OCF_2H$ | |
| H | H | $2\text{-}CO_2CH_3$ | H | $OCH_3$ | $OCF_2H$ | |
| H | H | $2\text{-}CO_2CH_3$ | H | $CH_3$ | $SCF_2H$ | |
| H | H | $2\text{-}CO_2CH_3$ | H | $OCH_3$ | $SCF_2H$ | |
| H | H | $2\text{-}CO_2CH_3$ | H | $OCH_3$ | $OCF_2CHFCl$ | |
| H | H | $2\text{-}CO_2CH_3$ | H | $CH_3$ | $SCF_2CHFCl$ | |
| H | H | $2\text{-}CO_2CH_3$ | H | $OCH_3$ | $OCF_2CHBrF$ | |
| H | H | $2\text{-}CO_2CH_3$ | H | $CH_3$ | $SCF_2CHBrF$ | |
| H | H | $2\text{-}CO_2CH_3$ | H | $OCH_3$ | $OCF_2CHFCF_3$ | |
| H | H | $2\text{-}CO_2CH_3$ | H | $CH_3$ | $SCF_2CHFCF_3$ | |
| H | H | $2\text{-}CO_2CH_3$ | H | $CH_3$ | $OCF_2CHF_2$ | |
| H | H | $2\text{-}CO_2CH_3$ | H | $OCH_3$ | $OCF_2CHF_2$ | |
| H | H | $2\text{-}CO_2CH_3$ | H | $CH_3$ | $SCF_2CHF_2$ | |
| H | H | $2\text{-}CO_2CH_3$ | H | $OCH_3$ | $SCF_2CHF_2$ | |

Table IX

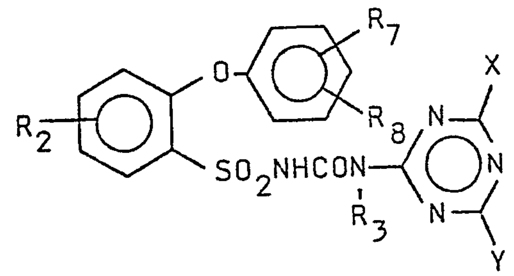

| $R_2$ | $R_3$ | $R_7$ | $R_8$ | X | Y | m.p.(°C) |
|---|---|---|---|---|---|---|
| H | $CH_3$ | 2-F | H | $CH_3$ | $CH_3$ | |
| H | $CH_3$ | 2-F | H | $CH_3$ | $OCH_3$ | |
| H | $CH_3$ | 2-F | H | $OCH_3$ | $OCH_3$ | |
| H | H | 2-F | H | $CH_3$ | $C_2H_5$ | |
| H | H | 2-F | H | $OCH_3$ | $C_2H_5$ | |
| H | H | 2-F | H | $CH_3$ | $OC_2H_5$ | |
| H | H | 2-F | H | $OCH_3$ | $OC_2H_5$ | |
| H | H | 2-F | H | $CH_3$ | $CH_2OCH_3$ | |
| H | H | 2-F | H | $OCH_3$ | $CH_2OCH_3$ | |
| H | H | 2-F | H | $CH_3$ | $CH(OCH_3)_2$ | |
| H | H | 2-F | H | $OCH_3$ | $CH(OCH_3)_2$ | |
| H | H | 2-F | H | $CH_3$ | 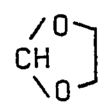 | |
| H | H | 2-F | H | $OCH_3$ | 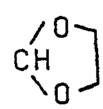 | |
| H | H | 2-F | H | $CH_3$ | $NH_2$ | |
| H | H | 2-F | H | $OCH_3$ | $NH_2$ | |
| H | H | 2-F | H | $CH_3$ | $NHCH_3$ | |

Table IX (continued)

| $R_2$ | $R_3$ | $R_7$ | $R_8$ | X | Y | m.p.(°C) |
|---|---|---|---|---|---|---|
| H | H | 2-F | H | $OCH_3$ | $NHCH_3$ | |
| H | H | 2-F | H | $CH_3$ | $N(CH_3)_2$ | |
| H | H | 2-F | H | $OCH_3$ | $N(CH_3)_2$ | |
| H | H | $2-CH_3$ | H | $CH_3$ | $CH_3$ | |
| H | H | $2-CH_3$ | H | $CH_3$ | $OCH_3$ | 175-177° |
| H | H | $2-CH_3$ | H | $OCH_3$ | $OCH_3$ | 152-158° |
| H | H | $3-CH_3$ | H | $CH_3$ | $CH_3$ | |
| H | H | $3-CH_3$ | H | $CH_3$ | $OCH_3$ | |
| H | H | $3-CH_3$ | H | $OCH_3$ | $OCH_3$ | |
| H | H | $4-CH_3$ | H | $CH_3$ | $CH_3$ | |
| H | H | $4-CH_3$ | H | $CH_3$ | $OCH_3$ | |
| H | H | $4-CH_3$ | H | $OCH_3$ | $OCH_3$ | |
| H | H | $2-CO_2CH_3$ | H | $CH_3$ | $CH_3$ | |
| H | H | $2-CO_2CH_3$ | H | $CH_3$ | $OCH_3$ | |
| H | H | $2-CO_2CH_3$ | H | $OCH_3$ | $OCH_3$ | |
| H | H | $3-CO_2CH_3$ | H | $CH_3$ | $CH_3$ | |
| H | H | $3-CO_2CH_3$ | H | $CH_3$ | $OCH_3$ | |
| H | H | $3-CO_2CH_3$ | H | $OCH_3$ | $OCH_3$ | |
| H | H | $4-CO_2CH_3$ | H | $CH_3$ | $CH_3$ | |
| H | H | $4-CO_2CH_3$ | H | $CH_3$ | $OCH_3$ | |
| H | H | $4-CO_2CH_3$ | H | $OCH_3$ | $OCH_3$ | |
| H | H | 2-Cl | H | $CH_3$ | $CH_3$ | |
| H | H | 2-Cl | H | $CH_3$ | $OCH_3$ | 207-208° |
| H | H | 2-Cl | H | $OCH_3$ | $OCH_3$ | 205-209° |
| H | H | 2-Cl | 4-Cl | $CH_3$ | $CH_3$ | |
| H | H | 2-Cl | 4-Cl | $CH_3$ | $OCH_3$ | |
| H | H | 2-Cl | 4-Cl | $OCH_3$ | $OCH_3$ | 179-181° |
| H | H | 2-Cl | 3-Cl | $CH_3$ | $CH_3$ | |
| H | H | 2-Cl | 3-Cl | $CH_3$ | $OCH_3$ | 178-181° |
| H | H | 2-Cl | 3-Cl | $OCH_3$ | $OCH_3$ | 154-170° |
| H | H | $2-NO_2$ | H | $CH_3$ | $CH_3$ | |
| H | H | $2-NO_2$ | H | $CH_3$ | $OCH_3$ | |

Table IX (continued)

| $R_2$ | $R_3$ | $R_7$ | $R_8$ | X | Y | m.p.(°C) |
|---|---|---|---|---|---|---|
| H | H | $2-NO_2$ | H | $OCH_3$ | $OCH_3$ | |
| H | H | $2-CF_3$ | H | $CH_3$ | $CH_3$ | |
| H | H | $2-CF_3$ | H | $CH_3$ | $OCH_3$ | |
| H | H | $2-CF_3$ | H | $OCH_3$ | $OCH_3$ | |
| H | H | $3-NO_2$ | H | $CH_3$ | $CH_3$ | |
| H | H | $3-NO_2$ | H | $CH_3$ | $OCH_3$ | |
| H | H | $3-NO_2$ | H | $OCH_3$ | $OCH_3$ | |
| H | H | $2-SO_2CH_3$ | H | $CH_3$ | $CH_3$ | |
| H | H | $2-SO_2CH_3$ | H | $CH_3$ | $OCH_3$ | |
| H | H | $2-SO_2CH_3$ | H | $OCH_3$ | $OCH_3$ | |
| H | H | $2-CO_2CH_3$ | $4-CH_3$ | $CH_3$ | $OCH_3$ | |
| H | H | $2-OCH_3$ | H | $CH_3$ | $CH_3$ | |
| H | H | $2-OCH_3$ | H | $CH_3$ | $OCH_3$ | |
| H | H | $2-OCH_3$ | H | $OCH_3$ | $OCH_3$ | 140-145° |
| H | H | $3-OCH_3$ | H | $CH_3$ | $CH_3$ | |
| H | H | $3-OCH_3$ | H | $CH_3$ | $OCH_3$ | 115°(d) |
| H | H | $3-OCH_3$ | H | $OCH_3$ | $OCH_3$ | 150°(d) |
| 5-Cl | H | 2-F | H | $CH_3$ | $CH_3$ | |
| 5-Cl | H | 2-F | H | $CH_3$ | $OCH_3$ | |
| 5-Cl | H | 2-F | H | $OCH_3$ | $OCH_3$ | |
| 6-Cl | H | 2-F | H | $CH_3$ | $OCH_3$ | |
| $5-CH_3$ | H | 2-F | H | $CH_3$ | $OCH_3$ | |
| $6-CH_3$ | H | 2-F | H | $CH_3$ | $OCH_3$ | |
| $5-CF_3$ | H | 2-F | H | $CH_3$ | $OCH_3$ | |
| $5-OCH_3$ | H | 2-F | H | $CH_3$ | $OCH_3$ | |
| $3-OCH_3$ | H | 2-F | H | $CH_3$ | $OCH_3$ | |
| 3-Cl | H | 2-F | H | $CH_3$ | $OCH_3$ | |
| $5-CF_3$ | H | $2-SO_2CH_3$ | H | $CH_3$ | $OCH_3$ | |
| $5-CF_3$ | H | $2-SO_2CH_3$ | H | $OCH_3$ | $OCH_3$ | |
| H | $CH_3$ | $2-CO_2CH_3$ | H | $OCH_3$ | $OCH_3$ | |
| H | $CH_3$ | $2-CH_3$ | H | $OCH_3$ | $OCH_3$ | |
| H | $CH_3$ | $2-CF_3$ | H | $OCH_3$ | $OCH_3$ | |

Table IX (continued)

| $R_2$ | $R_3$ | $R_7$ | $R_8$ | X | Y | m.p.(°C) |
|---|---|---|---|---|---|---|
| H | H | $2-CO_2CH_3$ | H | $OCF_2H$ | $OCF_2H$ | |
| H | H | $2-CO_2CH_3$ | H | $OCF_2H$ | $SCF_2H$ | |
| H | H | $2-CO_2CH_3$ | H | $SCF_2H$ | $SCF_2H$ | |
| H | H | $2-CO_2CH_3$ | H | $CH_3$ | $OCF_3$ | |
| H | H | $2-CO_2CH_3$ | H | $OCH_3$ | $OCF_3$ | |
| H | H | $2-CO_2CH_3$ | H | $CH_3$ | $OCF_2H$ | |
| H | H | $2-CO_2CH_3$ | H | $OCH_3$ | $OCF_2H$ | |
| H | H | $2-CO_2CH_3$ | H | $CH_3$ | $SCF_2H$ | |
| H | H | $2-CO_2CH_3$ | H | $OCH_3$ | $SCF_2H$ | |
| H | H | $2-CO_2CH_3$ | H | $OCH_3$ | $OCF_2CHFCl$ | |
| H | H | $2-CO_2CH_3$ | H | $CH_3$ | $SCF_2CHFCl$ | |
| H | H | $2-CO_2CH_3$ | H | $OCH_3$ | $OCF_2CHBrF$ | |
| H | H | $2-CO_2CH_3$ | H | $CH_3$ | $SCF_2CHBrF$ | |
| H | H | $2-CO_2CH_3$ | H | $OCH_3$ | $OCF_2CHFCF_3$ | |
| H | H | $2-CO_2CH_3$ | H | $CH_3$ | $SCF_2CHFCF_3$ | |
| H | H | $2-CO_2CH_3$ | H | $CH_3$ | $OCF_2CHF_2$ | |
| H | H | $2-CO_2CH_3$ | H | $OCH_3$ | $OCF_2CHF_2$ | |
| H | H | $2-CO_2CH_3$ | H | $CH_3$ | $SCF_2CHF_2$ | |
| H | H | $2-CO_2CH_3$ | H | $OCH_3$ | $SCF_2CHF_2$ | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table X

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", <u>Chemical Engineering</u>,

December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 15

Wettable Powder

| | |
|---|---|
| N-[(4,6-dimethoxy-1,3,5-triazin-2-yl)aminocarbonyl]-2-(2-methylphenoxy)benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

## Example 16

Wettable Powder

| | |
|---|---|
| N-[(4,6-dimethoxypyrimidin-2-yl)aminocarbonyl]-2-(cyclopropylmethyloxy)benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

## Example 17

Granule

| | |
|---|---|
| Wettable Powder of Example 16 | 5% |
| attapulgite granules (U.S.S. 20-40 mesh; 0.84-0.42 mm) | 95% |

A slurry of wettable powder containing ≈25% solids is sprayed on the surface of attapulgite granules in a double-cone blender. The granules are dried and packaged.

## Example 18

Extruded Pellet

| | |
|---|---|
| N-[(4-methoxy-6-methyl-1,3,5-triazin-2-yl)aminocarbonyl]-2-(cyclopropylmethyloxy)benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm

openings). The granules held on a U.S.S. No. 40 sieve (0.42 mm openings) may be packaged for use and the fines recycled.

Example 19

Wettable Powder

| | |
|---|---|
| N-[(4,6-dimethoxy-1,3,5-triazin-2-yl)aminocarbonyl]-2-(2-methylphenoxy)benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 20

Low Strength Granule

| | |
|---|---|
| N-[(4,6-dimethoxy-1,3,5-triazin-2-yl)aminocarbonyl]-2-(cyclopropylmethyloxy)benzenesulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 21

Wettable Powder

| | |
|---|---|
| N-[(4-methoxy-6-methyl-1,3,5-triazin-2-yl)aminocarbonyl]-2-(2-hydroxyethoxy)benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 22

Wettable Powder

| | |
|---|---|
| N-[(4,6-dimethoxy-1,3,5-triazin-2-yl)aminocarbonyl]-2-(2-methylphenoxy)benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 23

Oil Suspension

| | |
|---|---|
| N-[(4,6-dimethoxypyrimidin-2-yl)aminocarbonyl]-2-(2-hydroxyethoxy)benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 24

Dust

| | |
|---|---|
| N-[(4,6-dimethoxypyrimidin-2-yl)aminocarbonyl]-2-(2-cyclopropylmethyloxy)benzenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

## Example 25

Wettable Powder

| | |
|---|---|
| N-[(4-methoxy-6-methyl-1,3,5-triazin-2-yl)aminocarbonyl]-2-(2-hydroxyethoxy)benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

## Example 26

Wettable Powder

| | |
|---|---|
| N-[(4,6-dimethoxy-1,3,5-triazin-2-yl)aminocarbonyl]-2-(2-hydroxyethoxy)benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

## Example 27

Granule

| | |
|---|---|
| Wettable Powder of Example 26 | 5% |
| attapulgite granules (U.S.S. 20-40 mesh; 0.84-0.42 mm) | 95% |

A slurry of wettable powder containing ≈25% solids is sprayed on the surface of attapulgite granules in a double-cone blender. The granules are dried and packaged.

Example 28

Extruded Pellet

| | |
|---|---|
| N-[(4-methoxy-6-methylpyrimidin-2-yl)aminocarbonyl]-2-(2-methylsulfonyloxyethoxy)benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 29

Oil Suspension

| | |
|---|---|
| N-[(4,6-dimethoxypyrimidin-2-yl)aminocarbonyl]-2-(2-hydroxyethoxy)benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 30

Wettable Powder

| | |
|---|---|
| N-[(4-methoxy-6-methylpyrimidin-2-yl)aminocarbonyl]-2-(2-hydroxyethoxy)benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 31

Low Strength Granule

| | |
|---|---|
| N-[(4,6-dimethylpyrimidin-2-yl)aminocarbonyl]-2-(2-hydroxyethoxy)benzenesulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 32

Aqueous Suspension

| | |
|---|---|
| N-[(4,6-dimethoxypyrimidin-2-yl)aminocarbonyl]-2-(2-hydroxyethoxy)benzenesulfonamide, ammonium salt | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 33

Solution

| | |
|---|---|
| N-[(4-methoxy-6-methylpyrimidin-2-yl)aminocarbonyl]-2-(2-hydroxyethoxy)benzenesulfonamide, ammonium salt | 5% |
| water | 95% |

The salt is added directly to the water with stirring to produce the solution, which may then be packaged for use.

Example 34

Low Strength Granule

| | |
|---|---|
| N-[(4,6-dimethoxy-1,3,5-triazin-2-yl)aminocarbonyl]-2-(2-hydroxyethoxy)benzenesulfonamide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 35

Granule

N-[(4-methoxy-6-methyl-1,3,5-triazin-2-yl)aminocarbonyl]-2-(2-hydroxyethoxy)benzenesulfonamide 80%

| | |
|---|---|
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 36

High Strength Concentrate

N-[(4-methoxy-6-methylpyrimidin-2-yl)aminocarbonyl]-2-(2-methylsulfonyloxyethoxy)benzenesulfonamide 99%

| | |
|---|---|
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 37

Wettable Powder

| | |
|---|---|
| N-[(4,6-dimethylpyrimidin-2-yl)aminocarbonyl]-2-(2-hydroxyethoxy)benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 38

Wettable Powder

| | |
|---|---|
| N-[(4-methoxy-6-methylpyrimidin-2-yl)aminocarbonyl]-2-(2-methylsulfonyloxyethoxy)benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 39

Oil Suspension

| | |
|---|---|
| N-[(4,6-dimethylpyrimidin-2-yl)aminocarbonyl]-2-(2-hydroxyethoxy)benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 40

Dust

| | |
|---|---|
| N-[(4-methoxy-6-methylpyrimidin-2-yl)aminocarbonyl]-2-(2-hydroxyethoxy)benzenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

The compounds of the present invention are highly active herbicides, which are specifically useful for pre-emergence weed control in wheat and post-emergence weed control in wheat, corn, sugar beets and rice. They are also useful as pre- and/or post-emergence herbicides in other crops and as general, broad-spectrum herbicides where complete control of all vegetation is desired, such as around fuel storage tanks, ammunition depots, industrial storage areas, drive-in theaters, around billboards, highway, and railroad structures.

The rates of application for the compounds of the invention are determined by a number of factors depending upon the particular end result desired. These factors include: use as selective or general herbicides, crop species involved, types of weeds to be controlled, weather and climate, formulations selected, mode of application, amount of foliage present, etc. In general terms, the subject compounds should be applied at levels of around 0.001 to 5 kg/ha with a preferred range of 0.004 to 1 kg/ha. The lower rates are suggested for use as selective post-emergence herbicides in wheat, rice, sugar beets, corn and other crops. Intermediate rates are suggested for use as pre-emergence herbicides on lighter soils and/or those having a low organic matter content. The highest rates are suggested for pre-emergence weed control on heavy soils and for general, broad-spectrum weed control where control of all vegetation is desired.

The compounds of this invention may be used in combination with other commercial herbicides. They are particularly useful in combination with the following herbicides:

| Common Name | Chemical Name |
|---|---|
| acifluorfen | 5-[2-chloro-4-(trifluoromethyl)phenoxy]-2-nitrobenzoic acid |
| acrolein | acrolein |
| alachlor | 2-chloro-2',6'-diethyl-N-(methoxymethyl)-acetanilide |
| ametryn | 2-(ethylamino)-4-(isopropylamino)-6-methylthio)-s-triazine |
| amitrole | 3-amino-s-triazole |
| AMS | ammonium sulfamate |
| asulam | methyl sulfanilylcarbamate |
| atrazine | 2-chloro-4-(ethylamino)-6-(isopropyl-amino)-s-triazine |
| barban | 4-chloro-2-butynyl m-chlorocarbanilate |
| benefin | N-butyl-N-ethyl-α,α,α-trifluoro-2,6-dinitro-p-toluidine |
| bensulide | O,O-diisopropyl phosphorodithioate S-ester with N-(2-mercaptoethyl)benzene-sulfonamide |
| benzipram | 3,5-dimethyl-N-(1-methyethyl)-N-(phenyl-methyl)benzamide |
| benzoylprop | N-benzoyl-N-(3,4-dichlorophenyl)-DL-alaine |
| bifenox | methyl 5-(2,4-dichlorophenoxy)-2-nitroben-zoate |
| bromacil | 5-bromo-3-sec-butyl-6-methyluracil |
| bromoxynil | 3,5-dibromo-4-hydroxybenzonitrile |
| butachlor | N-(butoxymethyl)-2-chloro-2',6'-diethyl-acetanilide |
| butam | 2,2-dimethyl-N-(1-methylethyl)-N-(phenyl-methyl)propanamide |
| buthidazole | 3-[5-(1,1-dimethylethyl)-1,3,4-thiadiazol-2-yl]-4-hydroxy-1-methyl-2-imidazoli-dinone |

| Common Name | Chemical Name |
| --- | --- |
| butralin | 4-(1;1-dimethylethyl)-N-(1-methylpropyl)-2,6-dinitrobenzenamine |
| cacodylic acid | hydroxydimethylarsine oxide |
| carbetamide | D-N-ethyllactamide carbanilate (ester) |
| CDAA | N-N-diallyl-2-chloroacetamide |
| CDEC | 2-chloroallyl diethyldithiocarbamate |
| chloramben | 3-amino-2,5-dichlorobenzoic acid |
| chlorbromuron | 3-(4-bromo-3-chlorophenyl)-1-methoxy-1-methylurea |
| chloroxuron | 3-[p-(p-chlorophenoxy)phenyl]-1,1-dimethylurea |
| chlorpropham | ispropyl m-chlorocarbanilate |
| cisanilide | cis-2,5-dimethyl-N-phenyl-1-pyrrolidinecarboxamide |
| CMA | calcium methanearsonate |
| cyanazine | 2-[[4-chloro-6-(ethylamino)-s-triazin-2-yl]amino]-2-methylpropionitrile |
| cycloate | S-ethyl N-ethylthiocyclohexanecarbamate |
| cycluron | 3-cyclooctyl-1,1-dimethylurea |
| cyperquat | 1-methyl-4-phenylpyridinium |
| cyprazine | 2-chloro-4-(cyclopropylamino)-6-(isopropylamino)-s-triazine |
| cyprazole | N-[5-(2-chloro-1,1-dimethylethyl)-1,3,4-thiadiazol-2-yl]cyclopropanecarboxamide |
| cypromid | 3',4'-dichlorocyclopropanecarboxanilide |
| dalapon | 2,2-dichloropropionic acid |
| dazomet | tetrahydro-3,5-dimethyl-2H-1,3,5-thiadiazine-2-thione |
| DCPA | dimethyl tetrachloroterephthalate |
| desmetryn | 2-(isopropylamino)-4-(methylamino)-6-methylthio)-s-triazine |
| diallate | S-(2,3-dichloroallyl)diisopropylthiocarbamate |
| dicamba | 3,6-dichloro-o-anisic acid |

| Common Name | Chemical Name |
|---|---|
| dichlobenil | 2,6-dichlorobenzonitrile |
| dichlorprop | 2-(2,4-dichlorophenoxy)propionic acid |
| diclofop | 2-[4-(2,4-dichlorophenoxy)phenoxy]propanoic acid |
| diethatyl | N-(chloroacetyl)-N-(2,6-diethylphenyl)-glycine |
| difenzoquat | 1,2-dimethyl-3,5-diphenyl-1H-pyrazolium |
| dinitramine | $N^4$,$N^4$-diethyl-α,α,α-trifluoro-3,5-dinitro-toluene-2,4-diamine |
| dinoseb | 2-sec-butyl-4,6-dinitrophenyl |
| diphenamid | N,N-dimethyl-2,2-diphenylacetamide |
| dipropetryn | 2-(ethylthio)-4,6-bis(isopropylamino)-s-triazine |
| diquat | 6,7-dihydrodipyrido[1,2-α:2',1'-c]pyrazinediium ion |
| diuron | 3-(3,4-dichlorophenyl)-1,1-dimethylurea |
| DSMA | disodium methanearsonate |
| endothall | 7-oxabicyclo[2.2.1]heptane-2,3-dicarboxylic acid |
| erbon | 2-(2,4,5-trichlorophenoxy)ethyl 2,2-dichloropropionate |
| ethafluralin | N-ethyl-N-(2-methyl-2-propenyl)-2,6-dinitro-4-(trifluoromethyl)benzenamine |
| ethofumesate | (±)-2-ethoxy-2,3-dihydro-3,3-dimethyl-5-benzofuranyl methanesulfonate |
| fenac | (2,3,6-trichlorophenyl)acetic acid |
| fenuron | 1,1-dimethyl-3-phenylurea |
| fenuron TCA | 1,1-dimethyl-3-phenylurea mono(trichloroacetate) |
| flamprop | N-benzoyl-N-(3-chloro-4-fluorophenyl)-DL-alanine |
| fluchloralin | N-(2-chloroethyl)-2,6-dinitro-N-propyl-4-(trifluoromethyl)aniline |
| fluometuron | 1,1-dimethyl-3-(α,α,α-trifluoro-m-tolyl)-urea |

| Common Name | Chemical Name |
|---|---|
| fluorodifen | *p*-nitrophenyl α,α,α-trifluoro-2-nitro-*p*-tolyl ether |
| fluridone | 1-methyl-3-phenyl-5-[3-(trifluoromethyl)-phenyl]-4(1H)-pyridinone |
| fosamine | ethyl hydrogen (aminocarbonyl)phosphonate |
| glyphosate | N-(phosphonomethyl)glycine |
| hexaflurate | potassium hexafluoroarsenate |
| hexazinone | 3-cyclohexyl-6-(dimethylamino)-1-methyl-1,3,5-triazin-2,4(1H,3H)-dione |
| ioxynil | 4-hydroxy-3,5-diiodobenzonitrile |
| isopropalin | 2,6-dinitro-N,N-dipropylcumidine |
| karbutilate | *tert*-butylcarbamic acid ester with 3(*m*-hydroxyphenyl)-1,1-dimethylurea |
| lenacil | 3-cyclohexyl-6,7-dihydro-1H-cyclopentapyrimidine-2,4(3H,5H)-dione |
| linuron | 3-(3,4-dichlorophenyl)-1-methoxy-1-methylurea |
| MAA | methanearsonic acid |
| MAMA | monoammonium methanearsonate |
| MCPA | [(4-chloro-*o*-tolyl)oxy]acetic acid |
| MCPB | 4-[(4-chloro-*o*-tolyl)oxy]butyric acid |
| mecoprop | 2-[(4-chloro-*o*-tolyl)oxy]propionic acid |
| mefluidide | N-[(2,4-dimethyl-5-[[(trifluoromethyl)sulfonyl]amino]phenyl]acetamide |
| methalpropalin | N-(2-methyl-2-propenyl)-2,6-dinitro-N-propyl-4-(trifluoromethyl)benzenamide |
| metham | sodium methyldithiocarbamate |
| methazole | 2-(3,4-dichlorophenyl)-4-methyl-1,2,4-oxadiazolidine-3,5-dione |
| metolachlor | 2-chloro-N-(2-ethyl-6-methylphenyl)-N-(2-methoxy-1-methylethyl)acetamide |
| metribuzin | 4-amino-6-*tert*-butyl-3-(methylthio-as-triazin-5(4H)-one |
| molinate | S-ethyl hexahydro-1H-azepine-1-carbothioate |

| Common Name | Chemical Name |
|---|---|
| monolinuron | 3-(p-chlorophenyl)-1-methoxy-1-methylurea |
| monuron | 3-(p-chlorophenyl)-1,1-dimethylurea |
| monuron TCA | 3-(p-chlorophenyl)-1,1-dimethylurea mono-(trichloroacetate) |
| MSMA | monosodium methanearsonate |
| napropamide | 2-(α-naphthoxy-N,N-diethylpropionamide |
| naptalam | N-1-naphthylphthalamic acid |
| neburon | 1-butyl-3-(3,4-dichlorophenyl)-1-methyl-urea |
| nitralin | 4-(methylsulfonyl)-2,6-dinitro-N,N-dipropylaniline |
| nitrofen | 2,4-dichlorophenyl p-nitrophenyl ether |
| nitrofluorfen | 2-chloro-1-(4-nitrophenoxy)-4-(trifluoro-methyl)benzene |
| norea | 3-(hexahydro-4,7-methanoindan-5-yl)-1,1-dimethylurea |
| norflurazon | 4-chloro-5-(methylamino)-2-(α,α,α-trifluoro-m-tolyl-3(2H)-pyridazinone |
| oryzalin | 3,5-dinitro-$N^4$,$N^4$-dipropylsulfanilamide |
| oxadiazon | 2-tert-butyl-4-(2,4-dichloro-5-isopropoxy-phenyl)$\Delta^2$-1,3,4-oxadiazolin-5-one |
| oxyfluorfen | 2-chloro-1-(3-ethoxy-4-nitrophenoxy)-4-(trifluoromethyl)benzene |
| paraquat | 1,1'-dimethyl-4,4'-bipyridinium ion |
| PBA | chlorinated benzoic acid |
| pendimethalin | N-(1-ethylpropyl)-3,4-dimethyl-2,6-dinitrobenzenamine |
| perfluidone | 1,1,1-trifluoro-N-[2-methyl-4-(phenylsulfonyl)phenyl]methanesulfonamide |
| picloram | 4-amino-3,5,6-trichloropicolinic acid |
| procyazine | 2-[[4-chloro-6-(cyclopropylamino)-1,3,5-triazine-2-yl]amino]-2-methylpropane-nitrile |
| profluralin | N-(cyclopropylmethyl)-α,α,α-trifluoro-2,6-dinitro-N-propyl-p-toluidine |

| Common Name | Chemical Name |
|---|---|
| prometon | 2,4-bis(isopropylamino)-6-methoxy-*s*-triazine |
| prometryn | 2,4-bis(isopropylamino)-6-methylthio)-*s*-triazine |
| pronamide | 3,5-dichloro(N-1,1-dimethyl-2-propynyl)-benzamide |
| propachlor | 2-chloro-N-isopropylacetanilide |
| propanil | 3',4'-dichloropropionalide |
| propazine | 2-chloro-4,6-bis(isopropylamino)-*s*-triazine |
| propham | isopropyl carbanilate |
| prosulfalin | N-[[4-(dipropylamino)-3,5-dinitrophenyl]-sulfonyl]-S,S-dimethylsulfilimine |
| prynachlor | 2-chloro-N-(1-methyl-2-propynyl)acet-anilide |
| secbumeton | N-ethyl-6-methoxy-N'(1-methylpropyl)-1,3,5-triazine-2,4-diamine |
| siduron | 1-(2-methylcyclohexyl)-3-phenylurea |
| simazine | 2-chloro-4,6-bis(ethylamino)-*s*-triazine |
| simetryn | 2,4-bis(ethylamino)-6-(methylthio)-*s*-triazine |
| TCA | trichloroacetic acid |
| tebuthiuron | N-[5-(1,1-dimethylethyl)-1,3,4-thiadiazol-2-yl]-N,N'-dimethylurea |
| terbacil | 3-*tert*-butyl-5-chloro-6-methyluracil |
| terbuchlor | N-(butoxymethyl)-2-chloro-N-[2-(1,1-dimethylethyl)-6-methylphenyl]acetamide |
| terbuthylazine | 2-(*tert*-butylamino)-4-chloro-6-(ethyl-amino)-*s*-triazine |
| terbutol | 2,6-di-*tert*-butyl-*p*-tolyl methylcarbamate |
| terbutryn | 2-(*tert*-butylamino)-4-(ethylamino)-6-(methylthio)-*s*-triazine |
| tetrafluron | N,N-dimethyl-N'-[3-(1,1,2,2-tetrafluoro-ethoxy)phenyl]urea |

| Common Name | Chemical Name |
|---|---|
| thiobencarb | S-[(4-chlorophenyl)methyl] diethylcarbamothioate |
| triallate | S-(2,3,3-trichloroallyl)diisopropylthiocarbamate |
| trifluralin | α,α,α-trifluoro-2,6-dinitro-N,N-dipropyl-p-toluidine |
| trimeturon | 1-(p-chlorophenyl)-2,3,3-trimethylpseudourea |
| 2,3,6-TBA[b] | 2,3,6-trichlorobenzoic acid |
| 2,4-D | (2,4-dichlorophenoxy)acetic acid |
| 2,4-DB | 4-(2,4-dichlorophenoxy)butyric acid |
| 2,4-DEP | tris[2-(2,4-dichlorophenoxy)ethyl]phosphite |
| methabenzthiazuron | 1,3-dimethyl-3-(2-benzothiazolyl)urea |
| chlortoluran | N'-(3-chloro-4-methylphenyl)-N'N-dimethylurea |
| isoproturan | N-(4-isopropylphenyl)-N'N'-dimethylurea |
| metoxuran | N'-(3-chloro-4-methoxyphenyl)-N,N-dimethylurea |

The selective herbicidal properties of the subject compounds which indicate utility as pre-emergence herbicides in wheat and post-emergence herbicides in wheat, rice, sugar beets and corn were discovered in a number of greenhouse tests. The test procedures and results follow.

Some of the test results presented in Table A indicate that several compounds showed little or no activity at the tested rates. It is believed that these compounds would be active if tested at higher rates.

0094790



Table XI

Compound 1

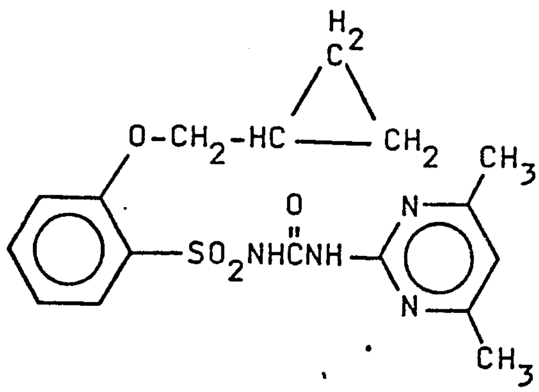

Compound 2

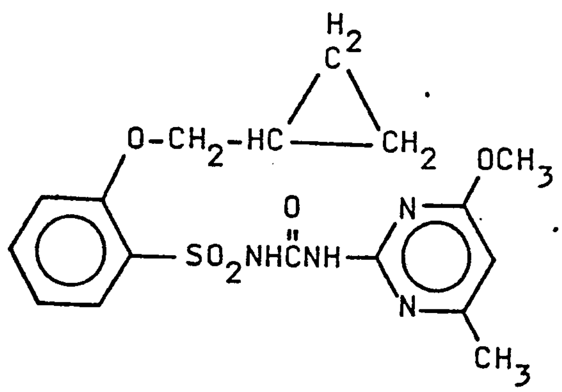

Compound 3

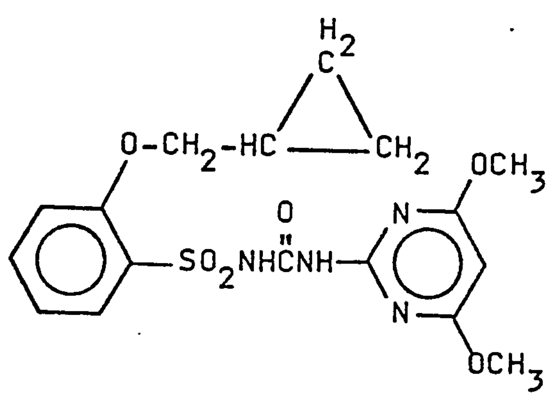

Table XI (continued)

Compound 4

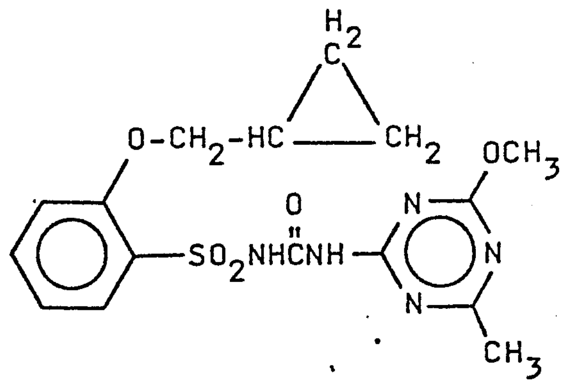

Compound 5

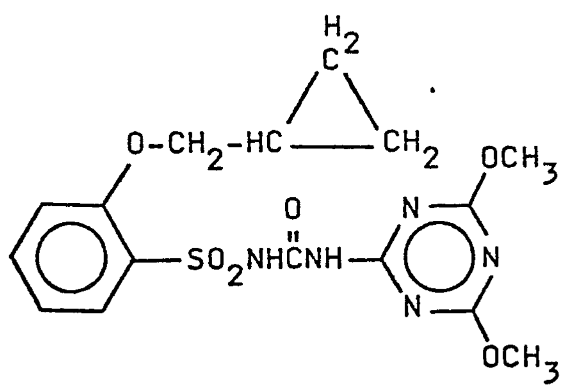

Compound 6

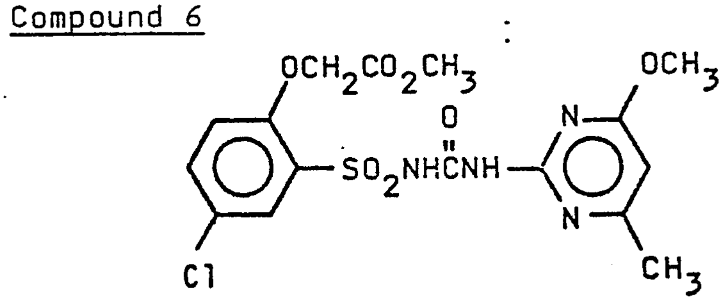

Table XI (continued)

Compound 7

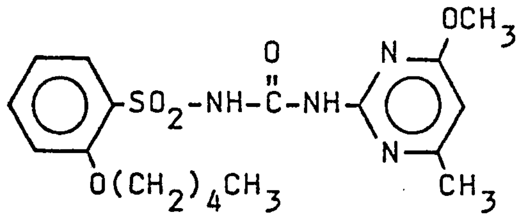

Compound 8

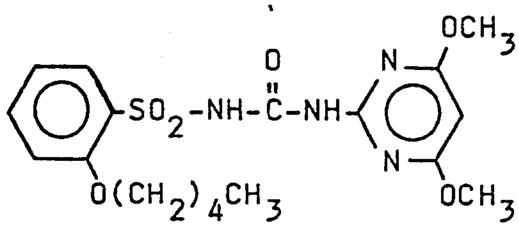

Compound 9

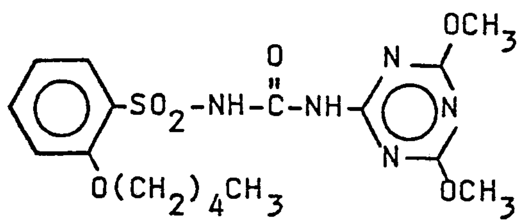

Compound 10

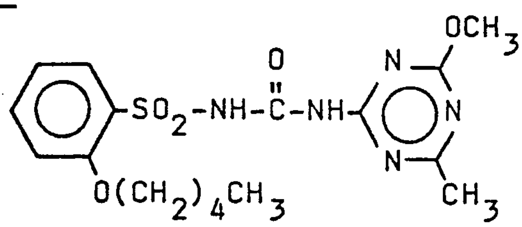

Table XI (continued)

Compound 11

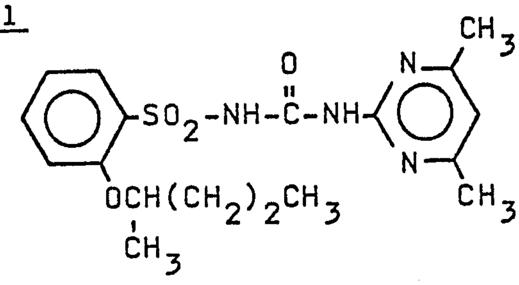

Compound 12

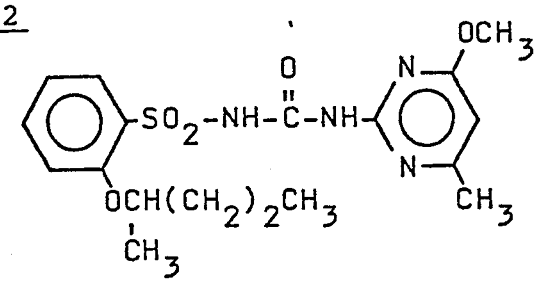

Compound 13

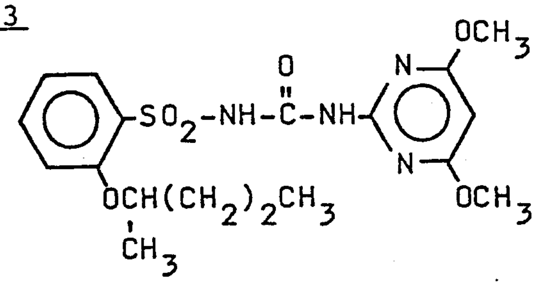

Compound 14

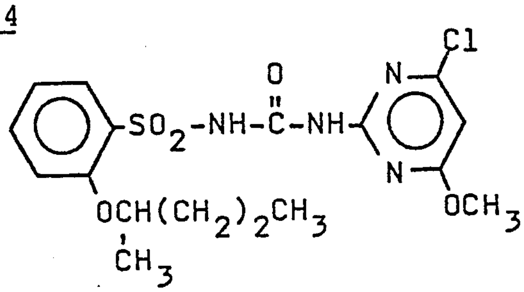

Table XI (continued)

Compound 15

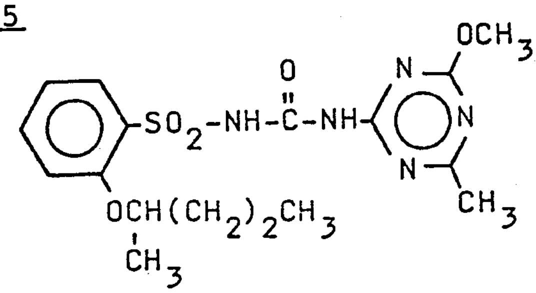

Compound 16

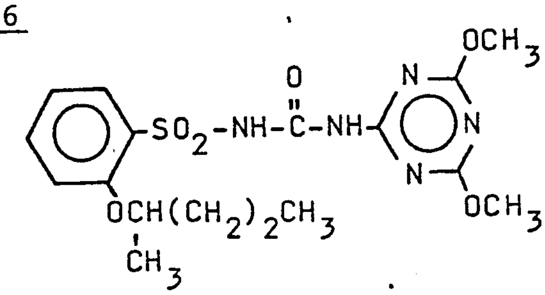

Compound 17

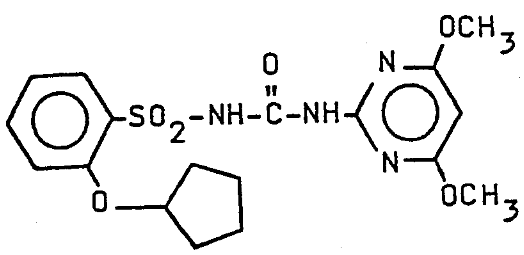

Compound 18

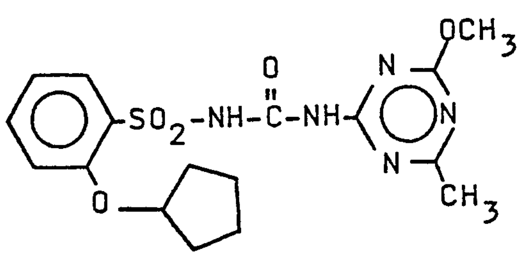

0094790

Table XI (continued)

Compound 19

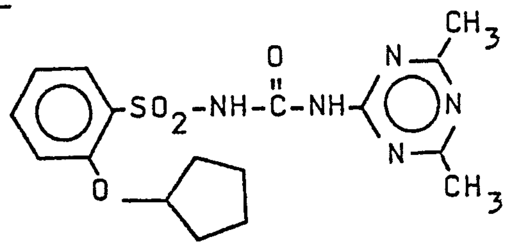

Compound 20

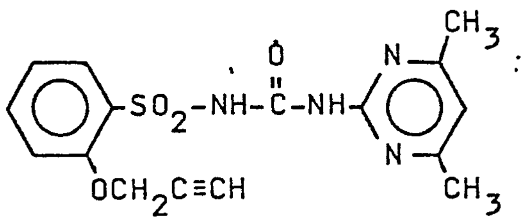

Compound 21

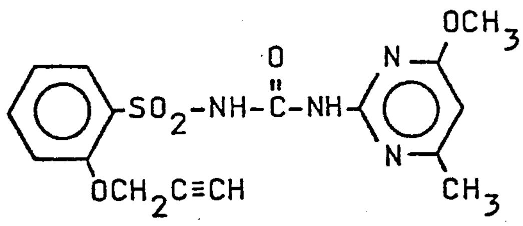

Compound 22

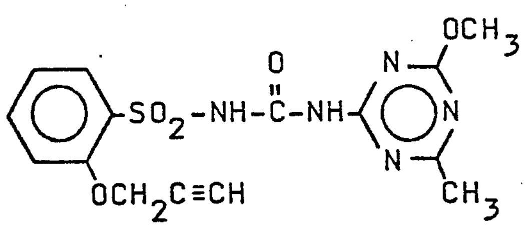

Compound 23

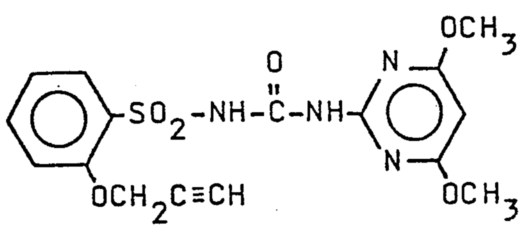

Table XI (continued)

Compound 24

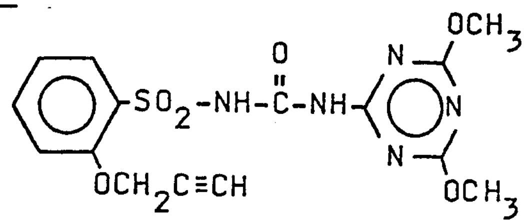

Compound 25

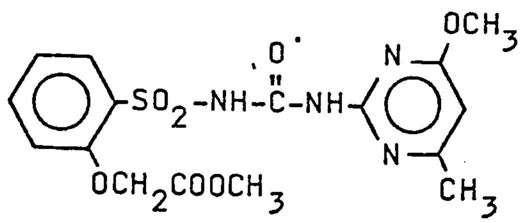

Compound 26

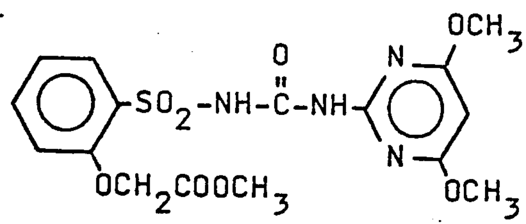

Compound 27

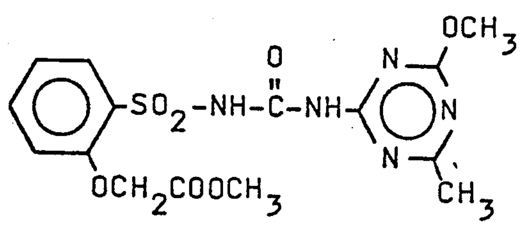

Compound 28

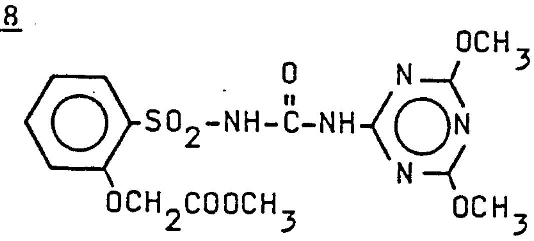

Table XI (continued)

Compound 29

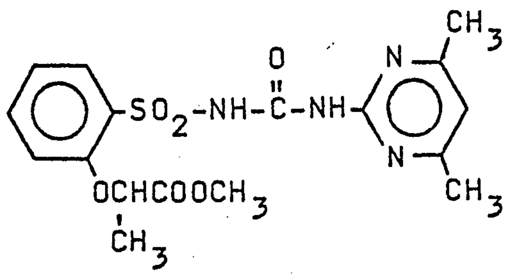

Compound 30

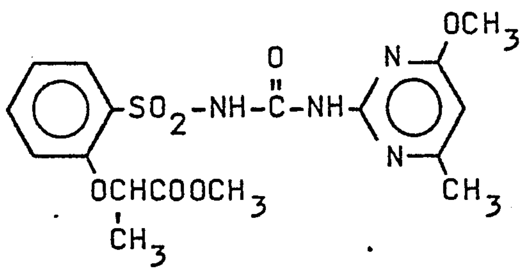

Compound 31

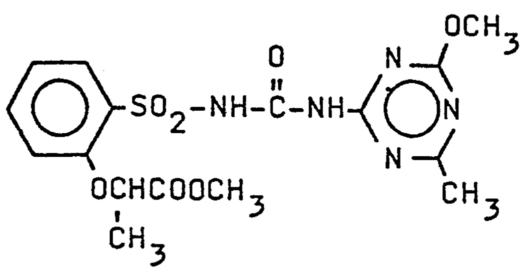

Compound 32

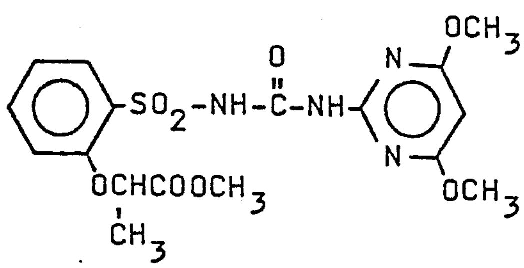

Table XI (continued)

Compound 33

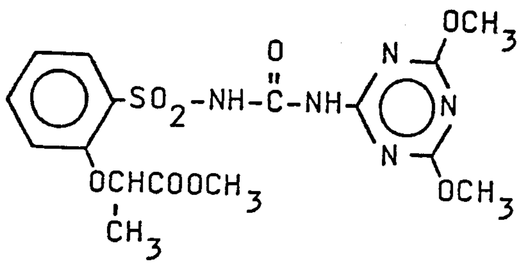

Compound 34

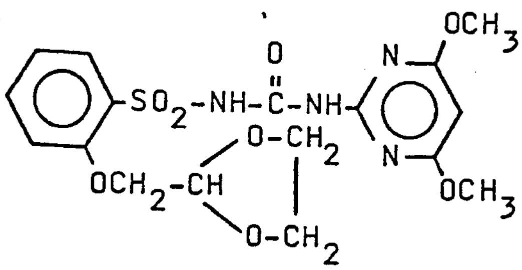

Compound 35

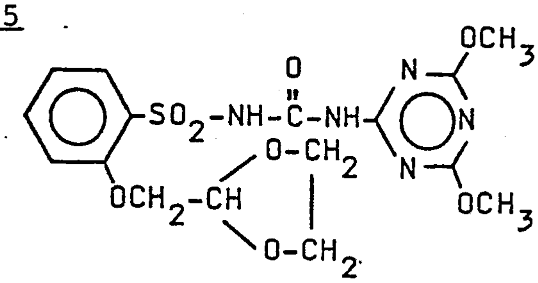

Compound 36

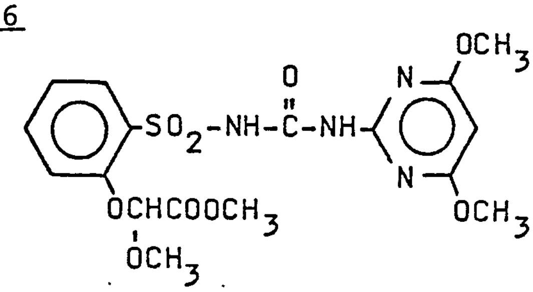

Table XI (continued)

Compound 37

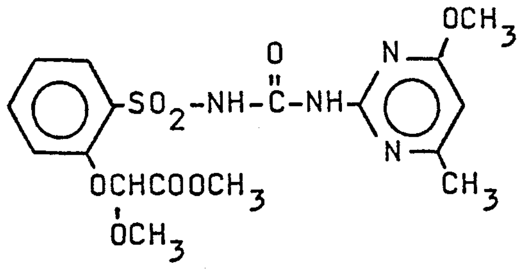

Compound 38

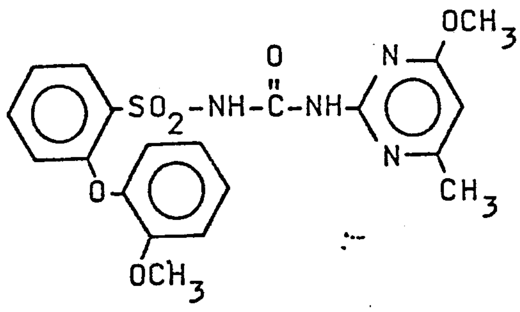

Compound 39

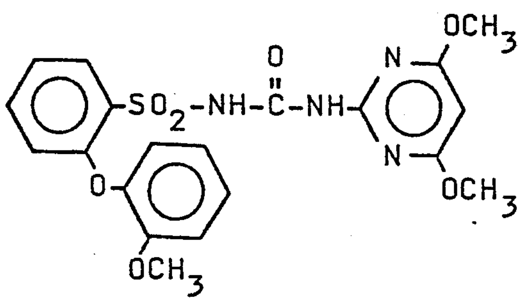

Compound 40

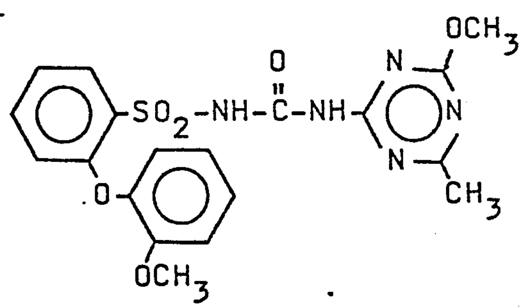

Table XI (continued)

Compound 41

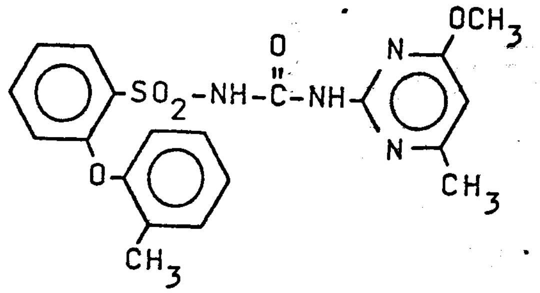

Compound 42

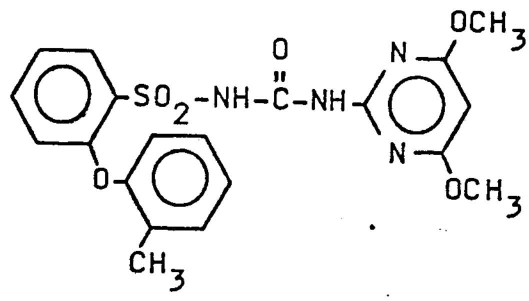

Compound 43

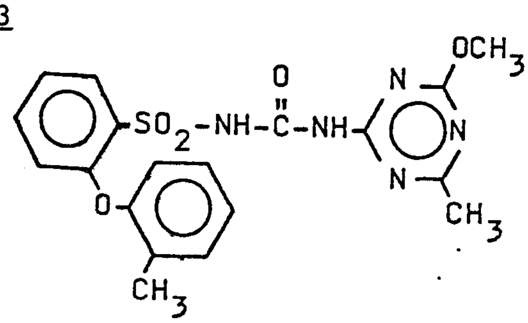

Compound 44

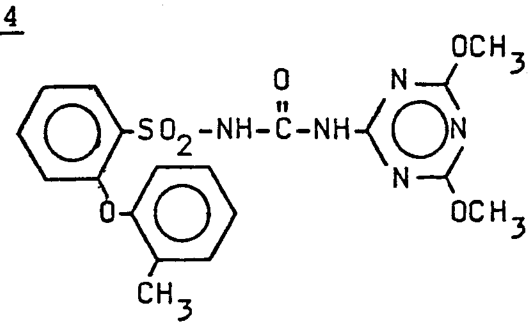

Table XI (continued)

Compound 45

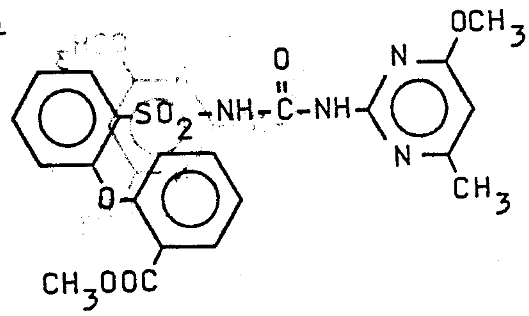

Compound 46

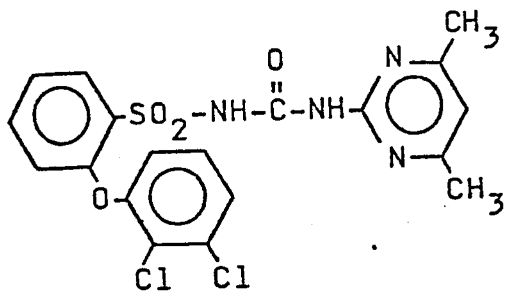

Compound 47

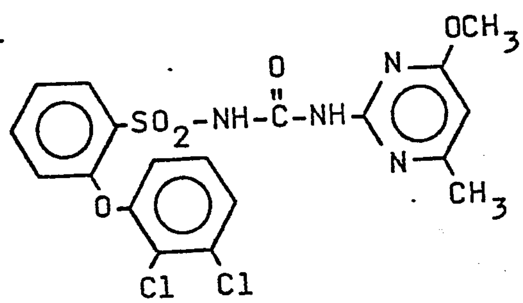

Compound 48

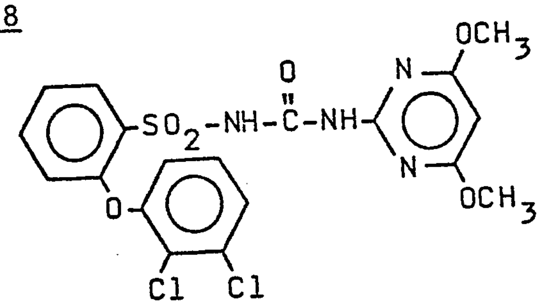

Table XI (continued)

Compound 49

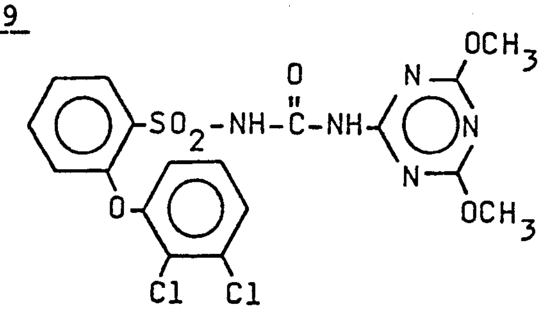

Compound 50

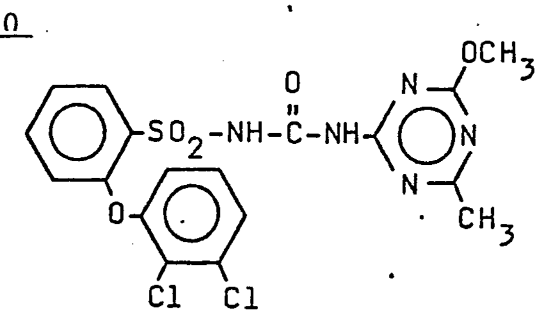

Compound 51

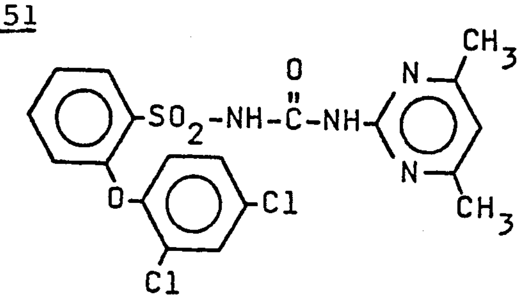

Compound 52

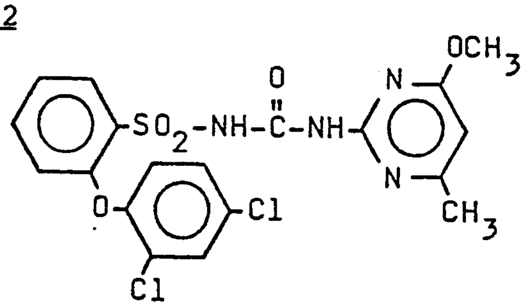

Table XI (continued)

Compound 53

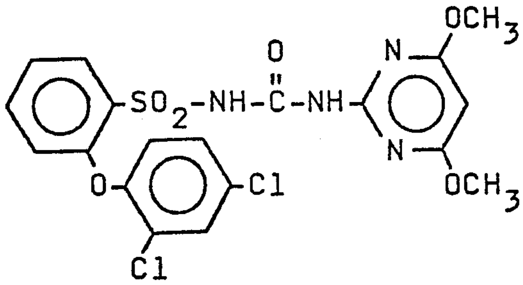

Compound 54

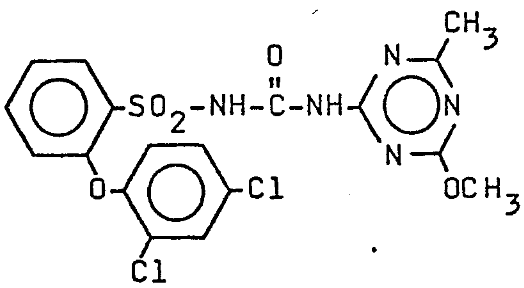

Compound 55

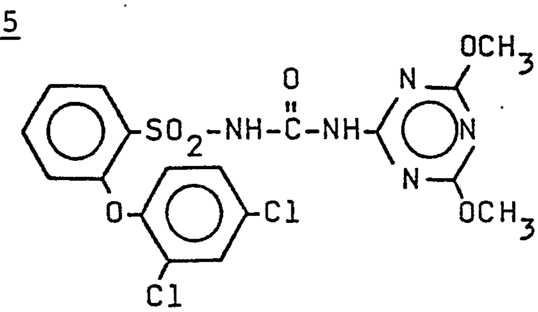

Compound 56

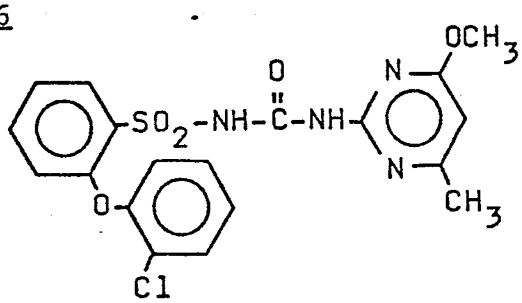

Table XI (continued)

Compound 57

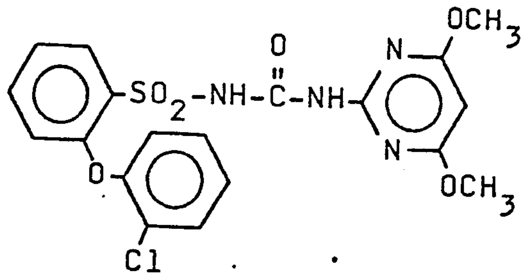

Compound 58

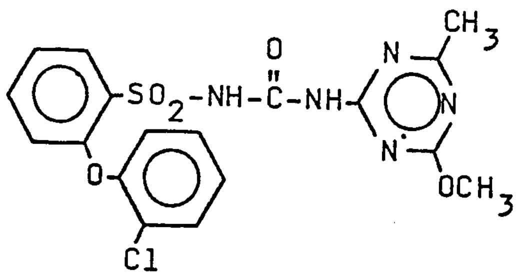

Compound 59

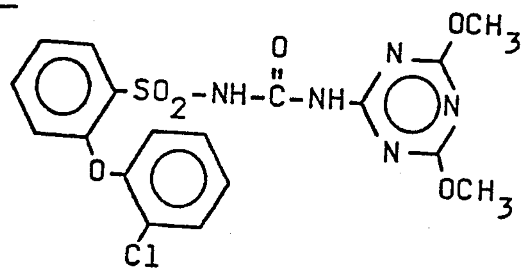

Table XI (continued)

Compound 60

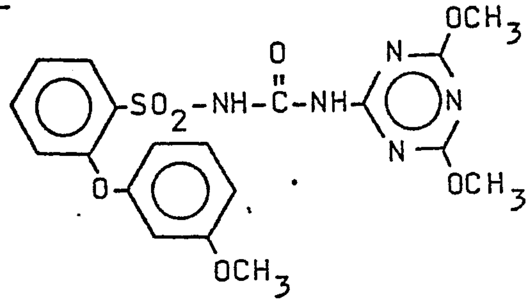

Compound 61

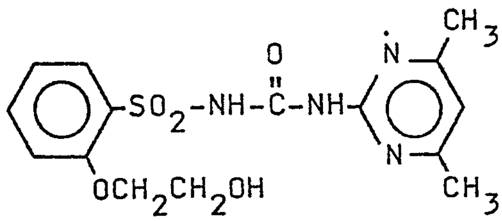

Compound 62

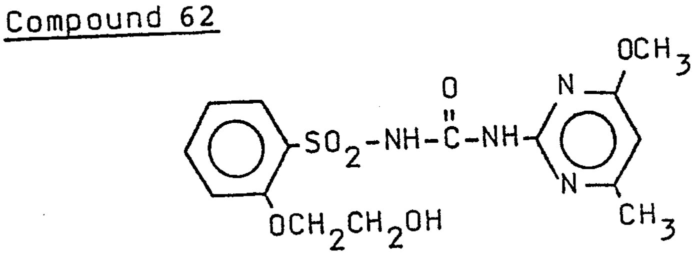

Table XI (continued)

Compound 63

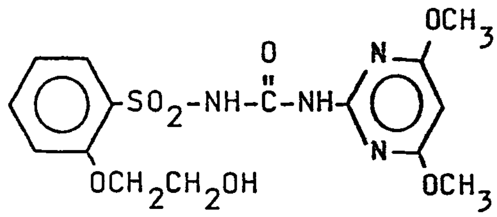

Compound 64

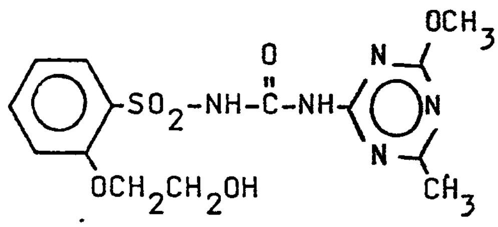

Compound 65

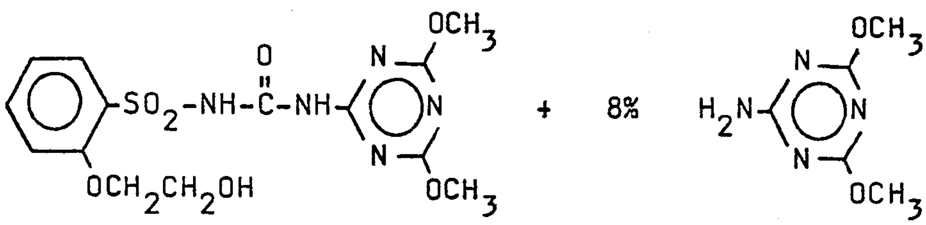

Compound 66

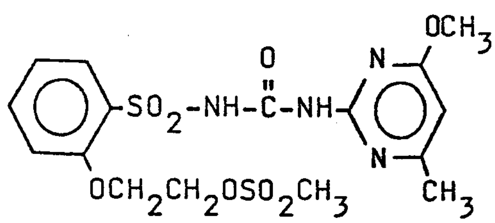

Test A

Seeds of crabgrass (*Digitaria* sp.), barnyardgrass (*Echinochloa crusgalli*), wild oats (*Avena fatua*), sicklepod (*Cassia obtusifolia*), morningglory (*Ipomoea* sp.), cocklebur (*Xanthium* sp.), sorghum, corn, soybean, rice, wheat and nutsedge tubers (*Cyperus rotundus*) were planted in a growth medium and treated pre-emergence with a non-phytotoxic solvent solution of the compounds of Table IX. At the same time, cotton having five leaves (including cotyledonary ones), bush beans with the third trifoliate leaf expanding, crabgrass and barnyardgrass with two leaves, wild oats with two leaves, sicklepod with three leaves (including cotyledonary ones), morningglory and cocklebur with four leaves (including the cotyledonary ones), sorghum with four leaves, corn with four leaves, soybean with two cotyledonary leaves, rice with three leaves, wheat with one leaf, and nutsedge with three to five leaves were sprayed with a non-phytotoxic solvent solution of the compounds of Table XI. Other containers of the above mentioned weeds and crops were treated pre- or post-emergence with the same non-phytotoxic solvent so as to provide a solvent control. A set of untreated control plants containing all the above named plants was also included for comparison. Pre-emergence and post-emergence treated plants and controls were maintained in a greenhouse for sixteen days, then all treated plants were compared with their respective controls and rated visually for response to treatment.

The following rating system was used:

0 = no effect;
10 = maximum effect;
C = chlorosis or necrosis;
D = defoliation;
E = emergence inhibition;
G = growth retardation;
H = formative effects;
U = unusual pigmentation;
X = axillary stimulation;
6F = delayed flowering; and
6Y = abscised buds or flowers.

Table A

| | Compound Nos. | | | |
|---|---|---|---|---|
| | 1 | 1 | 2 | 3 |
| Rate kg/ha | 0.4 | .05 | .05 | .05 |
| POST-EMERGENCE | | | | |
| Bush bean | 5C,9G,6Y | 5C,9G,6Y | 9C | 6C,9G,6Y |
| Cotton | 4C,9G | 4C,9G | 5C,9G | 4C,9G |
| Morningglory | 3C,8G | 1C,3G | 2C,5G | 3C,7G |
| Cocklebur | 4C,9G | 3C,9H | 3C,5H | 4C,9H |
| Sicklepod | 3C,9G | 2C | 2C | 3C,5G |
| Nutsedge | 3C,9G | 2C,8G | 3C,7G | 4C,9G |
| Crabgrass | 2C,7G | 2G | 2C,3G | 1C |
| Barnyardgrass | 3C,9H | 3C,8H | 3C,9H | 4C,9H |
| Wild Oats | 2C,8G | 0 | 2C,4G | 2G |
| Wheat | 2C,8G | 0 | 2C,7G | 1C,4G |
| Corn | 2C,9H | 2C,7H | 9H | 1C,4G |
| Soybean | 2C,9H | 3C,8H | 4C,9G | 3C,9G |
| Rice | 4C,9G | 9G | 3C,7G | 2C,7G |
| Sorghum | 3C,9G | 2C,9H | 3C,9H | 9G |
| PRE-EMERGENCE | | | | |
| Morningglory | 9G | 7G | 7G | 8G |
| Cocklebur | 9H | 9H | 9C | 1C,9H |
| Sicklepod | 9G | 2C,5G | 2C,8G | 8G |
| Nutsedge | 10E | 4G | 9G | 10E |
| Crabgrass | 1C,3G | 0 | 2C,5G | 2C,7G |
| Barnyardgrass | 5C,9H | 5C,7H | 4C,9H | 3C,9H |
| Wild Oats | 4C,9G | 2C,8G | 9G | 3C,7G |
| Wheat | 3C,9G | 1C,7G | 2C,9G | 8G |
| Corn | 9G | 3C,8H | 3C,9H | 4C,8G |
| Soybean | 9H | 1C,1H | 3C,8H | 4C,8H |
| Rice | 3C,9H | 2C,5G | 3C,9H | 5C,9H |
| Sorghum | 3C,9H | 2C,9H | 2C,9H | 2C,9H |

Table A (continued)

| | Compound Nos. | | |
|---|---|---|---|
| | 4 | 5 | 6 |
| Rate kg/ha | .05 | 0.4 | 2.0 |
| POST-EMERGENCE | | | |
| Bush bean | 9C | 9C | 2C,1H |
| Cotton | 5C,9G | 9C | 1C,1H |
| Morningglory | 5C,9G | 9C | 5G |
| Cocklebur | 9C | 10C | 1H |
| Sicklepod | 3C,8H | 9C | 2H |
| Nutsedge | 0 | 9C | 0 |
| Crabgrass | 0 | 1C,2H | 0 |
| Barnyardgrass | 2H | 5C,9H | 0 |
| Wild Oats | 2G | 2C,6G | 0 |
| Wheat | 0 | 0 | 0 |
| Corn | 1C,5G | 2C,8H | 0 |
| Soybean | 9C | 5C,9G | 1C |
| Rice | 1C,5H | 5C,9G | 0 |
| Sorghum | 2C,9H | 2U,9H | 0 |
| PRE-EMERGENCE | | | |
| Morningglory | 9H | 9G | 0 |
| Cocklebur | 9H | 2C,9H | 4G |
| Sicklepod | 9G | 2C,9G | 5G |
| Nutsedge | 5G | 10E | 0 |
| Crabgrass | 0 | 2C | 0 |
| Barnyardgrass | 4C,4H | 3C,9H | 2C |
| Wild Oats | 1C,3G | 2C,7G | 0 |
| Wheat | 0 | 2G | 0 |
| Corn | 3C,7G | 3C,9H | 2G |
| Soybean | 9H | 9H | 0 |
| Rice | 3C,7G | 2C,9H | 4G |
| Sorghum | 2C,8H | 9G | 2G |

Table A (continued)

| | Cmpd. 7 | Cmpd. 8 | Cmpd. 9 |
|---|---|---|---|
| Rate kg/ha | 0.05 | 0.05 | 0.05 |
| POST-EMERGENCE | | | |
| Bush bean | 0 | 1C,1H | 4C,8H,6Y |
| Cotton | 3G | 1C,3G | 4C,9G |
| Morningglory | 2C,6G | 1C | 4C,9G |
| Cocklebur | 1H | 1C | 5C,9G |
| Sicklepod | 1H | 0 | 2C,2H |
| Nutsedge | 0 | 0 | 2G |
| Crabgrass | 0 | 0 | 0 |
| Barnyardgrass | 6H | 3H | 0 |
| Wild Oats | 0 | 0 | 0 |
| Wheat | 0 | 0 | 0 |
| Corn | 1C | 0 | 0 |
| Soybean | 1C,3H | 2C,4G | 3C,9G |
| Rice | 2C,2G | 2C,3G | 0 |
| Sorghum | 2C,8H | 2C,3G | 2C |
| Sugar beet | 4G | 2C | 3C,8G |
| PRE-EMERGENCE | | | |
| Morningglory | 2C,2G | 2C,3G | 2C |
| Cocklebur | 0 | 5G | 9H |
| Sicklepod | 2C | 2C | 4G |
| Nutsedge | 2C,3G | 0 | 0 |
| Crabgrass | 2C | 1C | 0 |
| Barnyardgrass | 5C,7G | 3C,6G | 2C |
| Wild Oats | 2C,6G | 2C,3G | 0 |
| Wheat | 4G | 2G | 0 |
| Corn | 3C,9G | 3C,6G | 0 |
| Soybean | 2C,3G | 0 | 1C |
| Rice | 4C,7G | 3C,5G | 0 |
| Sorghum | 2C,8G | 2C,8G | 2C,6G |
| Sugar beet | 8G | 8G | 2C,9G |

Table A (continued)

| | Cmpd. 10 | Cmpd. 11 | Cmpd. 12 | Cmpd. 13 |
|---|---|---|---|---|
| Rate kg/ha | 0.05 | 0.05 | 0.05 | 0.05 |
| POST-EMERGENCE | | | | |
| Bush bean | 4C,4H,6Y | 0 | 1C,1H | 5C,8G,6Y |
| Cotton | 1C | 2C | 2C | 2C,3H |
| Morningglory | 0 | 1C | 2C | 1C |
| Cocklebur | 0 | 0 | 2C,4G | 2C,7G |
| Sicklepod | 0 | 0 | 2C | 1C |
| Nutsedge | 0 | 0 | 2C,4G | 2C,8G |
| Crabgrass | 0 | 0 | 3G | 0 |
| Barnyardgrass | 0 | 0 | 3C,8H | 3C,6H |
| Wild Oats | 0 | 0 | 0 | 0 |
| Wheat | 0 | 0 | 0 | 0 |
| Corn | 0 | 0 | 2C,4H | 2C,5G |
| Soybean | 0 | 0 | 3C,6G | 3C,9G |
| Rice | 0 | 0 | 2C,7G | 2C,9G |
| Sorghum | 0 | 0 | 2C,8H | 2C,5H |
| Sugar beet | 0 | 0 | 0 | 2C,2H |
| PRE-EMERGENCE | | | | |
| Morningglory | 2G | 0 | 1C | 0 |
| Cocklebur | 0 | 0 | 2C,3H | - |
| Sicklepod | 4G | 0 | 1G | 0 |
| Nutsedge | 0 | 0 | 5G | 3G |
| Crabgrass | 3G | 2G | 1C | 1H |
| Barnyardgrass | 1C | 3H | 5C,9H | 5C,8G |
| Wild Oats | 0 | 0 | 3C,7G | 2C,5G |
| Wheat | 0 | 0 | 2C,8G | 5G |
| Corn | 1C | 2C | 3C,9H | 4C,7G |
| Soybean | 1C | 1C | 2C | 2C,2H |
| Rice | 0 | 2C | 2C,7H | 3C,7G |
| Sorghum | 2G | 3C | 2C,9H | 2C,8H |
| Sugar beet | 3G | 0 | 8G | 8H |

Table A (continued)

| | Cmpd. 14 | Cmpd. 15 | Cmpd. 16 |
|---|---|---|---|
| Rate kg/ha | 0.05 | 0.05 | 0.05 |
| POST-EMERGENCE | | | |
| Bush bean | 2C,3G | 9C | 9C |
| Cotton | 2C,4G | 4C,8H | 5C,9G |
| Morningglory | 2C | 3C,7H | 4C,8G |
| Cocklebur | 2C,4G | 5C,9G | 4C,9G |
| Sicklepod | 0 | 3C,5G | 2C,2H |
| Nutsedge | 0 | 2C | 4G |
| Crabgrass | 0 | 0 | 0 |
| Barnyardgrass | 0 | 4H | 2H |
| Wild Oats | 0 | 0 | 0 |
| Wheat | 0 | 0 | 0 |
| Corn | 0 | 2C,6G | 2C,8G |
| Soybean | 1C | 5C,9G | 6C,9G |
| Rice | 1C | 1C,3G | 2C,6G |
| Sorghum | 0 | 1C,3H | 3C,9H |
| Sugar beet | 0 | 9C | 9C |
| PRE-EMERGENCE | | | |
| Morningglory | 2C,4G | 1C,5G | 9G |
| Cocklebur | 4H | 8H | 9H |
| Sicklepod | 0 | 1C,6G | 2C,6G |
| Nutsedge | 0 | 0 | 2C,7G |
| Crabgrass | 0 | 0 | 2G |
| Barnyardgrass | 2C,3H | 2C | 2C,2H |
| Wild Oats | 1C | 0 | 1C |
| Wheat | 0 | 0 | 0 |
| Corn | 3C,6G | 2C,5G | 2C,6G |
| Soybean | 1C | 3C,3H | 2C,6H |
| Rice | 3C,6G | 3C,5G | 2C,5G |
| Sorghum | 3C,7H | 3C,8H | 3C,9G |
| Sugar beet | 8H | 9G | 5C,9G |

Table A (continued)

| | Compound 17 | | Cmpd. 18 |
|---|---|---|---|
| Rate kg/ha | 0.05 | 0.4 | 0.05 |
| POST-EMERGENCE | | | |
| Bush bean | 6C,9G,6Y | 5C,9G,6Y | 9C |
| Cotton | 4C,9G | 5C,9G | 5C,9G |
| Morningglory | 2C,7G | 5C,9G | 3C,9G |
| Cocklebur | 6C,9H | 5C,9H | 9C |
| Sicklepod | 1C,5G | 4C,8G | 3C,5G |
| Nutsedge | 2C,9G | 3C,9G | 3G |
| Crabgrass | 2C,5G | 2C,9G | 0 |
| Barnyardgrass | 5C,9H | 5C,9H | 1H |
| Wild Oats | 1C | 9G | 0 |
| Wheat | 1C,5G | 3C,9G | 0 |
| Corn | 1C,3G | 2C,8H | 1C,5G |
| Soybean | 5C,9G | 5C,9G | 5C,9G |
| Rice | 2C,9G | 5C,9G | 5G |
| Sorghum | 3C,9H | 2C,9H | 2C,9H |
| Sugar beet | 3C,8H | 9C | 9C |
| PRE-EMERGENCE | | | |
| Morningglory | 8H | 8G | 9G |
| Cocklebur | 8H | 9H | 9H |
| Sicklepod | 7G | 8G | 2C,6G |
| Nutsedge | 2C,9G | 10E | 0 |
| Crabgrass | 3C | 4C,8G | 0 |
| Barnyardgrass | 9H | 9H | 3C |
| Wild Oats | 2C,6H | 9G | 2C |
| Wheat | 8G | 9G | 1C |
| Corn | 3C,8G | 2C,9G | 2C,7H |
| Soybean | 3C,8H | 2C,8H | 3C,8H |
| Rice | 5C,9H | 9C | 3C |
| Sorghum | 3C,9H | 6C,9H | 3C,9H |
| Sugar beet | 9G | 9G | 9G |

Table A (continued)

| | Compound 19 | |
|---|---|---|
| Rate kg/ha | 0.05 | 0.4 |
| POST-EMERGENCE | | |
| Bush bean | 9C | 9C |
| Cotton | 5C,9G | 5C,9G |
| Morningglory | 3C,9G | 9C |
| Cocklebur | 5C,9G | 10C |
| Sicklepod | 2C,5G | 2C,9G |
| Nutsedge | 5G | 9G |
| Crabgrass | 0 | 4G |
| Barnyardgrass | 3H | 2C,9H |
| Wild Oats | 0 | 0 |
| Wheat | 0 | 0 |
| Corn | 2C,5G | 2C,5G |
| Soybean | 5C,9G | 2C,9G |
| Rice | 2C,7G | 2C,9G |
| Sorghum | 2C,9G | 9G |
| Sugar beet | 9C | 9C |
| PRE-EMERGENCE | | |
| Morningglory | 9G | 9C |
| Cocklebur | 9H | 9H |
| Sicklepod | 8G | 9G |
| Nutsedge | 7G | 9G |
| Crabgrass | 0 | 1C |
| Barnyardgrass | 2C,4H | 3C,9H |
| Wild Oats | 0 | 1C,3G |
| Wheat | 0 | 1C |
| Corn | 3C,6G | 9G |
| Soybean | 3C,9H | 2C,9H |
| Rice | 4C,7G | 3C,9H |
| Sorghum | 5C,9G | 5C,9H |
| Sugar beet | 9C | 5C,9G |

Table A (continued)

| | Cmpd. 20 | Cmpd. 21 |
|---|---|---|
| Rate kg/ha | 0.05 | 0.05 |
| POST-EMERGENCE | | |
| Bush bean | 6C,9G,6Y | 6C,9G,6Y |
| Cotton | 6C,9G | 5C,9G |
| Morningglory | 7G | 3C,7G |
| Cocklebur | 7G | 3C,9H |
| Sicklepod | 5G | 2C,5G |
| Nutsedge | 9C | 1C,4G |
| Crabgrass | 5C,9G | 2C,9G |
| Barnyardgrass | 5C,9H | 5C,9H |
| Wild Oats | 2C | 2C,9G |
| Wheat | 0 | 2C,9G |
| Corn | 4C,9G | 3U,9G |
| Soybean | 3C,9G | 5C,9G |
| Rice | 2C,9G | 3C,9G |
| Sorghum | 9G | 2C,9G |
| Sugar beet | 1C | 8G |
| PRE-EMERGENCE | | |
| Morningglory | 8G | 9G |
| Cocklebur | 9H | 9H |
| Sicklepod | 9G | 3C,9G |
| Nutsedge | 5G | 8G |
| Crabgrass | 1C | 3C,9G |
| Barnyardgrass | 2C,8H | 6C,9H |
| Wild Oats | 2C,5G | 2C,9G |
| Wheat | 5G | 5C,9G |
| Corn | 2C,9G | 10H |
| Soybean | 3C,6H | 9H |
| Rice | 3C,7H | 10E |
| Sorghum | 2C,9H | 3C,9H |
| Sugar beet | 2H,7G | 10E |

Table A (continued)

| | Cmpd. 22 | Cmpd. 23 | Cmpd. 24 |
|---|---|---|---|
| Rate kg/ha | 0.05 | 0.05 | 0.05 |
| POST-EMERGENCE | | | |
| Bush bean | 9C | 7C,9G,6Y | 9C |
| Cotton | 6C,9G | 6C,9G | 6C,9G |
| Morningglory | 6C,9G | 2C,7G | 5C,9G |
| Cocklebur | 10C | 10C | 10C |
| Sicklepod | 6C,9G | 4C,8G | 6C,9G |
| Nutsedge | 1C,3G | 9C | 3C,8G |
| Crabgrass | 2C,8G | 2C,8G | 3C,7G |
| Barnyardgrass | 5C,9H | 9C | 9C |
| Wild Oats | 2G | 2C,9G | 3G |
| Wheat | 0 | 2C,9G | 3G |
| Corn | 4U,9G | 4U,9G | 3U,9G |
| Soybean | 9C | 9C | 9C |
| Rice | 4C,9G | 5C,9G | 5C,8G |
| Sorghum | 3U,9G | 3C,9G | 2C,9G |
| Sugar beet | 9C | 10C | 9C |
| PRE-EMERGENCE | | | |
| Morningglory | 9C | 9G | 9C |
| Cocklebur | - | 9H | 9H |
| Sicklepod | 5C,9G | 9G | 5C,9G |
| Nutsedge | 4G | 10E | 0 |
| Crabgrass | 2C,5G | 8G | 1C |
| Barnyardgrass | 2C,8H | 9H | 2C,7H |
| Wild Oats | 2C,7G | 2C,9G | 2C,6G |
| Wheat | 1C | 2C,9G | 0 |
| Corn | 5C,9H | 3C,9H | 2C,9H |
| Soybean | 9H | 2C,9H | 9H |
| Rice | 4C,9H | 10E | 3C,8H |
| Sorghum | 2C,9H | 6C,9H | 2C,7H |
| Sugar beet | 10E | 4C,9G | 9C |

Table A (continued)

| | Cmpd. 25 | Cmpd. 26 | Cmpd. 27 | Cmpd. 28 |
|---|---|---|---|---|
| Rate kg/ha | 0.4 | 0.4 | 0.4 | 0.4 |
| POST-EMERGENCE | | | | |
| Bush bean | 3C,3H,6F | 0 | 3C,9G,6Y | 2H,6Y |
| Cotton | 3C,4H | 2C,2H | 3C,6G | 2C,2H |
| Morningglory | 2C | 2G | 2C | 2C |
| Cocklebur | 1H | 0 | 2H,6G | 4G |
| Sicklepod | 0 | 1C | 0 | 1C |
| Nutsedge | 0 | 0 | 0 | 0 |
| Crabgrass | 1C | 0 | 0 | 0 |
| Barnyardgrass | 2C,6H | 0 | 0 | 0 |
| Wild Oats | 0 | 0 | 0 | 0 |
| Wheat | 0 | 0 | 0 | 0 |
| Corn | 0 | 1C,3G | 0 | 0 |
| Soybean | 1C,1H | 1H | 1H | 2G |
| Rice | 1C,3G | 0 | 2C | 3G |
| Sorghum | 1C,3H | 0 | 2C,8G | 1C |
| Sugar beet | 1C,3H | 0 | 0 | 4H |
| | | | | 1H |
| PRE-EMERGENCE | | | | |
| Morningglory | 5G | 2H | 5G | 2G |
| Cocklebur | 9H | 9H | 9H | 9H |
| Sicklepod | 2C | 2H | 1H | 4G |
| Nutsedge | 0 | 0 | 0 | 0 |
| Crabgrass | 0 | 0 | 0 | 0 |
| Barnyardgrass | 0 | 0 | 0 | 0 |
| Wild Oats | 0 | 0 | 0 | 0 |
| Wheat | 0 | 0 | 0 | 0 |
| Corn | 3G | 2C,3G | 1C,2G | 2C,5G |
| Soybean | 1C,1H | 1H | 0 | 0 |
| Rice | 2C | 2C,4G | 2C | 2C,6G |
| Sorghum | 2C,6H | 2C,4G | 5G | 2C,7H |
| Sugar beet | 6G | 7G | 5G | 8G |

Table A (continued)

| | Compound 29 |
|---|---|
| Rate kg/ha | 2 |
| POST-EMERGENCE | |
| Bush bean | - |
| Cotton | 2C,5H |
| Morningglory | 1C |
| Cocklebur | 1C |
| Sicklepod | 1C,4G |
| Nutsedge | 0 |
| Crabgrass | 0 |
| Barnyardgrass | 2C,4G |
| Wild Oats | 0 |
| Wheat | 0 |
| Corn | 0 |
| Soybean | 2C,2H |
| Rice | 2C |
| Sorghum | 2C,5G |
| Sugar beet | 2C |
| PRE-EMERGENCE | |
| Morningglory | 7G |
| Cocklebur | 6G |
| Sicklepod | 0 |
| Nutsedge | 0 |
| Crabgrass | 0 |
| Barnyardgrass | 0 |
| Wild Oats | 1C,4G |
| Wheat | 4G |
| Corn | 1C,3G |
| Soybean | 1C |
| Rice | 0 |
| Sorghum | 2C,5G |
| Sugar beet | 6G |
| Cotton | 7G |

Table A (continued)

| | Compound 30 | | Cmpd. 31 | Cmpd. 32 |
|---|---|---|---|---|
| Rate kg/ha | 0.05 | 0.4 | 0.4 | 0.05 |
| POST-EMERGENCE | | | | |
| Bush bean | 2C,2H | 3C,8G,6Y | 6C,9G,6Y | 4C,7G,6Y |
| Cotton | 2C | 3C,8H | 5C,9G | 2C |
| Morningglory | 0 | 2C,3G | 3C,7G | 2C |
| Cocklebur | 0 | 3G | 7H | 2C |
| Sicklepod | 0 | 0 | 2G | 2H |
| Nutsedge | 0 | 0 | 2G | 0 |
| Crabgrass | 0 | 0 | 0 | 2C,5G |
| Barnyardgrass | 0 | 0 | 0 | 0 |
| Wild Oats | 0 | 0 | 0 | 0 |
| Wheat | 0 | 0 | 0 | 0 |
| Corn | 0 | 0 | 0 | 0 |
| Soybean | 1H | 2C,5H | 2C,7H | 4G |
| Rice | 0 | 1C,3G | 2C | 2C,5G |
| Sorghum | 1C | 2C,3G | 1C | 2G |
| Sugar beet | 0 | 3G | 2C,9G | 2C,5G |
| | | | | 2C |
| PRE-EMERGENCE | | | | |
| Morningglory | 0 | 2C,3H | 8G | 6G |
| Cocklebur | 2C,5H | 6H | - | - |
| Sicklepod | 2H | 2C | 2C,6G | 5G |
| Nutsedge | 0 | 3G | 5G | 0 |
| Crabgrass | 2G | 2G | 0 | 3G |
| Barnyardgrass | 1C | 2C,5G | 0 | 2C,4G |
| Wild Oats | 0 | 2C,5G | 0 | 3G |
| Wheat | 0 | 7G | 0 | 4G |
| Corn | 2C | 2C,8G | 2C,5G | 5G |
| Soybean | 1C,1H | 3C,6H | 3C,5H | 2C |
| Rice | 2C | 4C,7G | 2C,5G | 2C,4G |
| Sorghum | 2C,5G | 5C,9G | 2C,8H | 2C,5G |
| Sugar beet | 3G | 8H | 5C,9G | 7G |

Table A (continued)

| | Cmpd. 33 | Cmpd. 34 | Cmpd. 35 |
|---|---|---|---|
| Rate kg/ha | 0.05 | 0.05 | 0.05 |
| POST-EMERGENCE | | | |
| Bush bean | 4C,9G,6Y | 4C,8G,6Y | 4C,9G,6Y |
| Cotton | 2C,2H | 4C,9G | 5C,9G |
| Morningglory | 1C | 2C,5G | 4C,8G |
| Cocklebur | 1C | 4C,9G | 9C |
| Sicklepod | 0 | 3G | 2C |
| Nutsedge | 0 | 3C,9G | 1C |
| Crabgrass | 0 | 2C,7G | 0 |
| Barnyardgrass | 0 | 5C,9H | 3C,5H |
| Wild Oats | 0 | 2C,6G | 0 |
| Wheat | 0 | 0 | 0 |
| Corn | 0 | 5U,9G | 1C,3H |
| Soybean | 1H | 5C,9H | 3C,9G |
| Rice | 0 | 5C,9G | 3G |
| Sorghum | 0 | 2C,9G | 2C,6H |
| Sugar beet | 2H | 1C,5G | 9C |
| PRE-EMERGENCE | | | |
| Morningglory | 5G | 2C,7G | 2C,7H |
| Cocklebur | 7G | 9H | 2C,6H |
| Sicklepod | 1H | 2C,7G | 2C,6G |
| Nutsedge | 0 | 2C,9G | 0 |
| Crabgrass | 0 | 2C | 0 |
| Barnyardgrass | 0 | 5C,9H | 1C |
| Wild Oats | 0 | 9H | 0 |
| Wheat | 0 | 2C,9G | 0 |
| Corn | 0 | 5C,9H | 2C,8H |
| Soybean | 0 | 2C,8H | 3C,6G |
| Rice | 0 | 5C,9H | 2C |
| Sorghum | 0 | 2C,9H | 2C,8H |
| Sugar beet | 3G | 9H | 4C,9G |
| Cotton | 9G | | |

Table A (continued)

| | Cmpd. 36 | Cmpd. 37 | Cmpd. 38 | Cmpd. 39 |
|---|---|---|---|---|
| Rate kg/ha | 0.05 | 0.05 | 0.4 | 0.4 |
| POST-EMERGENCE | | | | |
| Bush bean | - | - | 5C,8G,6Y | 1C,2H,6Y |
| Cotton | 0 | 0 | 2C,2H | 2C,2H |
| Morningglory | 1C,4G | 0 | 1C | 1C,1H |
| Cocklebur | 1C,1H | 0 | 2C,4G | 3C,6G |
| Sicklepod | 1C | 0 | 2C | 2G |
| Nutsedge | 2C,7G | 0 | 3G | 0 |
| Crabgrass | 0 | 0 | 2G | 0 |
| Barnyardgrass | 0 | 0 | 3C,9H | 1C,2H |
| Wild Oats | 0 | 0 | 0 | 0 |
| Wheat | 0 | 0 | 0 | 0 |
| Corn | 0 | 0 | 3C,8H | 0 |
| Soybean | 0 | 0 | 3C,8G | 1C,2H |
| Rice | 0 | 0 | 2C,7G | 1C,2G |
| Sorghum | 0 | 0 | 3C,7H | 0 |
| Sugar beet | 2C,3G | 0 | - | - |
| PRE-EMERGENCE | | | | |
| Morningglory | 4G,8H | 3H | 1C,3G | 2C |
| Cocklebur | 0 | 8H | 6H | - |
| Sicklepod | 0 | 0 | - | 3H |
| Nutsedge | 0 | 0 | 9G | 0 |
| Crabgrass | 2C,2H | 0 | 4G | 1G |
| Barnyardgrass | 0 | 0 | 2C,8H | 2C,5G |
| Wild Oats | 0 | 0 | 2C,5H | 0 |
| Wheat | 0 | 0 | 2C,8G | 0 |
| Corn | 0 | 0 | 2C,8G | 1C,5G |
| Soybean | 0 | 0 | 3C,6H | 0 |
| Rice | 0 | 0 | 3C,9H | 3C,7G |
| Sorghum | 0 | 2G | 3C,9H | 2C,7G |
| Sugar beet | 4G | 3G | 2C,9G | 2G |

Table A (continued)

| | Cmpd. 40 | Cmpd. 41 | Cmpd. 42 | Cmpd. 43 |
|---|---|---|---|---|
| Rate kg/ha | 0.4 | 0.4 | 0.4 | 0.4 |
| POST-EMERGENCE | | | | |
| Bush bean | 5C,8G,6Y | 4C,8G,6Y | 4C,8G,6Y | 5C,9G,6Y |
| Cotton | 4C,4H | 2C,3H,8G | 3C,3H | 4C,8G |
| Morningglory | 4C,8G | 2C,4H | 1C | 5C,9G |
| Cocklebur | 4C,8G | 2C,5H | 1C | 5C,9G |
| Sicklepod | 3C | 2C,4G | 2C,6G | 4C,8G |
| Nutsedge | 1C,3G | 2C | 5G | 0 |
| Crabgrass | 4G | 1C,5G | 2G | 5G |
| Barnyardgrass | 5H | 9H | 2C,6G | 2C,9H |
| Wild Oats | 0 | 2C,5G | 0 | 0 |
| Wheat | 0 | 3G | 0 | 0 |
| Corn | 4G | 7G | 1C,4G | 7G |
| Soybean | 3C,7G | 5C,8G | 5C,9G | 9C |
| Rice | 2C,6G | 4C,9G | 7G | 2C,8G |
| Sorghum | 2C,5G | 9G | 2C | 2C,9G |
| Sugar beet | - | - | - | - |
| PRE-EMERGENCE | | | | |
| Morningglory | 8G | 8G | 1C | 9G |
| Cocklebur | 8H | 8H | 8H | 8H |
| Sicklepod | 6H | 8G | 8G | 8G |
| Nutsedge | 5G | 10E | 10E | 10E |
| Crabgrass | 3G | 2C | 5G | 1C |
| Barnyardgrass | 1C,3G | 9H | 4G | 2C,7H |
| Wild Oats | 0 | 1C,4G | 1C | 1C |
| Wheat | 0 | 9G | 1C | 3G |
| Corn | 1C,4G | 2C,9H | 1C,6G | 2C,8G |
| Soybean | 1C,1H | 6H | 0 | 4C,7H |
| Rice | 1C,3G | 10E | 2C,9H | 3C,8H |
| Sorghum | 3C,7G | 2C,9H | 3C,6G | 2C,7G |
| Sugar beet | 1C,6G | 10E | 10E | 10E |

Table A (continued)

| | Cmpd. 44 | Compound 45 | | Cmpd. 46 |
|---|---|---|---|---|
| Rate kg/ha | 0.4 | 0.05 | 0.4 | 0.4 |
| POST-EMERGENCE | | | | |
| Bush bean | 5C,8G,6Y | 6C,8G,6Y | 5C,8G,6Y | 3C,9G,6Y |
| Cotton | 4C,6G | 4C,5G | 2C,5G | 3C,5G |
| Morningglory | 4C,9G | 2C | 2C,5G | 3C,7G |
| Cocklebur | 4C,9G | 2C,3G | 2C,5H | 2C,2H |
| Sicklepod | 4C,7G | 1C | 2C | 1C |
| Nutsedge | 3G | 2C,7G | 2C,9G | 2C,9G |
| Crabgrass | 4G | 0 | 1C,4G | 0 |
| Barnyardgrass | 2H | 0 | 3C,8H | 2H |
| Wild Oats | 0 | 0 | 3G | 0 |
| Wheat | 0 | 0 | 1C,4G | 0 |
| Corn | 1C,4G | 0 | 2C,8H | 0 |
| Soybean | 2C,8G | 3C,7G | 4C,7G | 2C,7G |
| Rice | 1C,3G | 3G | 2C,8G | 3C,4G |
| Sorghum | 1C | 3G | 2C,8H | 3C,6G |
| Sugar beet | - | 1C,3G | 2C,7H | 2C,2H |
| PRE-EMERGENCE | | | | |
| Morningglory | 8G | 2C,5H | 2C,7H | 2C,8G |
| Cocklebur | 9H | - | 3C,8H | 2C,5H |
| Sicklepod | 8G | 2G | 2C,8H | 2C |
| Nutsedge | 7G | 1C,5G | 3C,9G | 8G |
| Crabgrass | 1C | 0 | 1C,4G | 0 |
| Barnyardgrass | 2C | 3C,8H | 4C,9H | 5C,5G |
| Wild Oats | 0 | 2C,6G | 2C,9G | 4G |
| Wheat | 0 | 2C,6G | 2C,9G | 5G |
| Corn | 7G | 3C,7H | 3C,9H | 2C,5G |
| Soybean | 1C,4H | 1C | 3C,6G | 3C,2H |
| Rice | 1C,2G | 2C,8H | 5C,9H | 6G |
| Sorghum | 5G | 2C,8H | 4C,9H | 3C,6G |
| Sugar beet | 2C,9G | 2C,6G | 9G | 8G |

Table A (continued)

| | Cmpd. 47 | Cmpd. 48 | Cmpd. 49 | Cmpd. 50 |
|---|---|---|---|---|
| Rate kg/ha | 0.4 | 0.4 | 0.4 | 0.4 |
| POST-EMERGENCE | | | | |
| Bush bean | 4C,8G,6Y | 2C | 4C,9G,6Y | 5C,9G,6Y |
| Cotton | 2C,7G | 2C | 2C,4H | 4C,8G |
| Morningglory | 2C,4G | 2C,4G | 4C,9G | 4C,9G |
| Cocklebur | 2C,3H | 2C | 4C,9G | 3C,9G |
| Sicklepod | 3G | 0 | 2C | 2C,5G |
| Nutsedge | 0 | 0 | 5G | 2C,6G |
| Crabgrass | 0 | 0 | 0 | 0 |
| Barnyardgrass | 0 | 0 | 0 | 0 |
| Wild Oats | 0 | 0 | 0 | 0 |
| Wheat | 0 | 0 | 0 | 0 |
| Corn | 0 | 0 | 0 | 0 |
| Soybean | 3C,7G | 1C | 4C,9G | 4C,9G |
| Rice | 1C | 0 | 2C | 1C |
| Sorghum | 0 | 2G | 0 | 0 |
| Sugar beet | 1C,6G | 0 | 5C,9G | 5C,9G |
| PRE-EMERGENCE | | | | |
| Morningglory | 1C,5G | 2C | 8G | 9G |
| Cocklebur | 2C,3H | 0 | 8H | 9H |
| Sicklepod | 0 | 0 | 1C | 0 |
| Nutsedge | 7G | 0 | 0 | 4G |
| Crabgrass | 1C | 0 | 0 | 0 |
| Barnyardgrass | 4C,5G | 2C | 3C | 5C |
| Wild Oats | 2C,3G | 0 | 0 | 1C |
| Wheat | 3G | 0 | 0 | 1C |
| Corn | 2C,5G | 0 | 2C,3G | 2C,4G |
| Soybean | 2C,2H | 1C | 2C | 2C,2H |
| Rice | 3C,7G | 3C | 2C,3G | 2C,5G |
| Sorghum | 8G | 2C | 2C,3G | 2C,6G |
| Sugar beet | 7G | 5G | 7G | 4C,9G |

Table A (continued)

| | Cmpd. 51 | Cmpd. 52 | Cmpd. 53 | Cmpd. 54 |
|---|---|---|---|---|
| Rate kg/ha | 0.4 | 0.4 | 0.4 | 0.4 |
| POST-EMERGENCE | | | | |
| Bush bean | 5C,8G,6Y | 4C,8G,6Y | 4C,8G,6Y | 5C,9G,6Y |
| Cotton | 2C | 4C,7G | 2C,2H | 2C,4G |
| Morningglory | 2C,8G | 2C | 1C,2H | 4C,8G |
| Cocklebur | 2C,7G | 2C,4H | 3G | 2C,8G |
| Sicklepod | 0 | 0 | 0 | 4C,5G |
| Nutsedge | 6G | 0 | 0 | 2C,5G |
| Crabgrass | 0 | 0 | 0 | 0 |
| Barnyardgrass | 2H | 0 | 0 | 0 |
| Wild Oats | 0 | 0 | 0 | 0 |
| Wheat | 0 | 0 | 0 | 0 |
| Corn | 0 | 0 | 1C,1H | 0 |
| Soybean | 2C,8H | 3C,8G | 0 | 4C,9G |
| Rice | 1C | 2C,8G | 0 | 1C |
| Sorghum | 1C | 0 | 0 | 0 |
| Sugar beet | 2C | 0 | 0 | 5C,9G |
| PRE-EMERGENCE | | | | |
| Morningglory | 8G | 2C,7G | 2C | 2C,9G |
| Cocklebur | 2C,8H | 9H | 0 | 9H |
| Sicklepod | 0 | 1C | 0 | 2C,5G |
| Nutsedge | 2G | 8G | 9G | 0 |
| Crabgrass | 0 | 0 | 0 | 0 |
| Barnyardgrass | 5C,8G | 4C,7G | 5C | 4C |
| Wild Oats | 2C,6G | 2C,7G | 2C | 2C |
| Wheat | 7G | 2C,5G | 2G | 1C |
| Corn | 2C,8H | 4C,7G | 2C,3G | 4C,6G |
| Soybean | 2C,3H | 3C,3H | 1C | 2C,3H |
| Rice | 3C,7G | 3C,7G | 2C,5G | 2C,3G |
| Sorghum | 2C,8H | 3C,7G | 6G | 3G |
| Sugar beet | 8G | 8G | 5G | 10E |

Table A (continued)

| | Cmpd. 55 | Cmpd. 56 | Cmpd. 57 | Cmpd. 58 |
|---|---|---|---|---|
| Rate kg/ha | 0.4 | 0.4 | 0.4 | 0.4 |
| POST-EMERGENCE | | | | |
| Bush bean | 5C,9G,6Y | 4C,7H,6Y | 3C,3H,6F | 5C,9G,6Y |
| Cotton | 2C | 4C,9G | 3C,4H | 5C,9G |
| Morningglory | 2C,9G | 2C,5G | 2C | 2C,8H |
| Cocklebur | 2C,9G | 2C,3H | 2C | 2C,5G |
| Sicklepod | 3C,8G | 1C | 0 | 3C |
| Nutsedge | 10C | 5G | 0 | 3G |
| Crabgrass | 0 | 3G | 0 | 1C |
| Barnyardgrass | 1H | 9H | 1H | 2H |
| Wild Oats | 0 | 2C | 0 | 0 |
| Wheat | 0 | 2C,5G | 0 | 0 |
| Corn | 2G | 2C,6H | 2G | 3G |
| Soybean | 2C,9G | 4C,7G | 2C,2H | 2C,8G |
| Rice | 1C | 3C,6G | 0 | 2C |
| Sorghum | 0 | 3C,8G | 2C | 3G |
| Sugar beet | 2G | 1C,5G | 0 | 3C,7G |
| PRE-EMERGENCE | | | | |
| Morningglory | 9G | 2C,6G | 2C | 9C |
| Cocklebur | 7G | 6G | 0 | 9H |
| Sicklepod | 1C | 2C,5G | 2G | 8H |
| Nutsedge | 0 | 8G | 0 | 10E |
| Crabgrass | 0 | 1C | 2G | 0 |
| Barnyardgrass | 3C | 3C,8G | 4C | 4C |
| Wild Oats | 1C | 2C | 0 | 2C |
| Wheat | 0 | 2C,8G | 0 | 1C |
| Corn | 2C,4G | 3C,8H | 2G | 1C,4G |
| Soybean | 2C | 3C,5H | 1C | 3C |
| Rice | 2C | 2C,8G | 2C,4G | 2C |
| Sorghum | 2G | 2C,8H | 2C,6G | 1C,5G |
| Sugar beet | 9G | 7G | 4G | 2C,5G |

Table A (continued)

| | Cmpd. 59 | Cmpd. 60 |
|---|---|---|
| Rate kg/ha | 0.4 | 0.4 |
| POST-EMERGENCE | | |
| Bush bean | 6C,9G,6Y | 0 |
| Cotton | 4C,9G | 2C |
| Morningglory | 4C,9G | 2C |
| Cocklebur | 3C,9H | 2C |
| Sicklepod | 4C,8G | 1C |
| Nutsedge | 2C,8G | 0 |
| Crabgrass | 2C | 0 |
| Barnyardgrass | 0 | 0 |
| Wild Oats | 0 | 0 |
| Wheat | 0 | 0 |
| Corn | 0 | 0 |
| Soybean | 4C,9G | 2C,5H |
| Rice | 3C | 2C |
| Sorghum | 1C | 2C |
| Sugar beet | 2C,4G | 6G |
| PRE-EMERGENCE | | |
| Morningglory | 9C | 2C |
| Cocklebur | 9H | 1H |
| Sicklepod | 2C,4G | 2G |
| Nutsedge | 9G | 0 |
| Crabgrass | 2G | 2G |
| Barnyardgrass | 4C | 2H |
| Wild Oats | 1C | 0 |
| Wheat | 2G | 0 |
| Corn | 1C,5G | 2G |
| Soybean | 2C,6H | 0 |
| Rice | 2C,5G | 1C |
| Sorghum | 5G | 2G |
| Sugar beet | 10E | 3G |

Table A (continued)

| | Cmpd. 61 | Cmpd. 62 | Cmpd. 63 |
|---|---|---|---|
| Rate kg/ha | 0.4 | 0.4 | 0.4 |
| POST-EMERGENCE | | | |
| Bush bean | 5C,9G,6Y | 5C,9G,6Y | 9C |
| Cotton | 5C,9G | 9C | 5C,9G |
| Morningglory | 5C,9G | 9C | 5C,9G |
| Cocklebur | 5C,9G | 10C | 10C |
| Sicklepod | 5C,9G | 9C | 6C,9G |
| Nutsedge | 9G | 6C,9G | 10C |
| Crabgrass | 2C,9G | 10C | 3C,9G |
| Barnyardgrass | 6C,9H | 10C | 6C,9G |
| Wild Oats | 2C,9G | 5C,9G | 3C,9H |
| Wheat | 9G | 9C | 3C,9G |
| Corn | 5C,9G | 3C,9G | 2U,9G |
| Soybean | 4C,9G | 5C,9G | 5C,9G |
| Rice | 6C,9G | 6C,9G | 6C,9G |
| Sorghum | 3C,9G | 3C,9G | 4C,9G |
| Sugar beet | 9C | 9C | 9C |
| PRE-EMERGENCE | | | |
| Morningglory | 9G | 9C | 9C |
| Cocklebur | 9H | 9H | 9H |
| Sicklepod | 5C,9G | 9C | 2C,8G |
| Nutsedge | 10E | 10E | 10E |
| Crabgrass | 1C,5G | 2C,8G | 2C,5G |
| Barnyardgrass | 2C,9H | 5C,9H | 5C,9G |
| Wild Oats | 2C,9G | 5C,9H | 9G |
| Wheat | 5C,9H | 10E | 10H |
| Corn | 10E | 10H | 2U,9H |
| Soybean | 8H | 9H | 9H |
| Rice | 10E | 10E | 10E |
| Sorghum | 10H | 10H | 5C,9H |
| Sugar beet | 9C | 10E | 10C |
| Cotton | - | - | - |

Table A (continued)

| | Cmpd. 64 | Cmpd. 65 | Cmpd. 66 |
|---|---|---|---|
| Rate kg/ha | 0.4 | 0.4 | .05 |
| POST-EMERGENCE | | | |
| Bush bean | 9C | 6C,9G,6Y | - |
| Cotton | 6C,9G | 5C,9G | 10C |
| Morningglory | 10C | 9C | 3C,8H |
| Cocklebur | 10C | 9C | 3C,9G |
| Sicklepod | 10C | 9C | 3C,6G |
| Nutsedge | 9C | 9C | 2C,8G |
| Crabgrass | 8C | 1C,6G | 2C,5G |
| Barnyardgrass | 9C | 2C,9H | 3C,8H |
| Wild Oats | 6G | 4G | 2C,9G |
| Wheat | 2G | 0 | 8G |
| Corn | 5C,9G | 1U,8H | 3C,9H |
| Soybean | 6C,9G | 6C,9G | 5C,9G |
| Rice | 5C,9G | 2C,8G | 4C,8G |
| Sorghum | 2C,9G | 2C,8H | 4C,8H |
| Sugar beet | 10C | 9C | 3C,8G |
| PRE-EMERGENCE | | | |
| Morningglory | 9C | 9C | 8G |
| Cocklebur | 9H | 9H | 9H |
| Sicklepod | 4C,9G | 5C,9G | 5G |
| Nutsedge | 9G | 9G | 2C,5G |
| Crabgrass | 2C | 1C | 2C,3G |
| Barnyardgrass | 3C,8H | 3C,8H | 3C,9H |
| Wild Oats | 9G | 2C,4G | 3C,9G |
| Wheat | 1C,7G | 2G | 3C,9G |
| Corn | 3C,9G | 9G | 3C,9H |
| Soybean | 9H | 9H | 2C,5H |
| Rice | 10E | 9H | 3C,9H |
| Sorghum | 9H | 2C,8H | 3C,9H |
| Sugar beet | 10E | 10E | 3C,9G |
| Cotton | - | 2C,9G | |

Claims: Cognate

1. A compound of the formula:

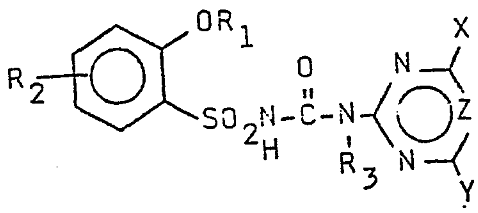 (1)

wherein

$R_1$ is 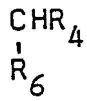, $CH_2CH_2R_5$; cyclopentyl, $C_5$-alkyl, $CH_2C{\equiv}CH$,

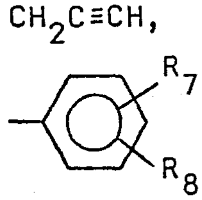, 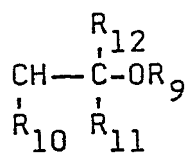 or 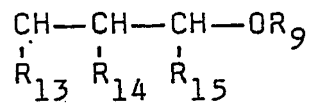;

$R_2$ is H, F, Cl, Br, $CH_3$; $OCH_3$ or $CF_3$;

$R_3$ is H or $CH_3$;

$R_4$ is CN, $CO_2CH_3$, $CO_2C_2H_5$, $SO_2N(CH_3)_2$, cyclopropyl,

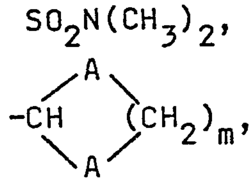, $CH(ACH_3)_2$ or

$CH(ACH_2CH_3)_2$;

$R_5$ is $N(CH_3)_2$, CN, $CO_2CH_3$, $CO_2C_2H_5$ or $SO_2N(CH_3)_2$;

$R_6$ is H, $CH_3$ or $OCH_3$;

$R_7$ is F, Cl, $CO_2CH_3$, $CH_3$, $OCH_3$, $NO_2$, $CF_3$ or $SO_2CH_3$;

$R_8$ is H, Cl, $CH_3$ or $OCH_3$;

$R_9$ is H, $COCH_3$, $COC_2H_5$, $COCH_2CH_2CH_3$, $COCH(CH_3)_2$, $COC_6H_5$, $CONHCH_3$, $CONHC_2H_5$,

$CON(CH_3)_2$, 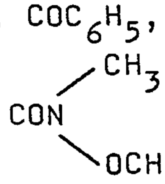, $CONHC_6H_5$, $SO_2CH_3$,

$SO_2C_2H_5$, $SO_2CF_3$, 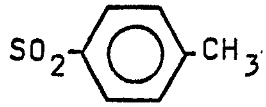 or

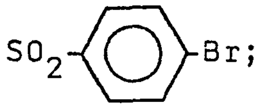;

$R_{10}$ is H or $CH_3$;
$R_{11}$ is H or $CH_3$;
$R_{12}$ is H or $CH_3$;
$R_{13}$ is H or $CH_3$;
$R_{14}$ is H or $CH_3$;
$R_{15}$ is H or $CH_3$;
m is 2 or 3;
A is O or S;
Z is CH or N;
X is $CH_3$, $OCH_3$, Cl, $OCF_2H$ or $SCF_2H$; and
Y is $CH_3$, $C_2H_5$, $OCH_3$, $OC_2H_5$, $CH_2OCH_3$, $OCF_3$, $CH(OCH_3)_2$, 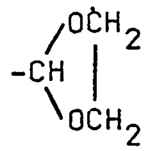, $NH_2$, $NHCH_3$, $N(CH_3)_2$ or $GCF_2T$ where G is O or S and T is H, CHClF, CHBrF, $CF_2H$ or $CHFCF_3$;

provided that

1) when $R_6$ is $OCH_3$, then $R_4$ is $CO_2CH_3$, $CO_2C_2H_5$ or CN;
2) when $R_{11}$ and $R_{12}$ are both $CH_3$, then $R_9$ is H;
3) when either $R_{13}$, $R_{14}$ or $R_{15}$ is $CH_3$, then the others must be H;
4) when X is Cl, then Y is $OCH_3$, $CH_3$, $OC_2H_5$, $NH_2$, $NHCH_3$ or $N(CH_3)_2$ and Z is CH; and
5) when $R_6$ is H and $R_4$ is $CO_2CH_3$, then X and Y are not simultaneously $CH_3$;

and its agriculturally suitable salts.

2. Compounds of Claim 1 where

$R_1$ is $\underset{R_6}{|}\!\!\!\!CHR_4$, $\underset{R_{10}}{CH}\text{—}\overset{R_{12}}{\underset{R_{11}}{C}}\text{-}OR_9$ or $\underset{R_{13}}{CH}\text{—}\underset{R_{14}}{CH}\text{—}\underset{R_{15}}{CH}\text{—}OR_9$;

$R_4$ is cyclopropyl;

$R_6$ is H or $CH_3$;

$R_{12}$ is H;

$R_{13}$ is H;

$R_{14}$ is H; and

$R_{15}$ is H.

3. Compounds of Claim 2 where $R_3$ is H.

4. Compounds of Claim 3 where $R_2$ is H and $R_9$ is H, $COCH_3$, $CONHCH_3$, $SO_2CH_3$ or $SO_2CF_3$.

5. Compounds of Claim 4 where

$R_1$ is $\underset{R_{10}}{CH}\text{—}\underset{R_{11}}{CH}\text{-}OR_9$, $CH_2CH_2CH_2OR_9$ or

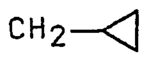 ;

$R_9$ is H;

$R_{10}$ is H or $CH_3$; and

$R_{11}$ is H.

6. Compounds of Claim 5 where X and Y are independently $CH_3$ or $OCH_3$.

7. The compound of Claim 1 which is N-[(4-methoxy-6-methyl-1,3,5-triazin-2-yl)aminocarbonyl]-2-(2-hydroxyethoxy)benzenesulfonamide.

8. The compound of Claim 1 which is N-[(4,6-dimethoxy-1,3,5-triazin-2-yl)aminocarbonyl]-2-(2-hydroxyethoxy)benzenesulfonamide.

9. The compound of Claim 1 which is N-[(4,6-dimethoxypyrimidin-2-yl)aminocarbonyl]-2-(2-hydroxyethoxy)benzenesulfonamide.

10. The compound of Claim 1 which is N-[4-methoxy-6-methylpyrimidin-2-yl)aminocarbonyl]-2-(2-hydroxyethoxy)benzenesulfonamide.

11. The compound of Claim 1 which is N-[4,6-dimethylpyrimidin-2-yl)aminocarbonyl]-2-(2-hydroxyethoxy)benzenesulfonamide.

12. The compound of Claim 1 which is N-[4-methoxy-6-methylpyrimidin-2-yl)aminocarbonyl]-2-(2-methylsulfonyloxyethoxy)benzenesulfonamide.

13. The compound of Claim 1 which is N-[(4,6-dimethoxypyrimidin-2-yl)aminocarbonyl]-2-(cyclopropylmethyloxy)benzenesulfonamide..

14. The compound of Claim 1 which is N-[(4-methoxy-6-methyl-1,3,5-triazin-2-yl)aminocarbonyl]-2-(cyclopropylmethyloxy)benzenesulfonamide.

15. The compound of Claim 1 which is N-[(4,6-dimethoxy-1,3,5-triazin-2-yl)aminocarbonyl]-2-(cyclopropylmethyloxy)benzenesulfonamide.

16. The compound of Claim 1 which is N-[(4,6-dimethoxy-1,3,5-triazin-2-yl)aminocarbonyl]-2-(2-methylphenoxy)benzenesulfonamide.

17. Compounds of claim 1 wherein

$R_1$ is $\underset{R_6}{CHR_4}$, $CH_2CH_2R_5$, $\underset{CH_3}{CHCH_2OCH_3}$,

$CH_2\underset{CH_3}{CHOCH_3}$ or cyclopentyl;

$R_2$ and $R_3$ are as defined in claim 1;

$R_4$ is CN, $CO_2CH_3$, $CO_2C_2H_5$, $SO_2N(CH_3)_2$ or cyclopropyl;

$R_5$ is as defined in claim 1;

$R_6$ is H or $CH_3$;

Z is as defined in claim 1;

X is $CH_3$, $OCH_3$ or Cl; and

Y is $CH_3$, $C_2H_5$, $OCH_3$, $OC_2H_5$, $CH_2OCH_3$,

$CH(OCH_3)_2$, 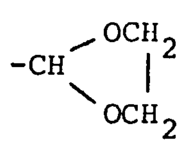 ,$NH_2$, $NHCH_3$ or $N(CH_3)_2$.

18. A process for making a compound of claim 1 which comprises

(a) reacting a sulfonyl isocyanate of formula

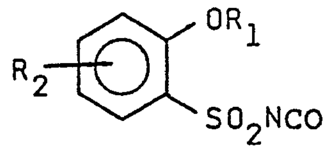

(2)

with a heterocyclic amine of formula

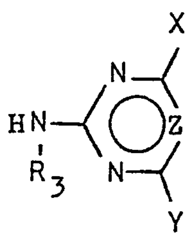 (3)

wherein $R_1$, $R_2$, $R_3$, $R_9$, X, Y and Z are as defined in claim 1 except that $R_9$ is not H; or

(b) displacing one of the chlorine atoms in a compound of formula

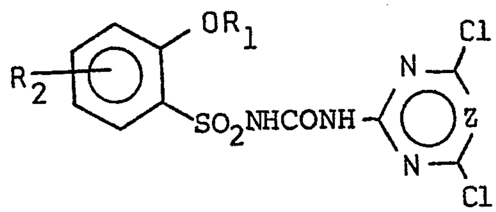 (6)

by $OCH_3$ or $OC_2H_5$ and optionally displacing the other chlorine atom by $OCH_3$;

wherein $R_1$, $R_2$ and Z are as defined in claim 1 except that $R_9$ is not H; or

(c) reacting a sulfonamide of formula

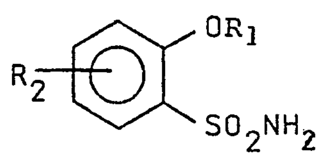

(4)

with a heterocyclic methyl carbamate of formula

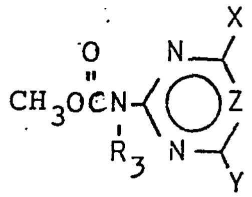

(7)

wherein $R_1$, $R_2$, $R_3$, X, Y and Z are as defined in claim 1 except that $R_9$ is not H; or

(d) reacting a phenylcarbamate of formula

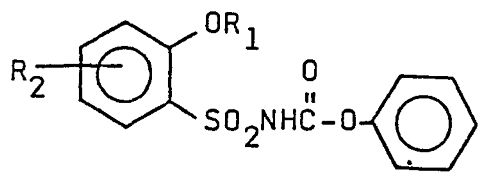

(10)

wherein $R_1$ and $R_2$ are as defined in claim 1, with the heterocyclic amine of formula (3) defined above; or

(e) reacting a 2-hydroxybenzenesulfonylurea of formula

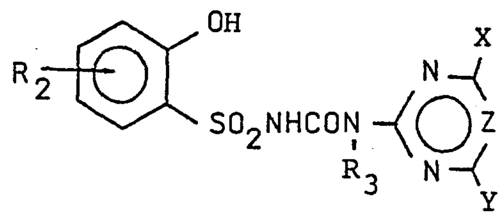

(11)

with $R_1Q$;

wherein $R_1$, $R_2$, $R_3$, X, Y and Z are as defined in claim 1 and Q is a leaving group; or

(f) reacting a compound of formula (1) wherein $R_9$ is H with an appropriate acyl halide, sulfonyl halide or carbamoyl halide in the presence of an acid acceptor, or with an appropriate isocyanate, to obtain a compound of claim 1 wherein $R_9$ is other than H; or

(g) hydrolyzing a compound of claim 1 wherein $R_9$ is alkylsulfonyl or arylsulfonyl to obtain a compound of claim 1 wherein $R_9$ is H; or

(h) reacting said compound of formula (11) as defined above with a carbonate ester of formula

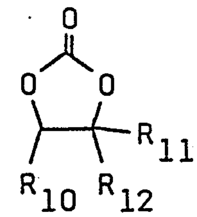

(12)

wherein $R_{10}$, $R_{11}$ and $R_{12}$ are as defined in claim 1.

19. A composition suitable for controlling the growth of undesired vegetation comprising an effective amount of a herbicidal compound and at least one of the following: surfactant, solid or liquid diluent, characterised in

that said herbicidal compound comprises a compound of any of claims 1 to 17.

20. A method for controlling the growth of undesired vegetation by applying to the locus to be protected an effective amount of a herbicidal compound, characterised in

that said herbicidal compound comprises a compound of any of claims 1 to 17.

# 0094790

Austria
BA-8474-A/8522
Cognate

Claims:

1. A process for the preparation of a compound of the formula:

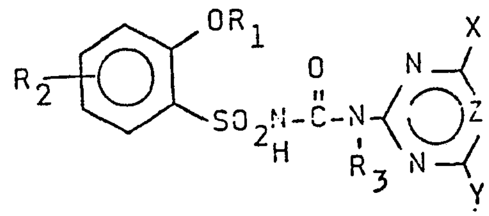

(1)

wherein

$R_1$ is 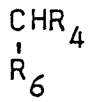, $CH_2CH_2R_5$, cyclopentyl, $C_5$-alkyl, $CH_2C{\equiv}CH$,

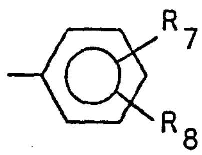, 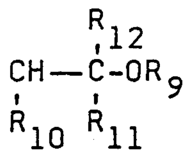 or 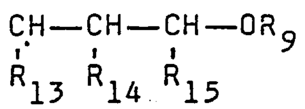;

$R_2$ is H, F, Cl, Br, $CH_3$, $OCH_3$ or $CF_3$;

$R_3$ is H or $CH_3$;

$R_4$ is CN, $CO_2CH_3$, $CO_2C_2H_5$, $SO_2N(CH_3)_2$, cyclopropyl,

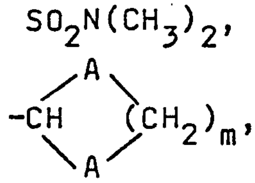, $CH(ACH_3)_2$ or $CH(ACH_2CH_3)_2$;

$R_5$ is $N(CH_3)_2$, CN, $CO_2CH_3$, $CO_2C_2H_5$ or $SO_2N(CH_3)_2$;

$R_6$ is H, $CH_3$ or $OCH_3$;

$R_7$ is F, Cl, $CO_2CH_3$, $CH_3$, $OCH_3$, $NO_2$, $CF_3$ or $SO_2CH_3$;

$R_8$ is H, Cl, $CH_3$ or $OCH_3$;

$R_9$ is H, $COCH_3$, $COC_2H_5$, $COCH_2CH_2CH_3$, $COCH(CH_3)_2$, $COC_6H_5$, $CONHCH_3$, $CONHC_2H_5$,

$CON(CH_3)_2$, 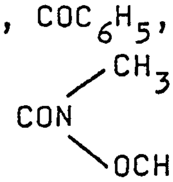, $CONHC_6H_5$, $SO_2CH_3$,

$SO_2C_2H_5$, $SO_2CF_3$, 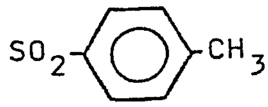 or

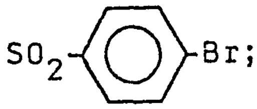;

$R_{10}$ is H or $CH_3$;
$R_{11}$ is H or $CH_3$;
$R_{12}$ is H or $CH_3$;
$R_{13}$ is H or $CH_3$;
$R_{14}$ is H or $CH_3$;
$R_{15}$ is H or $CH_3$;
m is 2 or 3;
A is O or S;
Z is CH or N;
X is $CH_3$, $OCH_3$, Cl, $OCF_2H$ or $SCF_2H$; and
Y is $CH_3$, $C_2H_5$, $OCH_3$, $OC_2H_5$, $CH_2OCH_3$, $OCF_3$, $CH(OCH_3)_2$, 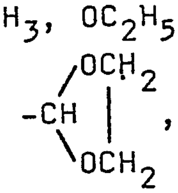, $NH_2$, $NHCH_3$, $N(CH_3)_2$ or $GCF_2T$ where G is O or S and T is H, CHClF, CHBrF, $CF_2H$ or $CHFCF_3$;

provided that

1) when $R_6$ is $OCH_3$, then $R_4$ is $CO_2CH_3$, $CO_2C_2H_5$ or CN;
2) when $R_{11}$ and $R_{12}$ are both $CH_3$, then $R_9$ is H;
3) when either $R_{13}$, $R_{14}$ or $R_{15}$ is $CH_3$, then the others must be H;
4) when X is Cl, then Y is $OCH_3$, $CH_3$, $OC_2H_5$, $NH_2$, $NHCH_3$ or $N(CH_3)_2$ and Z is CH; and
5) when $R_6$ is H and $R_4$ is $CO_2CH_3$, then X and Y are not simultaneously $CH_3$;

or an agriculturally suitable salt thereof,

which comprises

(a) reacting a sulfonyl isocyanate of formula

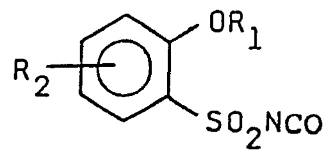

(2)

with a heterocyclic amine of formula

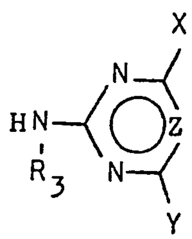

(3)

wherein $R_1$, $R_2$, $R_3$, $R_9$, X, Y and Z are as defined above except that $R_9$ is not H; or

(b) displacing one of the chlorine atoms in a compound of formula

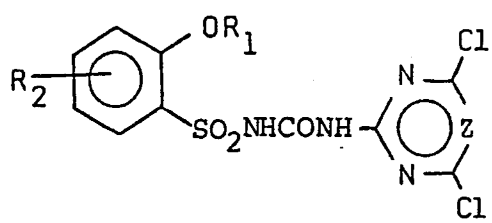

(6)

by $OCH_3$ or $OC_2H_5$ and optionally displacing the other chlorine atom by $OCH_3$;

wherein $R_1$, $R_2$ and Z are as defined above except that $R_9$ is not H; or

(c) reacting a sulfonamide of formula

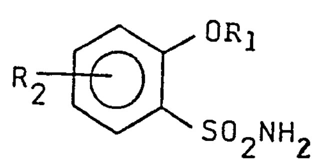

(4)

with a heterocyclic methyl carbamate of formula

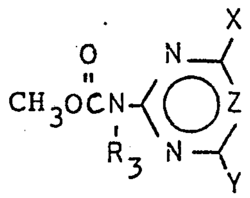

(7)

wherein $R_1$, $R_2$, $R_3$, X, Y and Z are as defined above except that $R_9$ is not H; or

(d) reacting a phenylcarbamate of formula

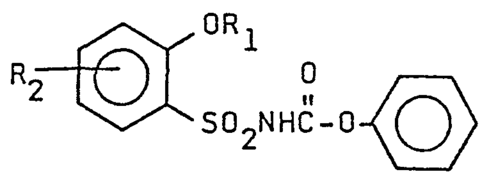

(10)

wherein $R_1$ and $R_2$ are as defined above, with the heterocyclic amine of formula (3) defined above; or

(e) reacting a 2-hydroxybenzenesulfonylurea of formula

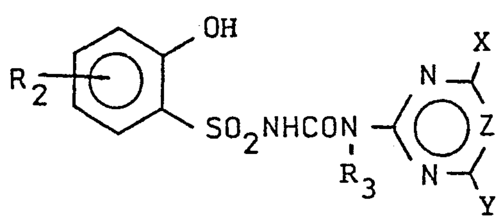

(11)

with $R_1Q$;

wherein $R_1$, $R_2$, $R_3$, X, Y and Z are as defined above and Q is a leaving group; or

(f) reacting a compound of formula (1) wherein $R_9$ is H with an appropriate acyl halide, sulfonyl halide or carbamoyl halide in the presence of an acid acceptor, or with an appropriate isocyanate, to obtain a compound of formula 1 wherein $R_9$ is other than H; or

(g) hydrolyzing a compound of formula 1 wherein $R_9$ is alkylsulfonyl or arylsulfonyl to obtain a compound of formula 1 wherein $R_9$ is H; or

(h) reacting said compound of formula (11) as defined above with a carbonate ester of formula

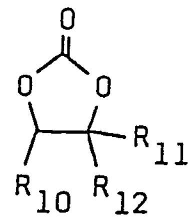

(12)

wherein $R_{10}$, $R_{11}$ and $R_{12}$ are as defined above.

2. A process of claim 1 wherein

$R_1$ is $CHR_4$, $CH_2CH_2R_5$, $CHCH_2OCH_3$,

$R_6$ $CH_3$

$CH_2CHOCH_3$ or cyclopentyl;

$CH_3$

$R_2$ and $R_3$ are as defined in claim 1;

$R_4$ is CN, $CO_2CH_3$, $CO_2C_2H_5$, $SO_2N(CH_3)_2$ or cyclopropyl;

$R_5$ is as defined in claim 1;

$R_6$ is H or $CH_3$;

Z is as defined in claim 1;

X is $CH_3$, $OCH_3$ or Cl; and

Y is $CH_3$, $C_2H_5$, $OCH_3$, $OC_2H_5$, $CH_2OCH_3$,

$CH(OCH_3)_2$, 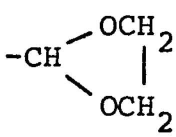 , $NH_2$, $NHCH_3$ or $N(CH_3)_2$.

3. A process of Claim 1 or 2 where

$R_1$ is $\underset{R_6}{\underset{|}{CHR_4}}$, $\underset{R_{10}}{\underset{|}{CH}}-\overset{R_{12}}{\overset{|}{\underset{R_{11}}{\underset{|}{C}}}}-OR_9$ or $\underset{R_{13}}{\underset{|}{CH}}-\underset{R_{14}}{\underset{|}{CH}}-\underset{R_{15}}{\underset{|}{CH}}-OR_9$;

$R_4$ is cyclopropyl;

$R_6$ is H or $CH_3$;

$R_{12}$ is H;

$R_{13}$ is H;

$R_{14}$ is H; and

$R_{15}$ is H.

4. A process of Claim 3 where $R_3$ is H.

5. A process of Claim 4 where $R_2$ is H and $R_9$ is H, $COCH_3$, $CONHCH_3$, $SO_2CH_3$ or $SO_2CF_3$.

6. A process of Claim 5 where

$R_1$ is $\underset{R_{10}}{\underset{|}{CH}}-\underset{R_{11}}{\underset{|}{CH}}-OR_9$, $CH_2CH_2CH_2OR_9$ or

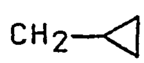 ;

$R_9$ is H;

$R_{10}$ is H or $CH_3$; and

$R_{11}$ is H.

7. A process of Claim 6 where X and Y are independently $CH_3$ or $OCH_3$.

8. A process of claim 1 or 2 wherein the product is a compound selected from

N-[(4-methoxy-6-methyl-1,3,5-triazin-2-yl)aminocarbonyl]-2-(2-hydroxyethoxy)benzenesulfonamide;

N-[(4,6-dimethoxy-1,3,5-triazin-2-yl)aminocarbonyl]-2-(2-hydroxyethoxy)benzenesulfonamide;

N-[(4,6-dimethoxypyrimidin-2-yl)aminocarbonyl]-2-(2-hydroxyethoxy)benzenesulfonamide;

N-[(4-methoxy-6-methylpyrimidin-2-yl)aminocarbonyl]-2-(2-hydroxyethoxy)benzenesulfonamide;

N-[(4,6-dimethylpyrimidin-2-yl)aminocarbonyl]-2-(2-hydroxyethoxy)benzensulfonamide;

N-[(4-methoxy-6-methylpyrimidin-2-yl)aminocarbonyl]-2-(2-methylsulfonyloxyethoxy)benzenesulfonamide;

N-[(4,6-dimethoxypyrimidin-2-yl)aminocarbonyl]-2-(cyclopropylmethyloxy)benzenesulfonamide;
N-[(4-methoxy-6-methyl-1,3,5-triazin-2-yl)aminocarbonyl]-2-(cyclopropylmethyloxy)benzenesulfonamide;
N-[(4,6-dimethoxy-1,3,5-triazin-2-yl)aminocarbonyl]-2-(cyclopropylmethyloxy)benzenesulfonamide; or
N-[(4,6-dimethoxy-1,3,5-triazin-2-yl)aminocarbonyl]-2-(2-methylphenoxy)benzenesulfonamide.

9. A method for controlling the growth of undesired vegetation by applying to the locus to be protected an effective amount of a herbicidal compound, characterised in

that said herbicidal compound comprises a compound of formula (1) as defined in any of claims 1 to 7.

10. The method of claim 9 wherein said herbicidal compound is selected from the compounds defined in claim 8.